# EUROPEAN PATENT SPECIFICATION

(11) **EP 2 458 985 B1**
(45) Date of publication and mention of the grant of the patent: **05.11.2014**
(21) Application number: 10737727.7
(22) Date of filing: 27.07.2010
(51) Int. Cl.: A01N 33/12, A01N 37/16, A01N 59/00, A61L 2/16, A01P 1/00, A01N 25/24, A01N 25/04, A01N 25/10

(54) **ENZYMATIC IN SITU PREPARATION OF PERACID-BASED REMOVABLE ANTIMICROBIAL COATING COMPOSITIONS AND METHODS OF USE**
ENZYMATISCHE IN-SITU-HERSTELLUNG VON ABLÖSBAREN ANTIMIKROBIELLEN BESCHICHTUNGSZUSAMMENSETZUNGEN AUF PERSÄUREBASIS UND ANWENDUNGSVERFAHREN
PRÉPARATION ENZYMATIQUE <I>IN SITU</I> DE COMPOSITIONS DE REVÊTEMENT ANTIMICROBIENNES, APTES À ÊTRE RETIRÉES, À BASE DE PERACIDE, ET PROCÉDÉS D'UTILISATION

(30) Priority: 27.07.2009 US 228732 P; 27.07.2009 US 228735 P; 27.07.2009 US 228737 P; 27.07.2009 US 228746 P; 26.07.2010 US 843087
(43) Date of publication of application: 06.06.2012
(73) Proprietor: E. I. du Pont de Nemours and Company, Wilmington, DE 19898 (US)
(72) Inventor: STAUFFER, Christina, S., Wilmington DE 19809 (US); DICOSIMO, Robert, Chadds Ford PA 19317-9720 (US); PAYNE, Mark, S., Wilmington DE 19808 (US); HOFFMANN, Christian, Newark DE 19711 (US); LEGER, Lynn, Mississauga Ontario L5M 5R3 (CA); ERKENBRECHER JR, Carl, W., Elkton MD 21921 (US)
(74) Representative: Morf, Jan Stefan
(86) International application number: PCT/US2010/043376
(87) International publication number: WO 2011/017095

(56) References cited:
- EP-A1- 1 393 629
- EP-A1- 2 020 180
- WO-A1-88/00795
- WO-A1-92/19230
- WO-A1-96/22687
- WO-A2-2007/070609
- US-A- 4 941 989

## Description

### FIELD OF INVENTION

This invention relates to a method for controlling microorganisms comprising coating a surface with a removable, antimicrobial film-forming composition that contains *in situ* enzymatically generated peroxyacids and methods of applying said compositions.

### BACKGROUND

Chemical disinfectants are now used in an increasing number of industries to ensure food safety and to comply with more stringent health and safety regulations. Compositions for decontamination, disinfection and sterilization must have excellent microbiocidal efficacy, act rapidly, be non-corrosive and be effective in minimal quantities. The ideal composition must possess multiple mechanisms for destroying microorganisms, thus providing efficacy against a broad range of microorganisms and reducing the possibility of leading to evolution of antimicrobial-resistant microorganisms.

Compositions containing peroxide, especially hydrogen peroxide (HP) and peracetic acid (also called peroxyacetic acid, PAA) have been proven to be very effective antimicrobial agents. Many such preparations have passed the necessary tests and are registered products as sanitizers, disinfectants, and sporicides. Many of these peroxide/peroxyacid compositions are liquid solutions, which can be used for treating aqueous solutions, surfaces and objects. Some are approved for food contact surfaces and for sanitization of some food products. Some are also registered as disinfectants or sterilants as vapor phase treatments.

Methods to clean, disinfect, and/or sanitize hard surfaces, meat products, living plant tissues, and medical devices against undesirable microbial growth have been described (U.S. Patent 6,545,047; U.S. Patent 6,183,807; U.S. Patent 6,518,307; U.S. Patent application publication 20030026846; and U.S. Patent 5,683,724). Peracids have also been reported to be useful in preparing bleaching compositions for laundry detergent applications (U.S. Patent 3,974,082; U.S. Patent 5,296,161; and U.S. Patent 5,364,554).

There are several methods of preparing peracids. For example WO2006/076334 describes a microbicidal and decontaminant composition comprising an aqueous solution of peroxides and peracids; U.S. patent 5,130,124 describes an aqueous antimicrobial film-forming composition with hydrogen peroxide; WO1996/022687 describes an oxidizing film-forming composition containing hydrogen peroxide. Enzyme catalysts may also be used to facilitate the generation of peracids. Commonly owned co-pending U.S. Patent Application 2008/0176299A1 described a process for enzymatic production of peracids. Use of enzymes such as esterases (U.S. Patent 3,974,082), proteases (U.S. Patent 5,364,554), esterases and lipases (U.S. Patent 5,296,161) for enzymatic generation of peracids have also been described.

Despite the descriptions of peracid manufacture set forth above, a need exists for an *in situ* enzymatically-generated, peracid-based, easily removable, homogenous antimicrobial coating composition providing both short-term and extended long-term (or residual) antimicrobial efficacy after application to a surface.

### SUMMARY OF INVENTION

The present invention addresses the problems identified above by providing a method and composition that is antimicrobial and which also provides extended effectiveness against microorganisms by forming an antimicrobial coating on a target surface.

In one aspect the present invention is directed to method of providing control of microorganisms at a locus comprising the steps:
a) forming a composition by combining components comprising:
   i) a film-forming water soluble or water-dispersible agent wherein the agent is polyvinyl alcohol, polyvinyl alcohol copolymer or polyvinyl pyrrolidone;
   ii) an inert solvent;
   iii) a carboxylic acid ester substrate;
   iv) a peroxygen source;
   v) an enzyme catalyst having perhydrolase activity thereby forming at least one peroxyacid to provide at least a first antimicrobial agent; and
   vi) a rheology modifier;
b) applying said composition to said locus; and
c) allowing said composition to dry thereby forming a coating on said locus.
wherein said components further comprise a second antimicrobial agent, wherein said second antimicrobial agent comprises a quaternary ammonium compound.

Another aspect of the invention is directed to a method of providing control of microorganisms at a locus comprising the steps:
(a) providing a first premixed component comprising an inert solvent, a rheology modifier and a film-forming agent wherein the agent is polyvinyl alcohol, polyvinyl alcohol copolymer or polyvinyl pyrrolidone;
(b) providing a second premixed component comprising an enzyme catalyst having perhydrolase activity, a carboxylic acid ester substrate and a peroxygen source;
(c) mixing said first premixed component and said second premixed component to obtain a liquid coating composition comprising a first antimicrobial agent comprising a peroxyacid;
(d) applying said coating composition to the locus; and
(e) allowing said coating composition to dry thereby forming a coating on said locus.
wherein at least one premixed component further comprises a second antimicrobial agent wherein said second antimicrobial agent comprises a quaternary ammonium compound.

Another aspect of the invention is directed to an antimicrobial composition comprising components which comprise:
a) a film-forming water soluble or water-dispersible agent; wherein the agent is polyvinyl alcohol, polyvinyl alcohol copolymer or polyvinyl pyrrolidone;
b) an inert solvent;
c) a carboxylic acid ester substrate;
d) a peroxygen source;
e) an enzyme catalyst having perhydrolase activity; and
f) a rheology modifier;
wherein, upon combining said components, a peroxyacid is formed; wherein said components further comprise a component that is an antimicrobial agent comprising a quaternary ammonium compound.

Another aspect of the invention is directed to an article comprising a coating on at least one surface thereof with an antimicrobial composition, wherein the antimicrobial composition comprises:
a) a film-forming water soluble or water-dispersible agent wherein the agent is polyvinyl alcohol, polyvinyl alcohol copolymer or polyvinyl pyrrolidones;
b) an inert solvent;
c) a carboxylic acid ester substrate;
d) a peroxygen source;
e) an enzyme catalyst having perhydrolase activity;
f) a rheology modifier; and
g) a quaternary ammonium compound;
wherein, upon combining said components of (a) through (g), a peroxyacid is formed.

### BRIEF DESCRIPTION OF BIOLOGICAL SEQUENCES

The following sequences comply with 37 C.F.R. §1.821-1.825 ("Requirements for Patent Applications Containing Nucleotide Sequences and/or Amino Acid Sequence Disclosures-the Sequence Rules") and are consistent with World Intellectual Property Organization (WIPO) Standard ST.25 (1998) and the sequence listing requirements of the European Patent Convention (EPC) and the Patent Cooperation Treaty (PCT) Rules 5.2 and 49.5(a-bis), and Section 208 and Annex C of the Administrative Instructions. The symbols and format used for nucleotide and amino acid sequence data comply with the rules set forth in 37 C.F.R. §1.822.

SEQ ID NO: 1 is the amino acid sequence of the cephalosporin C deacetylase from *Bacillus subtilis* ATCC31954.

SEQ ID NO: 2 is the amino acid sequence of the cephalosporin C deacetylase from *Bacillus subtilis* subsp. *subtilis* strain 168 (GenBank Accession # NP_388200).

SEQ ID NO: 3 is the amino acid sequence of the cephalosporin C deacetylase from *Bacillus subtilis* ATCC6633 (GenBank Accession #YP_077621.1).

SEQ ID NO: 4 is the amino acid sequence of the cephalosporin C deacetylase from *Bacillus licheniformis* ATCC14580 (GenBank Accession # YP_077621.1).

SEQ ID NO: 5 is the amino acid sequence of the acetyl xylan esterase from *Bacillus pumilis* (GenBank Accession # CAB76451.2).

SEQ ID NO: 6 is the amino acid sequence of the acetyl xylan esterase from *Clostridium thermocellum* ATCC27405 (GenBank Accession # ZP_00504991).

SEQ ID NO: 7 is the amino acid sequence of the acetyl xylan esterase from *Thermotoga neapolitana* GenBank Accession # AAB70869.1).

SEQ ID NO: 8 is the amino acid sequence of the acetyl xylan esterase from *Thermotoga maritima* MSB8 (GenBank Accession # NP_227893.1).

SEQ ID NO: 9 is the amino acid sequence of the acetyl xylan esterase from *Thermoanaerobacterium* sp. (GenBank Accession # AAB68821.1).

SEQ ID NO: 10 is the amino acid sequence of the cephalosporin C deacetylase from *Bacillus* sp. NRRL B14911 (GenBank Accession # ZP_01168674).

SEQ ID NO: 11 is the amino acid sequence of the acetyl xylan esterase from *Bacillus halodurans* C-125 (GenBank Accession # NP_244192).

SEQ ID NO: 12 is the amino acid sequence of the cephalosporin C deacetylase from *Bacillus clausii* KSM-K16.

SEQ ID NO: 13 is the amino acid sequence of the acetyl xylan esterase from *Thermoanaerobacterium saccharolyticum.*

SEQ ID NO: 14 is the amino acid sequence of the acetyl xylan esterase from *Thermotoga lettingae.*

SEQ ID NO: 15 is the amino acid sequence of the acetyl xylan esterase from *Thermotoga petrophilia.*

SEQ ID NO: 16 is the amino acid sequence of the acetyl xylan esterase from *Thermotoga* sp. RQ2"a".

SEQ ID NO: 17 is the amino acid sequence of the acetyl xylan esterase from *Thermotoga* sp. RQ2"b".

SEQ ID NO: 18 is the nucleotide sequence for primer f27405 used in Example 1.

SEQ ID NO: 19 is the nucleotide sequence for primer r27405 used in Example 1.

SEQ ID NO: 20 is the nucleic acid sequence of the nucleic acid product amplified by SEQ ID NO: 18 and 19 that was used to prepare plasmid pSW196 used in Example 1.

SEQ ID NO: 21 is the contiguous amino acid sequence of the 4 conserved motifs found in CE-7 carbohydrate esterases.

SEQ ID NO: 22 is the nucleotide sequences for the forward primer [TAACTGCAGT AAGGAGGAAT AGGACATGGC CTTCTTCGAT TTACCCACTC] used in Example 8.

SEQ ID NO: 23 is the nucleotide sequence for the reverse primer [TGATCTAGAT TAGCCTTTCT CAAATAGTTT TTTCAAGA] used in Example 8.

SEQ ID NO: 24 is the nucleic acid sequence of the nucleic acid product amplified by SEQ ID NO: 22 and 23 that was used to prepare plasmid pSW207.

SEQ ID NO: 25 is the nucleotide sequences for the forward primer [TAAGAATTCT AAGGAATAGG ACATGGCGTT TCTTCGACCT GCCTCTG] used in Example 8.

SEQ ID NO: 26 is the nucleotide sequences for the reverse primer [AAACTGCAGT TAGCCCTTCT CAAACAGTTT CTTCAG] used in Example 8.

SEQ ID NO: 27 the nucleic acid sequence of the nucleic acid product amplified by SEQ ID NO: 25 and 26 that was used to prepare plasmid pSW228 used in Example 8.

SEQ ID NO: 28 is the nucleotide sequence for forward primer Tma_C277Sf [GGACAACATCTCACCTCCTTCTA] used in Example 9.

SEQ ID NO: 29 is the forward reverse primer Tma_C227Sr [TAGAAGGAGGTGAGATGTTGTCC] used in Example 9.

SEQ ID NO: 30 is the codon optimized sequence of a *T. maritima* acetyl xylan esterase in plasmid pSW228 used in Example 9.

### DETAILED DESCRIPTION OF THE INVENTION

Unless stated otherwise, all percentages, parts, ratios, etc., are by weight. Trademarks are shown in upper case. Further, when an amount, concentration, or other value is disclosed as either a range, preferred range or a list of preferred upper and lower values, such disclosure is to have the same effect as if each individual value within the specified range - and any range obtained from a combination of any two individual values within the disclosed range - has been specifically disclosed, even if the individual values are not uniquely or individually disclosed herein. Where a range of numerical values is recited herein, unless otherwise stated, the range is intended to include the endpoints thereof, and all integers and fractions within the range. Unless specified, it is not intended that the scope of the invention be limited to the specific values recited when defining a range.

For clarity, terms used herein are to be understood as described herein or as such term would be understood by one of ordinary skill in the art of the invention. Additional explanation of certain terms used herein, are provided below:
"Peracid" is a carboxylic acid in which an acidic -OH group has been replaced by an -OOH group. As used herein, "peracid" is synonymous with peroxyacid, peroxy acid, percarboxylic acid and peroxoic acid. As is commonly known, peracid includes peracetic acid. The term "peracetic acid" or "PAA", as used herein, is synonymous with peroxyacetic acid, ethaneperoxoic acid and all other synonyms of CAS Registry Number 79-21-0.
"Triacetin" is synonymous with glycerin triacetate; glycerol triacetate; glyceryl triacetate, 1,2,3-triacetoxypropane, 1,2,3-propanetriol triacetate and all other synonyms of CAS Registry Number 102-76-1.
"Peroxygen source" refers to any peroxide compound or compound containing hydrogen peroxide that may be released in solution.
"Shear rate" refers to the velocity gradient in a flowing material and is measured in SI units of reciprocal seconds (s⁻¹).
"Shear-thinning properties" or "pseudoplastic properties" refers to a fluid that exhibits a decrease in viscosity with an increase in shear rate.
"Readily removable" refers to easily removing the coatings formed after application of the liquid coating composition to the surface of interest, but not so easily removed as to be removed by inadvertent physical contact.
"Non-volatile" refers to a compound whose vapor pressure at 25 °C is below 1000 Pascals.
"Inert solvent or aqueous solvent" refers to water or any other solvent that facilitates application of the coating composition to the locus. An aqueous solvent may also be employed to rinse coated surfaces to remove the coating as needed.
"Film-forming agent" or "water soluble or water dispersible coating agent", which may be used interchangeably herein, refer to agents that form a film and are employed to provide protective coating to the surface of interest. These agents are either water soluble or water dispersible. These agents are described in further detail below.
"Metal chelator" or "sequestrant" refers to agents that bind metals or metal-containing impurities and prevent their decomposition catalysis of hydrogen peroxide or peroxyacids.
"Rheology modifier" or "rheology agent" refers to compounds that increase viscosity and/or provide shear-thinning properties to a composition and cause the aqueous treatment or coating composition to cling to the surface of interest.
"wt%" refers to the weight percent relative to the total weight of the solution or dispersion.
"Liquid coating composition" refers to the composition comprising at least a water soluble film-forming agent, an inert solvent, a carboxylic acid ester substrate, a peroxygen source, and an enzyme catalyst having perhydrolase activity, wherein at least one peroxyacid is produced.
"Antimicrobial" or "antimicrobial properties" as used herein refers to the ability of an agent of killing microorganisms, blocking or preventing microbial contamination (such as a forming a barrier), or suppressing or preventing growth of microorganisms, trapping microorganisms for killing, preventing biofilm formation or eliminating/removing biofilm.
"Antimicrobial agent" as used herein refers to a compound or substance having antimicrobial properties.
"Microorganism" is meant to include any organism comprised of the phylogenetic domains of bacteria and archaea, as well as unicellular (e.g., yeasts) and filamentous (e.g., molds) fungi, unicellular and filamentous algae, unicellular and multicellular parasites, viruses, virinos and viroids.
"Biocide", as used herein, refers to a chemical agent, typically broad spectrum, which inactivates or destroys microorganisms. A chemical agent that exhibits the ability to inactivate or destroy microorganisms is described as having "biocidal" activity.
"Biofilm" refers to a structured community of microorganisms encapsulated within a self-developed polymeric matrix and adherent to a living or inert surface.
"Drying" refers to a process by which the inert solvent or any other liquid present in the formulation is removed by evaporation.
"Disinfectant" means an agent which provides antimicrobial activity. According to an official disinfectant test, a disinfectant is a chemical that kills 99.9% of the specific test microorganisms in 10 minutes under the conditions of the test. (Germicidal and Detergent Sanitizing Action of Disinfectants, Official Methods of Analysis of the Association of Official Analytical Chemists, paragraph 960.09 and applicable sections, 15th Edition, 1990 (EPA Guideline 91-2)).
"One unit of enzyme activity" or "one unit of activity" or "U" is defined as the amount of perhydrolase activity required for the production of 1 micromole of peracid product per minute at a specified temperature.
"Half-time" or "τ" is defined as the time at which the PAA concentration has decayed to half its maximum value.
"Enzyme catalyst" and "perhydrolase catalyst" are used interchangeably and refer to a catalyst comprising an enzyme having perhydrolysis activity, that is, an enzyme that exhibits the ability to catalyze the reaction between a peroxide source and a substrate to form a peracid and may be in the form of a whole microbial cell, permeabilized microbial cell(s), one or more cell components of a microbial cell extract, partially purified enzyme, or purified enzyme.
"Acetyl xylan esterase" refers to an enzyme (E.C. 3.1.1.72; AXEs) that catalyzes the deacetylation of acetylated xylans and other acetylated saccharides and also has significant perhydrolytic activity suitable for producing percarboxylic acids from carboxylic acid esters.
"Gene" refers to a nucleic acid molecule that expresses a specific protein, including regulatory sequences preceding (5' non-coding sequences) and following (3' non-coding sequences) the coding sequence.
"Isolated nucleic acid molecule" and "isolated nucleic acid fragment" may be used interchangeably and refer to a polymer of RNA or DNA that is single- or double-stranded, optionally containing synthetic, non-natural or altered nucleotide bases. An isolated nucleic acid molecule in the form of a polymer of DNA may be comprised of one or more segments of cDNA, genomic DNA or synthetic DNA.
"Expression" refers to the transcription and stable accumulation of sense (mRNA) or antisense RNA derived from the nucleic acid molecule of the invention. Expression may also refer to translation of mRNA into a polypeptide.
"Transformation" refers to the transfer of a nucleic acid molecule into the genome of a host organism, resulting in genetically stable inheritance. In the present invention, the host cell's genome includes chromosomal and extrachromosomal (e.g., plasmid) genes. Host organisms containing the transformed nucleic acid molecules are referred to as "transgenic" or "recombinant" or "transformed" organisms.
"ATCC" refers to the American Type Culture Collection. A repository of microorganisms located in Virginia, USA.
"First antimicrobial agent" refers to the antimicrobial agent generated *in situ* in coating compositions according to the methods of the invention. In a preferred embodiment, the first antimicrobial agent is a peroxyacid.
"Second antimicrobial agent" refers to an additional antimicrobial agent other than a peroxyacid that is present in the coating composition. According to the current method, this agent is not generated *in situ.* The second antimicrobial agent is a quaternary ammonium compound.
"RGQ" refers to a highly conserved motif of amino acid residues at positions 118-120 of SEQ ID NO: 1 that is diagnostic for the CE-7 enzyme family.
"GXSQG" refers to a highly conserved motif of amino acid residues at positions 179-183 of SEQ ID NO: 1 that is diagnostic for the CE-7 enzyme family.
"HE" refers to a highly conserved motif of amino acid residues at positions 298-299 of SEQ ID NO:1 that is diagnostic for the CE-7 enzyme family.
"LXD" refers to a highly conserved motif of amino acid residues at positions 267-269 of SEQ ID NO: 1 that is diagnostic for the CE-7 enzyme family.
"Substantially similar" refers to nucleic acid molecules wherein changes in one or more nucleotide bases results in the addition, substitution, or deletion of one or more amino acids, but does not affect the functional properties (i.e., perhydrolytic activity) of the protein encoded by the DNA sequence.
"Signature motif, "CE-7 signature motif, and "diagnostic motif refer to conserved structures shared among a family of enzymes having a defined activity such as perhydrolase activity.

In one aspect, the present invention is a method comprising the use of perhydrolases in combination with a peroxygen source and a carboxylic acid ester to form a peroxyacid. Perhydrolases are enzymes that catalyze the reaction of a peroxygen source, (e.g., hydrogen peroxide) with a selected substrate to form a peracid. As indicated above, enzymes possessing perhydrolase activity include, but are not limited to lipases, esterases, proteases, non-heme haloperoxidases, and carbohydrate esterases of the CE-7 family, such as acetyl xylan esterases or cephalosporin C deacetylases.

Application of these enzymes for their perhydrolytic activity has been described in commonly owned U.S. Patent Application 2009/0005590A1, U.S. Patent Application 2008/0176299A1 and U.S. Patent Application 2007/042924A1, and U.S. Patent Application 61/102,520, which are herein incorporated by reference in their entirety.

Certain enzymes belonging to the structural family of CE-7 esterases, such as cephalosporin C deacetylase (CAHs) and acetyl xylan esterases (AXEs), exhibit significant perhydrolysis activity for converting carboxylic acid esters in the presence of an inorganic source of peroxygen (e.g., hydrogen peroxide, sodium percarbonate, sodium perborate) into peracids at concentrations sufficient for use as a disinfectant and/or bleaching agent. The system achieves efficiency by producing the peracid in high concentrations without requiring a high concentration of peroxygen (e.g., as described in the commonly owned U.S. Application Publication 2008/0176299A1).

Perhydrolase catalysts may be used as whole microbial cell, permeabilized microbial cell(s), one or more cell components of a microbial cell extract, partially purified enzyme, or purified enzyme. The enzyme catalyst may also be chemically modified (e.g., by PEGylation or by reaction with cross-linking reagents). The perhydrolase catalyst may also be immobilized on a soluble or insoluble support using methods well-known to those skilled in the art.

### Carbohydrates esterases of the CE-7 family

Members of the CE-7 family, cephalosporin C deacetylases (E.C. 3.1.1.41; systematic name cephalosporin C acetylhydrolases; CAHs) and acetyl xylan esterases (E.C. 3.1.1.72) are found in plants, fungi (e.g., *Cephalosporidium acremonium*), yeasts (e.g., *Rhodosporidium toruloides, Rhodotorula glutinis*), and bacteria such as *Thermoanaerobacterium* sp.; *Norcardia lactamdurans,* and various members of the genus *Bacillus* (Politino et al., Appl. Environ. Microbiol, 63:4807-4811, 1997); Sakai et al., J. Ferment. Bioeng. 85:53-57, 1998); Lorenz, W. and Wiegel, J., J. Bacteriol.,179:5436-5441, 1997); Cardoza etal., Appl. Microbiol. Biotechnol., 54:406-412, 2000); Mitshushima *et al., supra,* Abbott, B. and Fukuda, D., Appl. Microbiol. 30:413-419, 1975); Vincent et al., J. Mol. Biol., 330:593-606, 2003, Takami et al., NAR, 28:4317-4331, 2000); Rey et al., Genome Biol., 5: article 77, 2004); Degrassi et al., Microbiology., 146:1585-1591, 2000); U.S. Patent 6,645,233; U.S. Patent 5,281,525; U.S. Patent 5,338,676; and WO 99/03984. A non-comprehensive list of CE-7 family members having significant homology to SEQ ID NO: 1 is provided in SEQ ID NOs 1-18.

As members of the CE-7 family of carbohydrate esterases (Vincent *et al., supra*), both cephalosporin C deacetylases (E.C. 3.1.1.41) and acetyl xylan esterases (E.C. 3.1.1.72) hydrolyze the acetyl group on acetylated polymeric xylan, acetylated xylose, acetylated glucose, and acetylated cellulose. As such, acetylated carbohydrates may be suitable substrates for generating percarboxylic acids using the present process (i.e., in the presence of a peroxygen source). Examples of acetylated carbohydrates include, but are not limited to acetylated glucose (such as glucose pentaacetate), acetylated mannose, acetylated xylose (such as xylose tetraacetate), and acetylated cellulose.

Some enzymes, such as those suitable for application in the current method possess signature motifs that can be used to define and/or identify the family of structurally related enzymes having similar enzymatic activity for a defined family of substrates. The signature motif can be a single contiguous amino acid sequence or a collection of discontinuous, conserved motifs that together form the signature motif. Typically, the conserved motif(s) is represented by an amino acid sequence. As described herein, the present perhydrolases belong to the family of CE-7 carbohydrate esterases. This family of enzymes can be defined by the presence of signature motifs as described below.

The CE-7 family members are unusual in that they typically exhibit ester hydrolysis activity for both acetylated xylooliogsaccharides and cephalosporin C, suggesting that the CE-7 family represents a single class of proteins with a multifunctional deacetylase activity against a range of small substrates (Vincent, *et al., supra*). Vincent *et al.* describe the structural similarity among the members of this family and define signature sequence motifs characteristic of the CE-7 family (SEQ ID NO: 21). A CE-7 signature motif that aligns with a reference sequence SEQ ID NO: 1 using CLUSTALW has several highly conserved motifs that are diagnostic for the family, which include residues 118-120 (RGQ) of SEQ ID NO: 1, residues 179-183 (GXSQG) of SEQ ID NO: 1, residues 298-299 (HE) of SEQ ID NO: 1 and residues 267-269 (LXD) of SEQ ID NO: 1. Multiple alignment of the amino acid sequences may be performed using the Clustal method of alignment Higgins and Sharp, CABIOS, 5:151-153 (1989) with the default parameters (GAP PENALTY=10, GAP LENGTH PENALTY=10). Default parameters for pairwise alignments using the Clustal method are typically KTUPLE 1, GAP PENALTY=3, WINDOW=5 and DIAGONALS SAVED=5.

Perhydrolase enzymes having amino acid sequences that are least 50%, preferably at least 60%, more preferably at least 70%, even more preferably at least 80%, yet even more preferably at least 90%, still yet even more preferably at least 95%, and most preferably at least 99% to the sequence reported herein wherein the resulting enzyme retains the present functional properties (i.e., perhydrolytic activity) are suitable for the present method. The term substantially similar" may also refer to an enzyme having perhydrolytic activity encoded by nucleic acid molecules that hybridize under stringent conditions to the nucleic acid molecules of the amino acid sequences reported herein (e.g., SEQ ID NOs:1-17 and 30). It is therefore understood that the invention encompasses more than the specific exemplary sequences.

A comparison of the overall percent identity among perhydrolases exemplified herein indicates that enzymes having as little as 33% identity to SEQ ID NO: 1 (while retaining the signature motif) exhibit significant perhydrolase activity and are structurally classified as CE-7 carbohydrate esterases.

In one embodiment, one of the perhydrolase catalysts useful for this method comprises an enzyme having a CE-7 signature motif that aligns with a reference sequence SEQ ID NO: 1 using CLUSTALW, said signature motifs comprising:
i) an RGQ motif at amino acid positions 118-120 of SEQ ID NO:1;
ii) a GXSQG motif at amino acid positions 179-183 of SEQ ID NO:1; and
iii) an HE motif at amino acid positions 298-299 of SEQ ID NO:1; wherein said enzyme also comprises at least 30% amino acid identity to SEQ ID NO: 1.

In a further embodiment, the signature motif additionally comprises a fourth conserved motif defined as an LXD motif at amino acid residues 267-269 when aligned to reference sequence SEQ ID NO: 1 using CLUSTALW.

In another embodiment, the perhydrolase catalyst comprises an enzyme having perhydrolase activity selected from the group consisting of SEQ ID NOs: 1-17 and 30 or a substantially similar enzyme having perhydrolase activity derived by substituting, deleting or adding one or more amino acids to said amino acid sequence.

In another embodiment, substantially similar enzyme having perhydrolase activity is at least 90%, 91 %, 92%, 93%, 94%, 95%, 96%, 97%, 98%, or 99% identical to one or more amino acid sequences selected from the group consisting of SEQ ID NOs: 1-17 and 30.

In another embodiment, the perhydrolase catalyst comprises an enzyme having at least 30%, preferably at least 36%, amino acid identity to a contiguous signature motif as defined as SEQ ID NO: 1 wherein the signature motifs described above (e.g., RGQ, GXSQG, and HE, and optionally LXD) are conserved.

In yet further embodiments, the perhydrolase system of the present method is used in conjunction with additional enzymes, including but not limited to proteases, amylases, etc. Indeed, it is contemplated that various enzymes will find use in conjunction with the present invention, including but not limited to microbial cell wall-degrading and glycoprotein-degrading enzymes, lysozyme, hemicellulases, peroxidases, proteases, cellulases, xylanases, lipases, phospholipases, esterases, cutinases, pectinases, keratinases, reductases, oxidases, phenoloxidases, lipoxygenases, ligninases, pullulanases, tannases, pentosanases, malanases, 13-glucanases, arabinosidases, hyaluronidase, chondroitinase, laccase, endoglucanases, PNGases, amylases, etc., as well as or mixtures thereof. In some embodiments, enzyme stabilizers find use in the present method. It is contemplated that by using combinations of enzymes, there will be a concurrent reduction in the amount of chemicals needed in the method.

Although in one preferred embodiment, the perhydrolysis activity of the structurally related members of the carbohydrate family esterase family CE-7 have been used for the current method, any perhydrolase enzyme obtained from any source, which in the presence of hydrogen peroxide converts an ester substrate into mostly peracid, may also be used in the present method.

Additionally, some hydrolase enzymes may be used in the current method. Suitable hydrolases for this purpose include: (1) proteases belonging to the peptidyl-peptide hydrolase class (e.g., pepsin, pepsin B, rennin, trypsin, chymotrypsin A, chymotrypsin B, elastase, enterokinase, cathepsin C, papain, chymopapain, ficin, thrombin, fibrinolysin, renin, subtilisin, aspergillopeptidase A, collagenase, clostridiopeptidase B, kallikrein, gastrisin, cathepsin D, bromelin, keratinase, chymotrypsin C, pepsin C, aspergillopeptidase B, urokinase, carboxypeptidase A and B, and aminopeptidase); (2) carboxylate ester hydrolase including carboxyl esterase, lipase, pectin esterase, and chlorophyllase; and (3) enzymes having high perhydrolysis to hydrolysis ratios. Especially effective among them are lipases, as well as esterases that exhibit high perhydrolysis to hydrolysis ratios, as well as protein engineered esterases, cutinases, and lipases, using the primary, secondary, tertiary, and/or quaternary structural features of the perhydrolases of the present method.

### Suitable Enzymatic reaction mixture

"Suitable enzymatic reaction mixture", "components suitable for *in situ* generation of a peracid", "suitable reaction components", and "suitable aqueous reaction mixture" are used interchangeably herein and refer to the materials and the solution in which the reactants and enzyme catalyst come into contact. The components of the suitable aqueous reaction mixture are provided herein and those skilled in the art appreciate the range of component variations suitable for this process.

In one embodiment, the suitable enzymatic reaction mixture produces peracid *in situ* upon combining the reaction components. As such, the reaction components may be provided as a multicomponent system wherein one or more of the reaction components remains separated until use. The design of systems for combining multiple active components are known in the art and generally will depend upon the physical form of the individual reaction components. For example, multiple active fluids (liquid-liquid) systems typically use multichamber dispenser bottles or two-phase systems (U.S. Patent Application Pub. No. 2005/0139608; U.S. Patent No. 5,398,846; U.S. Patent No. 5,624,634; U.S. Patent No. 6,391,840; EP Patent No. 0807156B1; U.S. Patent Appl. Pub. No. 2005/0008526; and PCT Publication No. WO 00/11713A1) such as found in some bleaching applications wherein the desired bleaching agent is produced upon mixing the reactive fluids. Other forms of multicomponent systems used to generate peracid may include, but are not limited to those designed for one or more solid components or combinations of solid-liquid components, such as powders (e.g., many commercially available bleaching compositions, U.S. Patent No. 5,116,575), multi-layered tablets (U.S. Patent No. 6,210,639), water dissolvable packets having multiple compartments (U.S. Patent No. 6,995,125) and solid agglomerates that react upon the addition of water (U.S. Patent No. 6,319,888).

In another aspect, a suitable system for combining reactive components is use of a twin-nozzle bottle as disclosed in U.S. Patent Application Pub. No. 2005/014427. An alternative device suitable for use with the method of the invention is a dual compartment trigger-activated fluid dispenser as disclosed in EP Patent No. 0715899B1.

In another aspect, a suitable system for mixing the suitable reaction components may be a container with a membrane separating the reactive components where upon rupturing the membrane by mechanical force, the reaction components are combined before use. In another aspect, a suitable device may be a bag-within-a-bag.

In another aspect, the means for combining or mixing the suitable enzymatic reaction mixture for use according to the methods of this invention include systems, devices, containers, kits, multi-packs, bags, dispensers, and applicators known to those skilled in the art that are used to keep reactive components separated before use.

In another aspect, the enzymatic perhydrolysis product composition may contain additional components that provide desirable functionality. These additional components include, but are not limited to buffers, detergent builders, thickening agents, emulsifiers, surfactants, wetting agents, corrosion inhibitors (e.g., benzotriazole), enzyme stabilizers, and peroxide stabilizers (e.g., metal ion chelating agents). Many of the additional components are well known in the detergent industry (see for example U.S. Patent No. 5,932,532; hereby incorporated by reference). Examples of emulsifiers include, but are not limited to polyvinyl alcohol or polyvinylpyrrolidone. Examples of thickening agents include, but are not limited to LAPONITE® RD, corn starch, PVP, CARBOWAX®, CARBOPOL®, CABOSIL®, polysorbate 20, PVA, and lecithin. Examples of buffering systems include, but are not limited to sodium phosphate monobasic/sodium phosphate dibasic; sulfamic acid/triethanolamine; citric acid/triethanolamine; tartaric acid/triethanolamine; succinic acid/triethanolamine; acetic acid/triethanolamine; and sodium bicarbonate/sodium carbonate. Examples of surfactants include, but are not limited to a) non-ionic surfactants such as block copolymers of ethylene oxide or propylene oxide, ethoxylated or propoxylated linear and branched primary and secondary alcohols, and aliphatic phosphine oxides b) cationic surfactants such as quaternary ammonium compounds, particularly quaternary ammonium compounds having a C8-C20 alkyl group bound to a nitrogen atom additionally bound to three C1-C2 alkyl groups, c) anionic surfactants such as alkane carboxylic acids (e.g., C8-C20 fatty acids), alkyl phosphonates, alkane sulfonates (e.g., sodium dodecylsulphate "SDS") or linear or branched alkyl benzene sulfonates, alkene sulfonates and d) amphoteric and zwitterionic surfactants such as aminocarboxylic acids, aminodicarboxylic acids, alkybetaines, and mixtures thereof. Additional components may include fragrances, dyes, stabilizers of hydrogen peroxide (e.g., metal chelators such as 1-hydroxyethylidene-1,1-diphosphonic acid (DEQUEST® 2010, Solutia Inc., St. Louis, Mo. and ethylenediaminetetraacetic acid (EDTA)), TURPINAL® SL, DEQUEST® 0520, DEQUEST® 0531, stabilizers of enzyme activity (e.g., polyethylene glycol, PEG), and detergent builders.

In another aspect, the enzymatic perhydrolysis product may be premixed to generate the desired concentration of peroxycarboxylic acid prior to contacting the surface or inanimate object to be disinfected.

In another aspect, the enzymatic perhydrolysis product may be premixed to generate the desired concentration of peroxycarboxylic acid and may be optionally diluted with water or a solution predominantly comprised of water, to produce a mixture with the desired lower concentration of peracid.

In another aspect, the enzymatic perhydrolysis product is not premixed to generate the desired concentration of peroxycarboxylic acid prior to contacting the surface or inanimate object to be disinfected, but instead, the components of the reaction mixture that generate the desired concentration of percarboxylic acid are contacted with the surface or inanimate object to be disinfected, generating the desired concentration of peroxycarboxylic acid. In some embodiments, the components of the reaction mixture combine or mix at the locus. In some embodiments, the reaction components are delivered or applied to the locus and subsequently mix or combine to generate the desired peroxyacid.

The concentration of the catalyst in the aqueous reaction mixture depends on the specific catalytic activity of the catalyst, and is chosen to obtain the desired rate of reaction. The concentration of catalyst in perhydrolysis reactions typically ranges from 0.0001 mg to 10 mg per mL of total reaction volume, preferably from 0.0005 mg to 2.0 mg per mL. The catalyst may also be immobilized on a soluble or insoluble support using methods well-known to those skilled in the art; see for example, Immobilization of Enzymes and Cells; Gordon F. Bickerstaff, Editor; Humana Press, Totowa, N.J., USA, 1997. The use of immobilized catalysts permits the recovery and reuse of the catalyst in subsequent reactions. The enzyme catalyst may be in the form of whole microbial cells, permeabilized microbial cells, microbial cell extracts, partially purified or purified enzymes, and mixtures thereof.

In one aspect, the concentration of peracid generated by the combination of chemical perhydrolysis and enzymatic perhydrolysis of the carboxylic acid ester is sufficient to provide an effective concentration of peracid for the desired application at a desired pH. In another aspect, the present method provides combinations of enzymes and enzyme substrates to produce the desired effective concentration of peracid, where, in the absence of added enzyme, there is a significantly lower concentration of peracid produced. Although there may in some cases be substantial chemical perhydrolysis of the enzyme substrate by direct chemical reaction of inorganic peroxide with the enzyme substrate, there may not be a sufficient concentration of peracid generated to provide an effective concentration of peracid in the desired applications, and a significant increase in total peracid concentration is achieved by the addition of an appropriate perhydrolase catalyst to the reaction mixture.

The concentration of peracid generated (e.g., peracetic acid) by the perhydrolysis of at least one carboxylic acid ester is at least about 2 ppm, preferably at least 20 ppm, preferably at least 100 ppm, more preferably at least about 200 ppm peracid, more preferably at least 300 ppm, more preferably at least 500 ppm, more preferably at least 700 ppm, more preferably at least about 1000 ppm peracid, most preferably at least 2000 ppm peracid within 10 minutes, preferably within 5 minutes, and most preferably within 1 minute of initiating the perhydrolysis reaction. In another aspect, the concentration of peracid generated by the perhydrolysis catalyst is at least about 3000 ppm, preferably at least 5000 ppm, more preferably 8000 ppm, most preferably at least 10000 ppm within one hour, more preferably within 30 minutes, most preferably within 5 minutes of initiating the perhydrolysis reaction. In one aspect, the reaction time required to produce the desired concentration of peracid is not greater than about two hours, preferably not greater than about 30 minutes, more preferably not greater than about 10 minutes, even more preferably not greater than about 5 minutes, and most preferably in about 1 minute or less. In other aspects, a hard surface or inanimate object contaminated with a concentration of a microbial population is contacted with the peracid formed in accordance with the processes described herein within about 1 minute to about 168 hours of combining said reaction components, or within about 1 minute to about 48 hours, or within about 1 minute to 8 hours, or within 1 minute to 2 hours of combining said reaction components, or any such time interval therein.

The temperature of the reaction is chosen to control both the reaction rate and the stability of the enzyme catalyst activity. The temperature of the reaction may range from just above the freezing point of the reaction mixture (approximately 0 °C) to about 75 °C, with a preferred range of reaction temperature of from about 5 °C to about 55 °C.

The pH of the final reaction mixture containing peracid is from about 5 to about 9.5, preferably from about 6 to about 8, more preferably from about 6.5 to about 7.5. In another embodiment, the pH of the reaction mixture is acidic (pH<7). The pH of the reaction mixture during the reaction or after completion of the reaction may optionally be controlled by the addition of a suitable buffer, including, but not limited to phosphate, pyrophosphate, bicarbonate, acetate, or citrate. The concentration of buffer, when employed, is typically from 0.1 mM to 1.0 M, preferably from 1 mM to 300 mM, most preferably from 10 mM to 100 mM.

In one embodiment of the present invention, at least some of the components are premixed. Premixed refers to the combination of one or more individual components of the coating composition, but not all of the desired components of the film-forming composition are combined. Additional components of the coating composition must be added to the premixed component prior to final application or use at a particular locus. In another embodiment, two premixed components must be combined to form the desired coating composition according to the methods of this invention.

The components of the coating composition of the present invention can be contained in a multicompartment containment system, also referred to herein as a multicompartment system. A multicompartment system refers to the means of keeping the two or more reactive components of the multicomponent system coating system separated before use. In one aspect, a multicompartment system comprises at least two compartments and may contain a multi-chamber dispenser bottle or a two-phase system used to combine reactive compounds in liquid form. In another aspect, powders, multi-layered tablets, or water dissolvable packets having multiple compartments, can be used for compounds in solid form or a combination of solid and liquid forms. In another aspect, any kind of system, device, container, package, bag, kit, multi-pack, dispenser, or applicator that is used to keep reactive components separated before use can be used according to the methods of this method.

Coatings of the present invention can be removed, after application, using an aqueous solution. An aqueous solution effective for coating removal is any solution containing 60 to 100 wt% water, the remaining components being dissolved or dispersed components. Dissolved or dispersed components may include but are not limited to solvents such as alcohols, solubilizing agents, surfactants, salts, chelators, acids and bases.

Antimicrobial coatings of the present invention are durable coatings. Durable in the context of the present invention is the characteristic or property of the applied antimicrobial coating, after drying, of remaining on the surface under typical use conditions until its removal is purposely initiated or allowed to occur. Use conditions are the ambient environmental conditions prevalent during the period the coating remains on the target surface for the application areas of this invention and may include inadvertent contact with water.

It can be preferable that the antimicrobial coatings applied to target surfaces be continuous or substantially continuous. Continuous, or substantially continuous, in the context of the present invention refers to a coating that covers the target surface without voids, breaks, uncovered areas, or coating defects that leave unintentionally exposed surface areas.

Coatings of the present invention are preferably homogeneous or substantially homogeneous. Homogeneous, or substantially homogenous, in the context of the present invention refers to a coating with only small thickness variations across the coating surface, with the standard deviation of the coating thickness across a coated surface being in the range of 0-40% of the coating thickness. Coatings that are not homogeneous or not substantially homogenous will not provide even antimicrobial and removal properties across the whole surface the coating is applied to and typically the appearance of inhomogeneous coatings is considered unattractive for many applications.

Pseudoplastic index or shear thinning index (STI) provides an indication of the resistance to sagging and dripping that the composition may exhibit. The value recorded at the lower shear rate is divided by the value at the higher shear rate to obtain the STI. Generally, the higher the STI, the higher the resistance to sagging and dripping the coating material will have. In this disclosure the shear thinning index is defined as the ratio of the viscosity measured at a first shear rate and a second shear rate, wherein said second shear rate is 10 times the value of said first shear rate. Alternatively, if the shear rates in s⁻¹ are not known, the shear-thinning index can also be calculated by measuring the viscosity using a rotational viscometer at a first rpm and a second rpm, wherein said second rpm is 10 times the value of said first rpm. Without being limited to specific first and second shear rates used or to specific first and second rpm values used to calculate the STI, in the Examples, the shear thinning index was calculated as the ratio of the viscosities measured at 1 rpm and 10 rpm.

The antimicrobial coating of the present invention can be used as a sanitizer. As defined herein, a sanitizer is a chemical or chemical mixture that can be either (i) a food-contact sanitizer if the intention is to control microorganisms on surfaces which actually or potentially come in contact with food, or (ii) a non-food-contact sanitizer if the surfaces are not indented to come into contact with food. As defined herein, a food-contact sanitizer kills at least 99.999% of the specific test microorganisms in 30 seconds under the conditions of the test method according to EPA policy DIS/TSS-4: "Efficacy data requirements - Sanitizing rises for previously cleaned food-contact surfaces", United States Environmental Protection Agency, January 30, 1979. A non-food contact sanitizer as defined herein kills at least 99.9% of the specific test microorganisms in 5 minutes under the conditions of the method according to ASTM standard E 1153-03: "Standard Test Method for Efficacy of Sanitizers Recommended for Inanimate Non-Food Contact Surfaces", edition April 10, 2003 and published July 2003.

A coating composition of the present invention can exhibit a residual antimicrobial efficacy, and exhibit self-sanitizing properties. "Residual antimicrobial efficacy" or "self-sanitizing properties" refers to the property of coatings formed as described herein which remain antimicrobially active after drying. The antimicrobial activity of dry coatings can be measured using the residual self-sanitizing (RSS) test.

There has been a longstanding need for antimicrobial agents having improved antimicrobial efficacy and improved speed of action. The specific requirements for such agents vary according to the intended application (e.g., sanitizer, disinfectant, sterilant, aseptic packaging treatment, etc.) and the applicable public health requirements. For example, as set out in Germicidal and Detergent Sanitizing Action of Disinfectants, Official Methods of Analysis of the Association of Official Analytical Chemists, paragraph 960.09 and applicable sections, 15th Edition, 1990 (EPA Guideline 91-2), a sanitizer should provide a 99.999% reduction (5-log order reduction) within 30 seconds at room temperature (23-27 °C), against several test organisms.

The *in situ* enzymatically produced peracid-based, removable antimicrobial coating composition useful for the invention may be used as a replacement or a supplement for standard sanitation products (such as diluted quaternary ammonium compound solutions, peracid foams, and the like), and may be used for daily sanitation as protective coatings for equipment in use or not-in use, as well as for longer term protection (weeks or months).

The *in situ* enzymatically produced peracid-based, removable antimicrobial coating composition of this disclosure provides several advantages including, but not limited to killing both free-living or planktonic microorganisms and microorganisms harbored in biofilms, reducing or preventing the growth of microorganisms by preventing the formation of biofilms and by trapping microorganisms in, beneath or otherwise in contact with the coating.

The coating composition disclosed herein may be modified by formulating the composition with rheology modifiers to coat vertical, inclined, geometrically complex or hard-to-reach surfaces. This enables application of the antimicrobial agent to surfaces on or in equipment otherwise not accessible by application of conventional antimicrobial solutions with traditional shear-viscosity profiles and viscosities below about 0.01 Pascal-seconds at 25 °C. Horizontal and vertical surfaces may be covered with a thin layer of protective coating without waste of antimicrobial agent as dripping is prevented or greatly reduced by the rheology modifier. By formulating compositions with appropriate rheology modifier and degree of cross-linking, coating compositions with various coating properties may be prepared that will vary in the degree of surface finish and protection as well as ease of removal.

The coating composition of the present invention offers several mechanisms of protection against contamination of microbial or non-microbial origin, such as soiling. For example, planktonic or loosely adhering cells on the surface are killed, or alternatively growth is reduced or prevented, by the antimicrobial agent in the coating formulation as the liquid coating composition is applied.

Further, after application of the liquid antimicrobial coating composition of the present invention, cells harbored by biofilms on the surface will be killed, or growth can be reduced or prevented, by diffusion of the antimicrobial(s) into the hydrated biofilm before the applied film-forming composition completely dries to provide an antimicrobial film. For sustained antimicrobial activity it is desirable that the antimicrobial films of the present invention be semi-permeable. The antimicrobial film thus formed constitutes a reservoir of antimicrobial agent providing much longer contact time than conventional sanitary rinse solutions that typically drip off within seconds or minutes.

The long lasting activity while the coating present on the locus is especially beneficial in a variety of applications. A film-forming antimicrobial composition of the present invention does not drip off of the target surface quickly, and is not easily removed by incidental contact, for example. The variation of film flexibility, viscosity, strength, and adhesion of the coating of the present invention permits it to be tailored to specific applications, thus making sustained antimicrobial protection available in numerous situations where such sustained activity (residual benefit) was not previously available.

The film-forming water soluble or water dispersible agent useful in the practice of the present invention may be at least one of any agent, as described below, that is durable and removable. A film of the present invention is designed to be removable under relatively mild conditions. For example, a film of the present invention can be removed when subjected to treatment with an aqueous solution at a temperature above 15 °C, preferably above 30 °C. Suitable film-forming agents are selected from polyvinyl alcohols, polyvinyl alcohol copolymers and polyvinyl pyrrolidones. One skilled in the art may easily select the range of suitable molecular weights in order to provide a range of water solubility to provide a readily removable coating according to the methods of this invention.

Polyvinyl alcohol, sometimes referred to as poly(vinyl alcohol), is made from polyvinyl acetate by hydrolysis, and is suitable for use herein. The physical properties of polyvinyl alcohol are controlled by the molecular weight and the degree of hydrolysis. The most commonly available grades of polyvinyl alcohol, ranked by degree of hydrolysis, are an 87-89% grade (containing 11-13 mol% residual vinylacetate units), a 96% hydrolysis grade (containing 4 mol% residual vinyl acetate units), and the "fully hydrolyzed" and "superhydrolyzed" grades, which are about 98% and greater-than-99% hydrolyzed, respectively. Lower degrees of hydrolysis (e.g., 74% and 79%) are also commercially available. Some preferred degrees of hydrolysis are greater than 85 mol%, or greater than 92 mol%. The polyvinyl alcohol component of the present invention may also be a copolymer of vinyl alcohol, such as one obtained by hydrolyzing a copolymer of vinyl acetate with small amounts (up to about 15 mol%) of other monomers. Suitable co-monomers are e.g., esters of acrylic acid, methacrylic acid, maleic or fumaric acids, itaconic acid, etc. Also, copolymerization of vinyl acetate with hydrocarbons e.g., alpha-olefins such as ethylene, propylene or octadecene, etc., with higher vinyl esters such as vinyl butyrate, 2-ethyl hexoate, stearate, trimethyl acetate, or homologues thereof ("W-10" type of vinyl esters sold by Shell Chem. Co.), etc. gives copolymers that may be hydrolyzed to suitable polyvinyl alcohol copolymers. Other suitable comonomers are N-substituted acrylamides, vinyl fluoride, allyl acetate, allyl alcohol, etc. Also the free unsaturated acids such as acrylic acid, methacrylic acid, monomethyl maleate, etc. may act as co-monomers.

Because of the variety of grades either known in the literature or commercially available, one skilled in the art may formulate a polyvinyl alcohol solution having an average degree of hydrolysis ranging from 74 to more than 99% simply by blending the known or commercial grades in any desired ratios.

Film flexibility, water sensitivity, ease of solvation, viscosity, film strength and adhesion of the polyvinyl alcohol film may be varied by adjusting molecular weight and degree of hydrolysis.

In one embodiment, the polyvinyl alcohol for use in the process of this invention has a degree of hydrolysis from about 85% to greater than 99%. In another embodiment, the polyvinyl alcohol has a degree of hydrolysis from about 87% to greater than 89%.

In one embodiment, the polyvinyl alcohol has a number-averaged molecular weight (Mn) that falls in the range of between about 4,000 to about 200,000, or about 4,000 to about 150,000, or 10,000 to about 100,000.

In one embodiment, the polyvinyl alcohol has a molecular weight that falls in the range of between about 10,000 and 130,000. In another embodiment, the polyvinyl alcohol of various molecular weights may be blended to give the desired properties.

In one embodiment, the polyvinyl alcohol is used at about 2% to about 30% by weight of the weight of the solution. In a more specific embodiment, the polyvinyl alcohol is used at about 2% to about 15% by weight of the weight of the solution. In an even more specific embodiment, the polyvinyl alcohol is used at about 5% to about 12% by weight of the weight of the solution.

The film-forming composition of the present invention may contain PVP at a concentration of about 0.25 to about 50% by weight. Suitable grades of PVP are available from International Specialty Products (Wayne, NJ, USA). Such grades include: K-15, having a molecular weight (Mw) average range of about 6,000 to about 15,000; K-30, having a molecular weight range of about 40,000 to about 80,000; K-60, having a molecular weight range of about 240,000 to about 450,000; K-90, having a molecular weight range of about 900,000 to about 1,500,000; and K-120, having a molecular weight range of about 2,000,000 to about 3,000,000. Mixtures of PVP's may be employed, as may combinations of PVP and other film-forming compounds. Suitable grades are described in the commonly owned and co-pending U.S. Application Nos: U.S. 2007/0275101 A1 and U.S. 2008/0026026A1.

Typically, lower molecular weight PVP will give a less viscous product than a higher molecular weight PVP at the same concentration. For a given concentration of PVP, as the molecular weight range increases, the viscosity will also increase. The present invention may employ PVP having any of a number of molecular weight ranges. It is preferred, however, to use PVP with a molecular weight distribution between about 15,000 and about 3,000,000 g/mol. PVP having this molecular weight distribution typically gives a film-forming composition with a viscosity, which may be easily adjusted and washes off a surface easily with no visible signs of interaction with a painted surface. In a preferred embodiment, PVP with a molecular weight distribution between about 15,000 and about 3,000,000 g/mol is present at a concentration of between about 0.25% and about 40% by weight. In another preferred embodiment, PVP with a molecular weight distribution between about 30,000 and about 1,200,000 g/mol is present at a concentration of between about 0.25% and about 10% by weight.

One class of molecules which is widely recognized as including highly effective disinfectants is that of the organic peroxyacids. In recent years research has been directed at these compounds, aimed not least in providing a stable and safe means of their delivery. Amongst the peroxyacids, the one which has been the subject of closest attention has been peroxyacetic acid (often referred to as peracetic acid or PAA). This particular peroxyacid is rarely isolated as a pure compound and is normally encountered in solution in both aqueous and non-aqueous media. Peroxyacetic acid is often supplied as an equilibrium mixture including primarily water, acetic acid and hydrogen peroxide, together with a number of other minor components such as stabilizers (WO 2006/076334; U.S. Patent 5,508,046). However, even equilibrium systems of peroxyacetic acid are inherently thermodynamically unstable due to the decomposition of peroxyacetic acid into acetic acid and oxygen. Such decomposition may be slowed down if special measures are taken but cannot be completely prevented. A further property of peroxyacids which makes them difficult to use or store is that they may decompose rapidly and violently, particularly peroxyacids of low molecular weight and of high purity. To solve this problem, peroxyacids are often generated *in situ* to perform the particular desired function. Such *in situ* generation has an advantage in that the amount of peroxyacid produced may be stoichiometrically controlled through selecting the relative composition of the starting materials. Moreover, higher concentrations of peroxyacid may be achieved than are available from equilibrium systems due to the non-equilibrium nature of the *in situ* systems.

There is also a need to generate the peroxyacid at the required rate: too slow a rate may result in a lack of performance. The present method provides combinations of enzymes and enzyme substrates to produce the desired effective concentration of peracid, where, in the absence of added enzyme, there is a significantly lower concentration of peracid produced. Although there may in some cases be substantial chemical perhydrolysis of the enzyme substrate by direct chemical reaction of inorganic peroxide with the enzyme substrate, there may not be a sufficient concentration of peracid generated to provide an effective concentration of peracid in the desired applications, and a significant increase in total peracid concentration is achieved by the addition of an appropriate perhydrolase catalyst to the reaction mixture.

One solution to the inherent lack of stability of the peracids is to provide a system in which the reaction components that produce or react to form the peracids are maintained separately until required (WO 2006/016145). Such a solution to the problem is contemplated in the method described herein.

In the present invention a film-forming component is combined with other components, including a carboxylic acid ester substrate, an enzyme catalyst having perhydrolase activity, and a peroxygen source. The film-forming component and one or more of the following components, carboxylic acid ester substrate, enzyme catalyst, and peroxygen source, can be stored together in one compartment, or stored in separate compartments, prior to combining to form the antimicrobial coating composition and application to a target surface. For example, the film forming component can be stored in a separate compartment from the carboxylic acid ester substrate, enzyme catalyst and peroxygen source, and combined just prior to application to the target surface or, alternatively, the film-forming component can be stored together with the carboxylic acid ester substrate but separately from the enzyme catalyst or peroxygen source until such time as they are combined and applied to a target surface.

The present method provides for *in situ* enzymatic production of efficacious concentrations of peracids in a durable, continuous and easily removable film-forming composition using the perhydrolase activity of an enzyme from the CE-7 family of carbohydrate esterases. As described herein, the genes encoding said enzymes (e.g., enzymes with amino acid sequence SEQ ID NO: 7 isolated from a *Thermotoga neapolitana,* amino acid sequence SEQ ID NO: 8 isolated from a *Thermotoga maritima,* or a substantially similar amino acid sequence) are suitable for application in the current method. As disclosed above, other enzymes, from various sources, having perhydrolase activity may also be used in the present method disclosed herein.

In a preferred embodiment, the source of inorganic peroxide is hydrogen peroxide. It is not intended that the present invention be limited to inorganic hydrogen peroxide. Hydrogen peroxide may be generated using an enzyme catalyst and a suitable substrate. Any enzyme that generates hydrogen peroxide and acid with a suitable substrate finds use in the present method. For example, lactate peroxidase from *Lactobacillus* species, which are known hydrogen peroxide producers from lactic acid and oxygen may be used in the current method.

Other enzymes (e.g., glucose oxidase, alcohol oxidase, ethylene glycol oxidase, glycerol oxidase, amino acid oxidase, etc.) that can generate hydrogen peroxide also find use with ester substrates in combination with the perhydrolase enzyme of the present method to generate peracids. Enzymes that generate acid from substrates without the generation of hydrogen peroxide also find use in the present invention. Examples of such enzymes include, but are not limited to proteases.

In another aspect, the peroxygen source may be any peroxide compound and may be selected from, but not limited to, the following: hydrogen peroxide, sodium perborate monohydrate, sodium perborate tetrahydrate, sodium pyrophosphate peroxyhydrate, urea peroxyhydrate, sodium percarbonate, sodium peroxide and mixtures thereof.

In one embodiment, suitable substrates include esters provided by the following formula:

[X]ₘR₅

wherein X=an ester group of the formula R₆C(O)O
R₆=C1 to C7 linear, branched or cyclic hydrocarbyl moiety, optionally substituted with hydroxyl groups or C1 to C4 alkoxy groups, wherein R₆ optionally comprises one or more ether linkages for R₆=C2 to C7;
R₅=a C1 to C6 linear, branched, or cyclic hydrocarbyl moiety optionally substituted with hydroxyl groups; wherein each carbon atom in R₅ individually comprises no more than one hydroxyl group or no more than one ester group; wherein R₅ optionally comprises one or more ether linkages;
m=1 to the number of carbon atoms in R₅; and wherein said esters have a solubility in water of at least 5 ppm at 25° C.

In another embodiment, suitable substrates also include esters of the formula: R₁-COO-R₂ wherein R₁=a C1 to C7 straight chain or branched chain alkyl optionally substituted with an hydroxyl or a C1 to C4 alkoxy group and R₂=C1 to C10 straight chain or branched chain alkyl, alkenyl, alkynyl, aryl, alkylaryl, alkylheteroaryl, heteroaryl, -(CH₂CH₂-O)ₙH or -(CH₂CH(CH₃)-O)ₙH and n=1 to 10.

In another embodiment, suitable substrates include glycerides of the formula: R₁-COO-CH₂-CH(OR₃)-CH₂-OR₄ wherein R₁=C1 to C7 straight chain or branched chain alkyl optionally substituted with an hydroxyl or a C1 to C4 alkoxy group and R₃ and R₄ are individually H or R₁C(O).

In another embodiment, R₆ is C1 to C7 linear hydrocarbyl moiety, optionally substituted with hydroxyl groups or C1 to C4 alkoxy groups, optionally comprising one or more ether linkages. In a further preferred embodiment, R₆ is C2 to C7 linear hydrocarbyl moiety, optionally substituted with hydroxyl groups, and/or optionally comprising one or more ether linkages.

In another embodiment, suitable substrates also include acetylated saccharides selected from the group consisting of acetylated mono-, di-, and polysaccharides. In a preferred embodiment, the acetylated saccharides include acetylated mono-, di-, and polysaccharides. In another embodiment, the acetylated saccharides are selected from the group consisting of acetylated xylan, fragments of acetylated xylan, acetylated xylose (such as xylose tetraacetate), acetylated glucose (such as glucose pentaacetate), β-D-ribofuranose-1,2,3,5-tetraacetate, tri-O-acetyl-D-galactal, and tri-O-acetyl-D-glucal, and acetylated cellulose. In a preferred embodiment, the acetylated saccharide is selected from the group consisting of β-D-ribofuranose-1,2,3,5-tetraacetate, tri-O-acetyl-D-galactal, and tri-O-acetyl-D-glucal, and acetylated cellulose. As such, acetylated carbohydrates may be suitable substrates for generating percarboxylic acids using the present process (i.e., in the presence of a peroxygen source).

In one embodiment, the substrate is selected from the group consisting of: monoacetin; diacetin; triacetin; monopropionin; dipropionin; tripropionin; monobutyrin; dibutyrin; tributyrin; glucose pentaacetate; xylose tetraacetate; acetylated xylan; acetylated xylan fragments; β-D-ribofuranose-1,2,3,5-tetraacetate; tri-O-acetyl-D-galactal; tri-O-acetyl-glucal; monoesters or diesters of 1,2-ethanediol, 1,2-propanediol, 1,3-propanediol, 1,2-butanediol, 1,3-butanediol, 2,3-butanediol, 1,4-butanediol, 1,2-pentanediol, 2,5-pentanediol, 1,6-pentanediol, 1,2-hexanediol, 2,5-hexanediol, 1,6-hexanediol; and mixtures thereof.

In a preferred embodiment, the substrate is selected from the group consisting of ethyl acetate, methyl lactate, ethyl lactate, methyl glycolate, ethyl glycolate, methyl methoxyacetate, ethyl methoxyacetate, methyl 3-hydroxy- butyrate, ethyl 3-hydroxybutyrate, triethyl 2-acetyl citrate, glucose pentaacetate, gluconolactone, glycerides (mono-, di-, and triglycerides) such as monoacetin, diacetin, triacetin, monopropionin, dipropionin (glyceryl dipropionate), tripropionin (1,2,3-tripropionylglycerol), monobutyrin, dibutyrin (glyceryl dibutyrate), tributyrin (1,2,3-tributyrylglycerol), acetylated saccharides, and mixtures thereof.

In a further preferred aspect, the carboxylic acid ester substrates are selected from the group consisting of monoacetin, diacetin, triacetin, monopropionin, dipropionin, tripropionin, monobutyrin, dibutyrin, tributyrin, ethyl acetate, and ethyl lactate. In yet another aspect, the carboxylic acid ester substrates are selected from the group consisting of diacetin, triacetin, ethyl acetate, and ethyl lactate. In a preferred aspect, the carboxylic acid ester is a glyceride selected from the group consisting of monoacetin, diacetin, triacetin, and mixtures thereof.

Inert solvents useful for the invention include water or solutions comprising at least 50 wt% water. Additional solvents include mono alcohols, monofunctional and polyfunctional alcohols, preferably containing from about 1 to about 6 carbon atoms and from 1 to about 6 hydroxy groups. Examples include ethanol, isopropanol, n-propanol, 1,2-propanediol, 1,2-butanediol, 2-methyl-2,4-pentanediol, mannitol and glucose. Also useful are the higher glycols, polyglycols, polyoxides, glycol ethers and propylene glycol ethers. Additional solvents include the free acids and alkali metal salts of sulfonated alkylaryls such as toluene, xylene, cumene and phenol or phenol ether or diphenyl ether sulfonates; alkyl and dialkyl naphthalene sulfonates and alkoxylated derivatives.

The compositions useful for the present invention may also contain one or more surfactants. While not being bound by theory, it is believed that a surfactant will aid wetting of the surface to be covered and will aid even coverage by the film. The surfactant is also believed to aid foaming by the film when removed, thereby aiding removal of the film and washing of the protected surface. Suitable surfactants have a preferred hydrophilic-lipophilic balance (HLB) of from about 9 to about 17. Suitable surfactants include, but are not limited to: amphoteric surfactants, such as Amphoteric N from Tomah Products; silicone surfactants, such as BYK 348 available from BYK Chemie (BYK-Chemie GmbH, Wesel, Germany); fluorinated surfactants such as Zonyl® FS300 from DuPont (Wilmington, DE, USA); and nonylphenoxypolyethoxyethanol based surfactants, such as Triton N-101 available from Dow (Midland, MI, USA). Other suitable surfactants include ethoxylated decynediols such as Surfynol 465 available from Air Products & Chemicals (Allentown, PA, USA); alkylaryl polyethers such as Triton CF-10 available from Dow; octylphenoxy polyethoxy ethanols such as Triton X-100 available from Dow; ethoxylated alcohols such as Neodol 23-5 or Neodol 91-8 available from Shell (The Hague, the Netherlands); Tergitol 15-S-7 available from Dow; Steol-4N, a 28% sodium laureth sulfate from Stepan Company (Northfield, IL, USA); amine oxides such as Ammonyx® LO available from Stepan; EO/PO block copolymers such as Pluronic® 17R4 available from BASF (Parsippany, NJ, USA); sorbitan derivatives such as Tween 20 or Tween 60 from Uniqema (New Castle, DE, USA); and quaternary ammonium compounds, such as benzalkonium chloride. Other suitable surfactants include organo-silicone surfactants such as Silwet®L-77 from Setre Chemical Company (Memphis, TN, USA); DowComing® Q2-5211 from DowCorning Silicones (Midland, MI, USA); or Silsurf® A008 by Siltech Corporation (Toronto, ON, Canada).

It can be important for flexibility and integrity of the protective film that the resultant film be plasticized. Plastization of the film has been accomplished for the purposes of this invention by the incorporation of a suitable plasticizing agent such as polyethylene glycol or glycerol. Other plasticizers suitable for the invention include, but are not limited, to solvents, polyols, polyethylene glycols of and average molecular weight between 200 and 800 g/mol and sorbitol. PEG is preferred over glycerol since glycerol is easily metabolized by microorganisms potentially resulting in microbial growth.

Inclusion of a plastisizer generally also allows the film to retain a slightly tacky surface feel. As the plastisizer level increases, the resulting film will also exhibit an increasing degree of tackiness. Such tackiness may be desirable at low levels in order to capture airborne particles and soil or other materials. If plastisizer levels are too high, however, the coating becomes too tacky and will show low resistance to accidental mechanical removal, by wiping, for example.

The preferred plasticizer amount is from about 1 wt% to about 20 wt% of the weight of the film former, and more preferably from about 5 wt% to about 10 wt%.

The composition useful for the invention may also contain one or more rheology modifiers employed to enhance viscosity, or thicken and cause the aqueous treatment or coating composition to cling to the surface. Clinging enables the composition to remain in contact with transient and resident microorganisms for longer periods of time, promoting microbiological efficacy and resisting waste because of excessive dripping. The rheology modifier may be a film former or act cooperatively with a film-forming agent to form a barrier that provides additional protection. Water soluble or water dispersible rheology modifiers that are useful may be classified as inorganic or organic. The organic thickeners may further be divided into natural and synthetic polymers with the latter still further subdivided into synthetic natural-based and synthetic petroleum-based.

Inorganic thickeners are generally compounds such as colloidal magnesium aluminum silicate (VEEGUM®), colloidal clays (Bentonites), or silicas (CAB-O-SIL®) which have been fumed or precipitated to create particles with large surface to size ratios. Natural hydrogel thickeners of use are primarily vegetable derived exudates. For example, tragacanth, karaya, and acacia gums; and extractives such as carrageenan, locust bean gum, guar gum and pectin; or, pure culture fermentation products such as xanthan gum are all potentially useful in this invention. Chemically, all of these materials are salts of complex anionic polysaccharides. Synthetic natural-based thickeners having application are cellulosic derivatives wherein the free hydroxyl groups on the linear anhydro-glucose polymers have been etherified or esterified to give a family of substances which dissolve in water and give viscous solutions. This group of materials includes the alkyl and hydroxyl- alkylcelluloses, specifically methylcellulose, hydroxyethylmethylcellulose, hydroxypropylmethylcellulose, hydroxybutylmethylcellulose, hydroxyethylcellulose, ethylhydroxyethylcellulose, hydroxypropylcellulose, and carboxy- methylcellulose. Another preferred group of thickeners include polyacrylates such as the proprietary Acusol thickeners, (e.g., Acusol 823, Rohm and Haas, Philadelphia, PA, USA), and Carbopol thickeners, such as Carbopol 934 or Carbopol Aqua-30 Polymer (B F Goodrich, Cleveland, OH, USA). Additional preferred acrylate-based rheology modifiers include the proprietary cationic Rheovis® thickeners (Ciba, Basel, Switzerland). A polyacrylate thickener may be used at concentrations of up to about 3 wt% of the film former weight. Mixtures of thickening agents may also be employed where the total amount may be up to about 3 wt% depending on the thickeners used and the desired viscosity of the final product.

Other potential thickeners for this application include dextrin, cornstarch and hydrous magnesium silicates, such as sodium magnesium silicate sold under the trade name Laponite XLG (Southern Clay Products, Inc., Gonzales, TX, USA).

In addition to the *in situ* generated peroxyacid, an additional antimicrobial agents is present in the composition. The antimicrobial agent useful for the invention comprises. quaternary ammonium salts which include the N-C₁₀-C₂₄ - alkyl-N-benzyl-quaternary ammonium salts which comprise water solubilizing anions such as halide, e.g., chloride, bromide and iodide; sulfate, methosulfate and the like and the heterocyclic imides such as the imidazolinium salts. Quaternary ammonium salts may also include other non-halogenated anions such as propionates and saccharinates and the like. The coating composition comprising the antimicrobial agent offers protection against diverse microorganisms.

In one embodiment, the coating composition protects against Gram positive or Gram negative bacteria. Gram positive bacteria which are inhibited or killed by the coating include, but are not limited to, *Clostridium tetani, C. perfringens, C. botulinum,* other *Clostridium* species, *Mycobacterium tuberculosis, M. bovis, M. typhimurium, M. bovis strain BCG, BCG substrains, M. avium, M. intracellulare, M. africanum, M. kansasii, M. marinum, M. ulcerans, M. avium subspecies paratuberculosis, Staphylococcus aureus, S. epidermidis, S. equi, Streptococcus pyogenes, S. agalactiae, Listeria monocytogenes, L. ivanovii, Bacillus anthracis, B. subtilis, Nocardia asteroides, and other Nocardia species, Streptococcus viridans group, Peptococcus species, Peptostreptococcus species, Actinomyces israelii* and other *Actinomyces* species; *Propionibacterium acnes,* and *Enterococcus* species. Gram negative bacteria which are inhibited or killed by the coating include, but are not limited to, *Pseudomonas aeruginosa,* other *Pseudomonas* species, *Campylobacter* species, *Vibrio cholerae, Ehrlichia species, Actinobacillus pleuropneumoniae, Pasteurella haemolytica, P. multocida,* other *Pasteurella* species, *Legionella pneumophila,* other *Legionella* species, *Salmonella typhi,* other *Salmonella* species, *Shigella* species *Brucella abortus,* other *Brucella* species, *Chlamydia trachomatis, C. psittaci, Coxiella bumetti, Neiserria meningitidis, N. gonorrhea, Haemophilus influenzae, H. ducreyi,* other *Haemophilus* species, *Yersinia pestis, Y. enterolitica,* other *Yersinia* species, *Escherichia coli, E. hirae* and other *Escherichia* species, as well as other Enterobacteriacae, *Burkholderia cepacia, B. pseudomallei, Francisella tularensis, Bacteroides fragilis, Fusobacterium nucleatum, Provetella* species, *Cowdria ruminantium, Klebsiella* species, and *Proteus* species.

In another embodiment, the coating provides protection against fungi, including but are not limited to *Altemaria alternata, Aspergillus niger, Aureobasidium pullulans, Cladosporium cladosporioides, Drechslera australiensis, Gliomastix cerealis, Monilia grisea, Penicillium commune, Phoma fimeti, Pithomyces chartarum,* and *Scolecobasidium humicola.*

Trace amounts of impurities, especially metals, may react with hydrogen peroxide and peroxyacids and cause decomposition. Therefore, many peroxide/peroxyacid compositions include stabilizing ingredients, such as compounds that sequester metals and metal-containing impurity materials. Examples of preferred chelating agents include, but are not limited to, alkylideneaminophosphonic acids or salts thereof, some of which are marked under the name Dequest® (Thermphos, Vlissingen, the Netherlands), 1-hydroxyethylidene-1,1-diphosphonic acids and salts thereof, some of which are marked under the name Turpinal® (Thermphos), and 2-phosphono-1,2,4-butanetricarboxylic acids and salts thereof, available from LANXESS Corporation (Pittsburgh, PA, USA) under Bayhibit®. A further class of compounds suitable for use is the aminocarboxylicacids or salts thereof. An example is ethylenediaminetetraacetic acid.

In addition, other suitable chelating agents include dipicolinic acid, ethane-1,1,2-triphosphonic acid and ethylidene-1,1-diphosphonic acid. Furthermore, also well know in the art as chelating agents are phosphates, polyphosphates, pyrophosphates, and carboxylic acids, such as citric acid or salicylic acid. The stabilizer or stabilizers should be present in sufficient amounts to inhibit breakdown of the peroxide/peroxyacids.

The present invention can optionally include colorants or dyes. Colorants useful for the present invention include dyes and pigments such as food grade pigments.

Dyes useful for the invention include both water soluble and water insoluble dyes. Water soluble dyes may be formulated easily in the aqueous systems of the invention. Water insoluble dyes may be included in an oil phase that may be dispersed or suspended in the antimicrobial coating compositions useful for the invention. Useful dyes for the purpose of this invention are typically organic compounds that absorb visible light resulting in the appearance of a detectable color. Fluorescent dyes may also be used, for example, for purposes of visualizing a film by ultraviolet light.

The dyes typically useful in this invention are colorants approved for use in foods, drugs, cosmetics and medical devices. Colorants currently in use and their status is as follows: Colorants permitted in foods that are (1) subject to certification: FD&C Blue No. 1, FD&C Blue No. 2, FD&C Green No. 3, FD&C Red No. 3, FD&C Red No. 40, FD&C Yellow No. 5, FD&C Yellow No. 6, Citrus Red No. 2, and Orange (B) (2) exempt from certification: annatto extract, theta-apo-8'-carotenal, canthaxanthin, caramel, theta-carotene, carrot oil, cochineal extract (carmine), corn endosperm oil, dehydrated beets (beet powder), dried algae meal, ferrous gluconate, fruit juice, grape color extract, grape skin extract, paprika, paprika oleoresin, riboflavin, saffron, synthetic iron oxide, tagetes meal and extract, titanium dioxide, toasted partially defatted cooked cottonseed flour, turmeric, termeric oleoresin, ultramarine blue, and vegetable juice. Colorants permitted in drugs (including colorants permitted in foods) that are (1) subject to certification: FD&C Red No. 4, D&C Blue No. 4, D&C Blue No. 9, D&C Green No. 5, D&C Green No. 6, D&C Green No. 8, D&C Orange No. 4, D&C Orange No. 5, D&C Orange No. 10, D&C Orange No. 11, D&C Red No. 6, D&C Red No. 7, D&C Red No. 17, D&C Red No. 21, D&C Red No. 22, D&C Red No. 27, D&C Red No. 28, D&C Red No. 30, D&C Red No. 31, D&C Red No. 33, D&C Red No. 34, D&C Red No. 36, D&C Red No. 39, D&C Violet No. 2, D&C Yellow No. 7, D&C Yellow No. 8, D&C Yellow No. 10, D&C Yellow No. 11, and Ext. D&C Yellow No. 7. Additionally cantaxanthin, beta carotene, chlorophyllin, and other colors are known. For a more detailed listing and/or discussion on approved colors, see D. M. Marmion, Handbook of U.S. Colorants, Foods, Drugs, Cosmetics and Medical Devices, John Wiley & Sons Inc., New York (1991) and U.S. Code of Federal Regulations, Title 21, parts 70-82.

The present invention may optionally include cross-linking agents. Advantages of using cross-linking agents with the film-forming composition include influencing the mechanical film properties, such as tackiness and mechanical strength, as well as solubility of the coating. Furthermore, cross-linking decreases tackiness and prevents soil and microorganisms from physically adhering to the polymer film, which may be desirable for certain applications. The degree of cross-linking is adjusted so to achieve the desired combination of properties.

Cross-linking agents suitable for use with polyvinyl alcohol and copolymers thereof include, but are not limited to: aldehydes (e.g., formaldehyde, glyoxal, glutaraldehyde), boric acid, sodium tetraborate, metal ions (e.g., ions of Zn, Fe, Al, Ni, V, Co, Cu, Zr, Ti, Mn), organometallic compounds (e.g., organic titanates such as DuPont Tyzor®, organic Cr(III) complexes such as DuPont Quilon®), siloxanes (e.g., tetraethoxysilane, polydimethylsiloxane), isocyanates (e.g., of the blocked, water-soluble or dispersed type), epoxides (e.g., diglycidyl ether), dicarboxylic acid (e.g., oxalic, maleic, fumaric, phthalic), urea based crosslinkers (e.g., Sunrez 700). Bi- and trivalent metal cations (e.g., Fe(II), Fe(III), Al(III)) are preferred because they provide the formation of a coordinative linkage between the PVOH polymer chains upon film drying. This allows the cross-linker to be added to the film-forming liquid in a 'one-pot' mixture. Care must be taken to choose an adequate concentration in order to efficiently cross-link the polymer without precipitating other ingredients such as particulate rheology control agents.

In most cases the cross-linking agent will be mixed with other ingredients using standard mixing techniques. The cross-linking reaction may optionally be carried out in the presence of a catalyst, as is well known to those skilled in the art. In the case of the aldehydes, isocyanates, siloxanes, diglycidyl ether, and dicarboxylic acid, heat and an acid catalyst or metal catalyst may be used additionally.

The cross-linking agent concentration in the formulation may be zero to an upper limit which is either determined by the stability limit of the formulation where precipitation starts to occur, or the inability of the resulting film to be removed efficiently.

The preferred cross-linking agent concentration may depend strongly on the type of cross-linking agent used and is typically below 25 wt% of the polymer content, more preferably below 10 wt% of the polymer content.

In addition to the foregoing components, the composition of the present invention may also comprise one or more performance enhancing additives also known as "performance enhancers". These include flash rust inhibitors, which include any of a number of organic or inorganic materials used in a water-based system to prevent rust from forming on contact with the material and bare metal. Two examples are sodium benzoate or benzotriazole.

Another optional performance enhancing additive include one or more of an array of defoamers recommended for water-based systems, to prevent unwanted foaming of the product during application. Too much foam may disrupt the required continuous film formation of the product and result in product failure. It may also be advantageous to add a foam control product, to aid in mixing and processing the masking composition, such as Drewplus L475 from Ashland Chemical, Inc. Drew Industrial Division (Covington, KY, USA). Additional optional performance enhancing additives are antioxidants to increase the shelf life of the coating formulation. One example is butylated hydroxytoluene. Further additional additives include fragrances.

Foaming agents may additionally be added to create gas bubbles in the applied coating. Gas bubbles may function as an opacifying agent to facilitate the application and/or to allow for longer contact time with a surface; e.g., by preventing dripping from an inclined surface and/or to reduce the amount of coating formulation needed to treat a certain surface area or volume.

Application indicators may also be added. Some of these are described above, but include pigments, dyes, fluorescent dyes, pH indicators or gas bubbles generated during application.

Small amounts (typically less than 1 percent by weight) of these additional materials may be added with an appropriate adjustment of the water or other components. It is to be understood that mixtures of any one or more of the foregoing optional components may also be employed.

For loci comprised of fibrous substrates, an optional performance-enhancing ingredient is an agent that provides a surface effect. Such surface effects include no iron, easy to iron, shrinkage control, wrinkle free, permanent press, moisture control, softness, strength, anti-slip, antistatic, anti-snag, anti-pill, stain repellency, stain release, soil repellency, soil release, water repellency, oil repellency, odor control, antimicrobial, or sun protection.

The film or coating may be applied to the target surface or locus by any means, including pouring. The film or coating is applied to achieve a continuous and/or homogenous layer on a target surface. Coating systems routinely used for paints and coatings, such as, but not limited to, brushes, rollers, paint pads, mats, sponges, combs, hand-operated pump dispensers, compressed air operated spray guns, airless spray guns, electric or electrostatic atomizers, backpack spray application equipment, aerosol spray cans, clothes, papers, feathers, styluses, knives, and other applicator tools may be used for coating. If dipping is used as a method to apply the coating, no special equipment is required. If an aerosol spray can is used for application, the coating composition may be mixed with an aerosol propellant (such as a compressed gas) or the coating composition may be physically separated from the propellant by a barrier material such as a polymer bag inside the can; if the coating composition and the propellant are mixed the mixture may constitute one or more liquid phases.

For fibrous substrates, such as textiles and carpets, the coating may be applied by exhaustion, foam, flex-nip, nip, pad, kiss-roll, beck, skein, winch, liquid injection, overflow flood, roll, brush, roller, spray, dipping, immersion, and the like. The coating may also be applied by use of the conventional beck dyeing procedure, continuous dyeing procedure or thread-line application.

In one embodiment of the current disclosure, electrostatic sprayers can be used to coat the surface. Electrostatic sprayers impart energy to the aqueous coating composition via a high electrical potential. This energy serves to atomize and charge the aqueous coating composition, creating a spray of fine, charged particles. Electrostatic sprayers are readily available from suppliers such as Tae In Tech Co., South Korea and Spectrum, Houston, TX, USA.

In another embodiment of the invention, an airless spray gun may be used to coat the target surface. Airless spray guns use high fluid pressures and special nozzles, rather than compressed air, to convey and atomize the liquid. The liquid is supplied to an airless gun by a fluid pump at pressures typically ranging from 3.5 to 45 MPa. When the paint exits the fluid nozzle at this pressure, it expands slightly and atomizes into tiny droplets without the impingement of atomizing air. The high velocity of the exiting paint propels the droplets toward the target surface. The fluid nozzle on an airless gun differs substantially from the fluid nozzle on an air atomized gun. Selection of the proper nozzle determines how much paint is delivered and the fan pattern of application. The size of the airless nozzle orifice determines the quantity of paint to be sprayed. Airless fluid delivery is high, ranging from 700-2000 mL/min. Recommended gun distance is about 30 cm from the target, and depending upon the nozzle type, a fan pattern of 10 to 45 cm is possible. Thus, nozzles may be selected for each application based on the size and shape of the target surface and the thickness of the coating to be applied. Airless guns create little air turbulence that may repel the liquid from "hard to reach areas", such as would be found in food processing equipment, hatcheries etc. The high flow rate makes airless advantageous in cleaning and disinfecting situations, where the antimicrobial coating is to be applied over a large surface area and multiple surfaces.

The thickness of the applied and dried film will depend on a variety of factors. These factors include the concentration of the film forming agent, the concentration of rheology control additives and/or other additives, as well as the application temperature and humidity. Film thickness and film uniformity also depend, at least in part, on parameters of the application equipment, such as fluid delivery, spray orifice diameter, air pressure or piston pump pressure in the case of airless application, and the distance of the spray applicator to the target surface. Therefore, the liquid formulation may be adjusted to yield the desired film thickness. The atomization of the coating solution is chosen such that a thin film is applied homogeneously to the target area.

Target surfaces (loci) include all surfaces that may potentially be contaminated with microorganisms, including surfaces typically difficult to apply a disinfectant or sanitizer to (such as hard-to-reach surfaces). Examples of target surfaces include equipment surfaces found in the food or beverage industry (such as tanks, conveyors, floors, drains, coolers, freezers, refrigerators, equipment surfaces, ceilings, walls, valves, belts, pipes, drains, ductwork, joints, crevasses, combinations thereof, and the like); building surfaces, including buildings under construction, new home construction, and surfaces in or on seasonal properties like vacation home surfaces (such as ceilings, walls, wood frames, floors, windows, ductwork), kitchens (sinks, drains, counter-tops, refrigerators, cutting boards), bathrooms (showers, toilets, drains, pipes, ductwork, bath-tubs), (especially for mold removal), decks, wood, siding and other home exteriors, asphalt shingle roofing, patio or stone areas (especially for algae treatment); boats and boating equipment surfaces; garbage disposals, garbage cans and dumpsters or other trash removal equipment and surfaces; non-food-industry related pipes and drains; surfaces found in hospitals; or surfaces where surgery, out-patient, or veterinary services are provided (such as ceilings, walls, floors, ductwork, beds, equipment, clothing worn in hospital/veterinary or other healthcare settings, including scrubs, shoes, and other hospital or veterinary surfaces) first-responder or other emergency services equipment and clothing; lumber-mill equipment, surfaces and wood products; restaurant surfaces; supermarket, grocery, retail and convenience store equipment and surfaces; deli equipment and surfaces and food preparation surfaces; brewery and bakery surfaces; bathroom surfaces such as sinks, showers, counters, and toilets; clothes and shoes; toys; school and gymnasium equipment, ceilings, walls, floors, windows, ductwork and other surfaces; kitchen surfaces such as sinks, counters, appliances; wooden or composite decks, pool, hot tub and spa surfaces; carpet; paper; leather; animal carcasses, fur and hides; surfaces of barns, or stables for livestock, such as poultry, cattle, dairy cows, goats, horses and pigs; and hatcheries for poultry or for shrimp. Surfaces within structures wherein animals are housed, such as cages and pens for example, can be coated using the antimicrobial coatings described herein. Additional surfaces also include food products, such as beef, poultry, pork, vegetables, fruits, seafood, combinations thereof, and the like.

Additional loci suitable for use in the present invention comprise fibrous surface substrates and include fibers, yarns, fabrics, textiles, nonwovens, carpets, leather, or paper. The fibrous substrates are made with natural fibers such as wool, cotton, jute, sisal, sea grass, paper, coir and cellulose, or mixtures thereof; or are made with synthetic fibers such as polyamides, polyesters, polyolefins, polyaramids, acrylics and blends thereof; or blends of at least one natural fiber and at least one synthetic fiber. By "fabrics" is meant natural or synthetic fabrics, or blends thereof, composed of fibers such as cotton, rayon, silk, wool, polyester, polypropylene, polyolefins, nylon, and aramids such as "NOMEX®" and "KEVLAR®." By "fabric blends" is meant fabric made of two or more types of fibers. Typically these blends are a combination of at least one natural fiber and at least one synthetic fiber, but also may be a blend of two or more natural fibers or of two or more synthetic fibers. Nonwoven substrates include, for example, spunlaced nonwovens, such as SONTARA available from E. I. du Pont de Nemours and Company (Wilmington, DE, USA), and laminated nonwovens, such as spunbonded-meltblown-spunbonded nonwovens.

Examples of surface materials are metals (e.g., steel, stainless steel, chrome, titanium, iron, copper, brass, aluminum, and alloys thereof), minerals (e.g., concrete), natural or synthetic polymers and plastics (e.g., polyolefins, such as polyethylene, polypropylene, polystyrene, poly(meth)acrylate, polyacrylonitrile, polybutadiene, poly(acrylonitrile, butadiene, styrene), poly(acrylonitrile, butadiene), acrylonitrile butadiene; polyesters such as polyethylene terephthalate; and polyamides such as nylon). Additional surfaces include brick, tile, ceramic, porcelain, glass, wood, vinyl, and linoleum.

Equipment or surfaces protected with a temporary coating may be in use or not in use while protected. The target surface may be hydrophobic or hydrophilic.

The coating system may also be one or more components, and may include a catalyst. Generally, the coating is allowed to set or dry for about greater than 5 minutes in order to form the film. However, the coating may be antimicrobially effective in a shorter time-frame, such as after 30 seconds. The coating may be removed before it is dried or anytime thereafter depending on the desired use. The drying time will be partially dependent on a number of factors, including environmental conditions such as humidity and temperature. The drying time will also depend on the thickness of the applied coating.

The thickness of the film or coating applied onto the target surface influences the time needed for removal and the amount of biocide per unit area applied to the surface. Thicker films increase the time interval until the film has to be re-applied to maintain the desired antimicrobial properties. Thinner films will be easier and faster to remove by rinsing. It is thus important to apply the formulation in a fashion that results in a film thickness that allows both easy removal of the coating and long-lasting antimicrobial properties. As described above, the film or coating has a thickness of about 0.3 to about 300 micrometers. In a more specific embodiment, the film or coating has a thickness of about 0.5 to about 100 micrometers. In an even more specific embodiment, the film or coating has a thickness of about 1.0 to about 30 micrometers.

This invention is directed toward films that may be removed at a time determined appropriate by the user. The coatings are removed with ease and may be removed by rinsing the surface with an aqueous solvent or solution. The time of removal may be determined by either (i) the desired minimum contact time to allow for the desired antimicrobial activity, typically expressed as amount of killed or inactivated microorganisms out of a starting population or (ii) the need or desire to take the coating off the surface before starting a subsequent operation or process step. Although the coating may be removed at any time, such as after drying, the film thickness, concentration of antimicrobial agent, and specific use determines the appropriate time for removal. For instance the user may wish to put treated equipment back into normal operation after a period of operational shutdown. Fruits, for example, will require washing prior to eating. Upon exhaustion of the biocide in the film, the film could be removed and a fresh coating layer could be applied. For example, drains may be treated periodically such as daily, weekly or biweekly. Antimicrobial activity may be measured as early as after 30 seconds, hours, days, weeks, months, even years after application of the film. Therefore, timing of removing the coating is a function of the application for which the coating is employed.

Film removal may be achieved by dissolution or dispersion of the resulting coating. This may be achieved by the application of an aqueous solution onto the coating. In one embodiment, the temperature of the solution is in the range of about 15 °C to about 100 °C. In another embodiment, the temperature of the solution is from about 30 to about 80 °C. The application of the solution, or water, may be achieved by a simple rinse or spray onto the surface. Coating removal may also be achieved by use of a pressure washer, facilitating removal by additional mechanical forces. Coating removal may also be achieved by washing with water together with a cloth or sponge. Further, mild additives may be utilized or mixed with the aqueous solution to help solubilize or disperse the film-forming or water-dispersible agents, including commonly used acids or bases, chelators or detergents. Alternatively, the film may be degraded, such as in a drain, by repeated washing of water and/or other components down the drain. The film may also be removed by peeling it off a surface, being abraded or brushed from the surface, or other mechanical mechanisms of removal.

Besides the intentional removal by an operator, removal also includes the removal by an automated or robotic system and the non-intentional removal by a liquid continuously or periodically contacting the coating over time, e.g., in a pipe or drain, or by continuous or periodical application of mechanical forces, such as wear.

Contact time refers to the time the coating or coating composition provides antimicrobial properties to microorganisms that come into contact or the vicinity of said coating or coating composition. Depending on the specific requirements for the antimicrobial formulations, the contact time would vary, as set out in Germicidal and Detergent Sanitizing Action of Disinfectants, Official Methods of Analysis of the Association of Official Analytical Chemists, paragraph 960.09 and applicable sections, 15th Edition, 1990 (EPA Guideline 91-2). For example, if the intended application of the present disclosure is use as a sanitizer for food-contact surfaces, then the composition should provide a 99.999% reduction (5-log order reduction) within 30 seconds at room temperature against several test microorganisms. If the intended application is as a sanitizer for non-food contact surfaces, then the composition should provide a 99.9% reduction (3-log order reduction) within 5 minutes at room temperature against several test microorganisms. If the intention is to use the disclosure as a disinfectant, then the composition should provide a 99.9% reduction (3-log order reduction) within 10 minutes. If the intended application is to provide residual antimicrobial activity, then the present method would be allowed to have greater than 10 minute contact time with microorganisms.

The coating of the present invention provides a physical barrier. A physical barrier is defined herein as the film formed from the present film forming composition. The resulting film seals the treated surface from contamination from the surrounding, such as soil, fat, dust, microorganisms etc. These contaminants will remain on the surface of the coating and will wash off at the time of removal of the coating.

All of the methods and compositions disclosed and claimed herein may be made and executed without undue experimentation in light of the present disclosure.

### GENERAL METHODS

### Recombinant techniques and growth of microorganisms

Standard recombinant DNA and molecular cloning techniques used in the Examples are well known in the art and are described by Sambrook, J., Fritsch, E. F. and Maniatis, T., Molecular Cloning: A Laboratory Manual, Cold Spring Harbor Laboratory Press, Cold Spring Harbor, N.Y., 1989, by T. J. Silhavy, M. L. Bennan, and L. W. Enquist, Experiments with Gene Fusions, Cold Spring Harbor Laboratory, Cold Spring Harbor, N.Y., 1984, and by Ausubel, F. M. et al., Current Protocols in Molecular Biology. Greene Publishing Assoc. and Wiley-Interscience, N.Y., 1987.

Materials and methods suitable for the maintenance and growth of bacterial cultures are also well known in the art. Techniques suitable for use in the following Examples may be found in Manual of Methods for General Bacteriology, Phillipp Gerhardt, R. G. E. Murray, Ralph N. Costilow, Eugene W. Nester, Willis A. Wood, Noel R. Krieg and G. Briggs Phillips, eds., American Society for Microbiology, Washington, DC, USA, 1994, or by Thomas D. Brock in Biotechnolooy: A Textbook of Industrial Microbiology, Second Edition, Sinauer Associates, Inc., Sunderland, MA, USA, 1989.

All reagents, restriction enzymes and materials used for the growth and maintenance of bacterial cells were obtained from Aldrich Chemicals (Milwaukee, WI, USA), BD Diagnostic Systems (Sparks, MD, USA), Life Technologies (Rockville, MD, USA), or Sigma Chemical Company (St. Louis, MO, USA), unless otherwise specified.

### SDS Gel electrophoresis

SDS gel electrophoresis was performed using methods well known in the art.

### Test methods for antimicrobial activity on hard surfaces

Peracids may have biocidal activity. Typical alternative biocides known in the art, which may be suitable for use in the present invention include, for example, chlorine, chlorine dioxide, chloroisocyanurates, hypochlorites, ozone, amines, chlorinated phenolics, copper salts, organo-sulfur compounds, and quaternary ammonium salts. Biocidal or antimicrobial efficacy of the coating compositions was measured using the two test methods described below.

*Non-food contact sanitizer test:* To assess the antimicrobial activity of coating compositions for a situation where a microbial contamination was already present on the target surface at the time of the application of the antimicrobial coating composition the "Standard Test Method for Efficacy of Sanitizers Recommended for Inanimate Non-Food Contact Surfaces" according to ASTM standard E1153-03 was used. The test method is referred to as non-food contact sanitizer test or the "NFC test".

*Residual self-sanitizing test:* To assess the antimicrobial activity of coating compositions for a situation where microbial contamination comes into contact with the already dry coating, the following residual self-sanitizing test method was used. The test method is referred to as the residual self-sanitizing test or the "RSS test". Non-porous, pre-cleaned, stainless steel (type SS316) coupons of 25.4 x 25.4 mm in size were used for the test. From a stock plate (transferred three times consecutively but no more than 30 transfers) of the organism, a colony was selected and placed into 10 mL of AOAC Nutrient Broth (NaCl 2.5 g; Beef Extract 2.5 g; Anatone 5 g; Deionized water 500 mL). The inoculated culture was incubated under static conditions for 24 hr at 35°C. In preparation of the test inoculum, the static culture was agitated vigorously using a Vortex mixer, allowed to stand for 15 min, and the upper two-thirds of the culture was transferred to a sterile tube (approximately 6 mL). An organic soil load of fetal bovine serum (FBS) was added for a final concentration of 5 wt% organic soil load to this test inoculum. The final test inoculum density was approximately 1 x 10⁸ CFU/mL.

The test coupons were placed in 70 wt% ethanol overnight, soaked in a mild detergent for at least 30 min, rinsed with tap water thoroughly and allowed to air dry. All handling of surfaces, once cleaned, was done using sterile forceps. Coupons were sprayed with 70 wt% ethanol and allowed to dry completely. An aliquot (50 µL) of the coating composition to be tested was applied to each stainless steel coupon, spread evenly with a sterile plastic spreader, placed in a sterile plastic petri dish and allowed to air dry overnight in the biological safety cabinet. Ambient air temperature and relative humidity were recorded. Uncoated coupons were used as control surfaces which were handled under the same conditions as the coupons treated with the coating compositions.

Coupons were inoculated by spotting 0.01 mL of the inoculum using at least 30 spots over the surface of the coated coupon. At least two replicate coupons were inoculated per coating composition. After 5 min, the test coupons were aseptically transferred to 20 mL of Bacto™ D/E neutralizing broth. The tubes were shaken vigorously by hand, sonicated for 10 s in a sonicating water bath at the maximum setting, and finally shaken for 4 min on a rotary shaker at 250 rpm. The cell density of the culture was determined using a serial-dilution spread plate technique with Butterfield Phosphate Buffer dilution tubes and Trypticase™ Soy Agar (TSA) petri plates. All samples were enumerated within approximately 30 min of their transfer to the D/E Neutralizing Broth.

Average cell densities (CFU/mL) for replicate measurements for both treated and untreated coupons were calculated as the geometric mean of the individual CFU measurements. All log numbers are base-10 logarithms.

### EXAMPLES

The following examples are provided to demonstrate preferred aspects of the invention. It should be appreciated by those of skill in the art that the techniques disclosed in the Examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### Abbreviations used in the Examples

The following abbreviations in the specification correspond to units of measure, techniques, properties, or compounds as follows: "sec" or "s" means second(s), "min" means minute(s), "h" or "hr" means hour(s), "mm" means millimeter; "µL" means microliters, "mL" means milliliters, "L" means liters, "mM" means millimol per liter, "M" means mol per liter, "mmol" means millimole(s), "°C" means degrees Celsius; "ppm" means parts per million and refers to mg/L (milligrams per liter) in the following examples, "wt" means weight, "wt%" indicates percent by weight, "g" means grams, "µg" means micrograms, "g" means earth gravitational constant, "HPLC" means high performance liquid chromatography, "dd H₂O" means distilled and deionized water, "DI" means deionized, "dcw" means dry cell weight, "ATCC" or "ATCC®" means the American Type Culture Collection (Manassas, VA, USA), "U" means units of perhydrolase activity, "U/mg protein" means unit of perhydrolase activity per milligram protein, "rpm" means revolutions per minute, "EDTA" means ethylenediaminetetraacetic acid, "PAA" means peracetic acid, "PEG" means polyethylene glycol, "Pa·s" means pascal seconds; "IPTG" means isopropyl-beta-D-thiogalactopyranoside; "MPa" means megapascals; "NMWCO" indicates nominal molecular weight cut-off, "LB" means Luria broth medium, "OD" means optical density, "g feed/min" means gram feed per minute, "SDS" means sodium dodecyl sulfate, "o-DA" means orthodianisidine, "pNPA" means *para*-nitrophenyl acetate, "L·cm⁻¹·mol^{-1"} means liter per centimeter per mole, "mg/mL" means milligram per milliliter, "kg" means kilogram, U/mg" means units per milligram; "CFU" means colony forming unit; "FBS" means Fetal bovine serum; "log CFU" means the base 10 logarithm of the CFU number; "CFU/mL" means CFU per milliliter; "NFC" means Non-food contact sanitizer test; "RSS" means residual self-sanitizing activity; "SS316" means stainless steel, type 316 (ASTM standard); "TAED" means tetraacetylethylene diamine.

### Chemicals

All chemicals were obtained from Sigma-Aldrich (St. Louis, MO, USA) unless stated otherwise. Elvanol® 51-04 (partially hydrolyzed grade polyvinyl alcohol, 88% grade) was from DuPont (Wilmington, DE, USA). Polyethylene glycol of an average molecular weight of 300 g/mol (Carbowax™ PEG-300) was from Dow (Midland, MI, USA). BTC® 885, Onyxide® 3300 and Biosoft® N25-7 were from Stepan (Northfield, IL, USA). BTC® 885 contains 50 wt% quaternary ammonium compounds as antimicrobial agents and 50 wt% inert materials. Rheovis® FRC was obtained from Ciba (Basel, Switzerland). Glucopon® 215 UP was obtained from Cognis Corporation (Cincinnati, OH). PC 5450 NF was from Performance Chemicals, LLC (Concord, NH). Tetraacetylethylenediamine (TAED B675) was from Warwick International Ltd. (Flintshire, UK). Sodium percarbonate (Provox@ C) was from OCI Chemical Corp. (Decatur, AL, USA). Turpinal® SL was obtained from Thermphos (Vlissingen, the Netherlands). Silwet® L-77 was obtained from Setre Chemical Company (Memphis, TN, USA). Microbial growth media were obtained from DIFCO Laboratories (Detroit, MI, USA), GIBCO/BRL (Gaithersburg, MD, USA), TCI America (Portland, OR, USA), Roche Diagnostics Corporation (Indianapolis, IN, USA) or Sigma-Aldrich Chemical Company (St. Louis, MO, USA). Bacto™ D/E neutralizing broth was from Difco (Cat. No. 281910, Difco™ Laboratories, Detroit, MI, USA).

### pNPA microplate assay

Into each of four wells of a 96-well microplate were added 170 µL of potassium phosphate buffer (50 mM, pH 7.2) and either 10 µL of 0.1 mg total protein/mL of cell lysate, or 10 µL of 0.02 mg total protein/mL of a spray-dried enzyme solution. The plate was covered and placed in a microplate reader (Molecular Devices SPECTRAmax® 384 Plus), then mixed for several seconds and equilibrated at 30 °C for 10 minutes. To each well of the plate was then simultaneously added 20 µL of a 30 mM pNPA solution prepared by mixing (immediately prior to use) 0.6 mL of 100 mM pNPA in acetonitrile with 1.4 mL of 50 mM potassium phosphate buffer (pH 7.2). The change in absorbance at 400 nm was measured over 1 minute of reaction time. The background rate of pNPA hydrolysis (subtracted from the rate of hydrolysis measured in the presence of enzyme) was separately measured in four wells by substituting 10 µL/well of potassium phosphate buffer (50 mM, pH 7.2) for cell lysate or enzyme-containing solution. The extinction coefficient for para-nitrophenol, measured at pH 7.2 in a 1-cm path length cell, was 10,909 L·cm⁻¹·mol⁻¹. One pNPA Unit of enzyme activity is equivalent to the amount of protein that catalyzes the hydrolysis of one micromole of pNPA in one minute.

### Determination of rheoloqical properties

The rheological properties of the liquid antimicrobial formulations were assessed using a Brookfield Digital Viscometer Model DV-II (Brookfield Engineering Laboratories, Middleboro, MA, USA) with RV Series spindle #6 and a tall glass beaker. Samples were loaded by pouring into the glass beaker. Viscosity measurements were taken at different rpm.

### High performance chromatography (HPLC)

The concentration of PAA in the reaction mixtures was determined using HPLC. A Supelco Discovery C8 column (15 cm x 4.0 mm, 5 µm) with precolumn Supelco Superguard Discovery C8 was used. The injection volume was 10 µL. The gradient method with acetonitrile and deionized water at 1.0 mL/min and ambient temperature is shown in the Table below.

| Time (min:sec) | % CH₃CN |
|---|---|
| 0:00 | 41 |
| 3:00 | 41 |
| 3:10 | 100 |
| 6:00 | 100 |
| 6:10 | 41 |
| 10:00 (stop) | 41 |

Retention times of compounds of interest, using this HPLC method, were: MTSO (methyl p-tolyl sulfoxide) = 2.34 min; DEET (N,N-diethyl-m-toluamide) = 4.40 min; TPPO = (triphenylphosphine oxide) = 5.02 min; MTS = (methyl p-tolyl sulfide) = 5.99 min and TPP = (triphenylphosphine) = 6.48 min.

### EXAMPLE 1

### CLONING AND EXPRESSION OF ACETYL XYLAN ESTERASE FROM THERMOTOGA NEAPOLITANA

A coding region encoding an acetyl xylan esterase from *Thermotoga neapolitana* (GENBANK® accession # AAB70869, SEQ ID NO:7) was synthesized using codons optimized for expression in *E*. *coli* (DNA 2.0, Menlo Park, CA, USA). The coding region was subsequently amplified by PCR (0.5 min at 94 °C, 0.5 min at 55 °C, 1 min at 70 °C, 30 cycles) using primers identified as SEQ ID NO:18 and SEQ ID NO:19. The resulting nucleic acid product (SEQ ID NO:20) was subcloned into pTrcHis2-TOPO® (Invitrogen, Carlsbad, CA, USA) to generate the plasmid identified as pSW196. The plasmid pSW196 was used to transform *E*. *coli* KLP18 to generate the strain identified as KLP18/pSW196 (See Published U.S. Patent Application No. 2008/0176299 incorporated herein by reference in its entirety). KLP18/pSW196 was grown in LB medium at 37 °C with shaking up to an OD₆₀₀ₙₘ of 0.4-0.5, at which time isopropyl-beta-D-thiogalactopyranoside (IPTG) was added to a final concentration of 1 mM, and incubation continued for 2-3 h. Cells were harvested by centrifugation and stored in 20 wt% glycerol at -80 °C; the expression of perhydrolase at 20-40% of total soluble protein was determined using SDS-PAGE.

### EXAMPLE 2

### FERMENTATION OF E. COLI KLP18/PSW196 EXPRESSING T. NEAPOLITANA PERHYDROLASE

A fermentation was performed in three stages: pre-seed preparation in a flask, seed preparation in a 14-L fermenter, and scale-up in a 200-L fermenter. The seed culture was prepared by charging a 2-L shake flask with 0.5 L seed medium containing yeast extract (Difco, 1.0 g/L), K₂HPO₄ (10.0 g/L), KH₂PO₄ (7.0 g/L), sodium citrate dihydrate (1.0 g/L), (NH₄)₂SO₄ (4.0 g/L), MgSO₄ heptahydrate (1.0 g/L) and ferric ammonium citrate (0.10 g/L). The pH of the medium was adjusted to 6.8 and the medium was sterilized in the flask. Post sterilization additions included glucose (50 wt%, 10.0 mL) and 1 mL ampicillin (25 mg/mL) stock solution. The seed medium was inoculated with a 1-mL culture of *E*. *coli* KLP18/pSW196 in 20% glycerol, and cultivated at 35 °C and 300 rpm.

The seed culture was transferred to a 14-L seed fermenter (Braun, B. Braun Biotech Inc., Allentown, PA, USA) at an optical density at 550 nm of about 1-2 with 8 L of medium containing KH₂PO₄ (5.0 g/L), FeSO₄ heptahydrate (0.05 g/L), MgSO₄ heptahydrate (1.0 g/L), sodium citrate dihydrate (1.90 g/L), Biospumex153K antifoam (0.5 mL/L, Cognis Corporation), NaCl (1.0 g/L), CaCl₂ dihydrate (0.1 g/L), and a trace elements solution (10 mL/L) containing citric acid monohydrate (10 g/L), MnSO₄ hydrate (2 g/L), NaCl (2 g/L), FeSO₄ heptahydrate (0.5 g/L), ZnSO₄ heptahydrate (0.2 g/L), CuSO₄ pentahydrate (0.02 g/L) and NaMoO₄ dihydrate (0.02 g/L). Post sterilization additions included glucose solution (50 wt%, 80.0 g), ampicillin (25 mg/mL) stock solution (16.00 mL) and thiamine (3.3 g/L) stock solution (6.00 mL). The dissolved oxygen (DO) concentration was controlled at 25% of air saturation. The DO was controlled first by impeller agitation rate (400 to 1400 rpm) and later by aeration rate (2 to 10 L/min at standard conditions). The temperature was controlled at 35 °C. The pH was controlled at 6.8. NH₄OH (29 wt%) and H₂SO₄ (200 g/L) were used for pH control. The overhead pressure was maintained at 0.50 bar. When the culture density reached an OD₅₅₀ nm of more than 5 or when glucose concentration in the broth decreased to less than 1 g/L, the 8 L culture was transferred to the 200 L Sartorius Biostet-DCU-D production fermenter.

The initial volume of the medium charged to the 200-L Sartorius Biostet-DCU-D production fermenter (Sartorius Stedium North America Inc., Bohemia, NY, USA) was 150 L, where the medium was prepared using the same ingredients and concentrations as in the 14-L seed fermenter. Post sterilization additions included glucose-MgSO₄ solution (glucose 50 wt% and MgSO₄ heptahydrate 1 wt%; 1600 g), ampicillin (25 g/L) stock solution (3200 mL) and thiamine (3.3 g/L) stock solution (120 mL). The dissolved oxygen (DO) concentration was controlled at 25% of air saturation. The DO was controlled first by impeller agitation rate (200 to 450 rpm) and later by aeration rate (35 to 80 L/min at standard conditions). The temperature was controlled at 35 °C. The pH was controlled at 6.8. NH₄OH (29 wt%) and H₂SO₄ (200 g/L) were used for pH control. The overhead pressure was maintained at 0.50 bar. A glucose-MgSO₄ solution (glucose (50 wt%) and MgSO4 heptahydrate (1 wt%) was used for fed batch growth. Glucose feed was initiated when glucose concentration decreased to 0.5 g/L, starting at a rate of 6.30 g feed/min and increasing progressively each hour to 7.30, 8.50, 9.8, 11.40, 13.30, 15.40, 17.2 g/min, respectively; the rate remained constant afterwards. Glucose concentration in the medium was monitored and if the concentration exceeded 0.1 g/L the feed rate was decreased or stopped temporarily. Induction was initiated at an OD₅₅₀ nm of 75 (at 26.7 h) with addition of 180 mL IPTG (0.5 M). The cells were harvested by centrifugation 16 h post IPTG addition. The fermentation was terminated at 42.7 h, and about 21 kg wet cells paste was harvested having perhydrolase specific activity of 3.4 pNPA U/mg cells dry weight.

### EXAMPLE 3

### PREPARATION OF T. NEAPOLITANA PERHYDROLASE

Cell paste (13 kg; *E*. *coli* KLP18/pSW196, prepared as described in Example 2) was suspended in 40 L of 50 mM sodium phosphate buffer at pH 7.4, the cell suspension homogenized using a APV 16.56 homogenizer (APV Fluid Handling and Homogenizers, Delavan, WI, USA) operating at 83 MPa and room temperature, and the resulting homogenate heated to 65 °C and held at this temperature for 30 minutes prior to cooling to room temperature. A 0.1 micrometer hollow-fiber membrane cartridge (GE Healthcare, Little Chalfont, United Kingdom) was used to separate the solids from the protein solution. The clarified protein solution was then concentrated using a 30,000 NMWCO hollow-fiber membrane cartridge (GE Healthcare) to obtain 14.7 L of final concentrate which was frozen at - 80 °C. This material was subsequently thawed and further concentrated (30,000 NMWCO) to give 7.5 liters of solution containing approximately 256 grams of protein (34.15 g protein /L; determined using a Micro-BCA protein assay (Sigma Life Sciences, Sigma-Aldrich Corp., St. Louis, MO, USA). To this solution was added 500 g Maltrin M100 maltodextrin, 110 g Maltrin M250 corn syrup solids, and 110 g Maltrin M040 maltodextrin (all from Grain Processing Corporation (GPC), Muscatine, IA, USA).

The resulting mixture (ca. 7.9 kg) was spray dried (inlet temperature = 225 °C, exit temperature = 76 °C, feed rate = 60 g/min) to produce a total of 786 grams of solids (68% yield) of enzyme powder: 93.2 % dry weight, 20.3 wt% protein, 34.5 pNPA U/mg protein.

### EXAMPLE 4

### IN SITU GENERATION OF PERACETIC ACID IN THE PRESENCE OF POLYVINYL ALCOHOL WITH AN ENZYME HAVING PERHYDROLASE ACTIVITY

This Example illustrates the *in situ* generation of peracetic acid with an enzyme having perhydrolase activity in the presence of polyvinyl alcohol. The reaction was performed using a perhydrolase enzyme isolated from *T. neapolitana.* Into a 75 mL glass bottle equipped with a magnetic stir bar, was placed 12.3 mL of 0.2 M sodium bicarbonate buffer (pH 7.2; 44 mM), 36.2 mL of DI water, 41.7 mg of Turpinal® SL (Thermphos, Vlissingen, the Netherlands), 0.95 mL of triacetin (90 mM) and 6.0 g of Elvanol® 51-04 (20% aqueous solution, DuPont, Wilmington, DE, USA) and stirred at room temperature until the sample was homogeneous. At this time, 0.51 mL of 30 wt% hydrogen peroxide (89 mM) was added, followed immediately by 125 µL of 20 mg/mL *T*. *neapolitana* perhydrolase enzyme in 50 mM sodium bicarbonate buffer (pH 7.2). The hydrogen peroxide and peracetic acid concentrations were monitored over time using the cerium sulfate/sodium thiosulfate titration analysis (Greenspan, F. P.; MacKeller, D. G. Anal. Chem., 20: 1061-1063, 1948). Control reactions were performed with (12.3 mL 0.2 M sodium bicarbonate buffer, pH 7.2, 50 mM; 0.51 mL 30% hydrogen peroxide, 100 mM; 36.2 mL DI water; 41.7 mg Turpinal® SL; 0.95 mL triacetin, 0.1 M; 125 ilL of 20 mg/mL of *T. neapolitana* perhydrolase enzyme (based on wt% protein in enzyme powder) in 50 mM sodium bicarbonate buffer, pH 7.2) and without added enzyme (identical to above control conditions without enzyme). Over a 30 minute time period, the control reaction performed with triacetin and hydrogen peroxide with no added enzyme produced 114 ppm peracetic acid from the chemical reaction of triacetin and hydrogen peroxide. The control reaction performed with triacetin and hydrogen peroxide with added enzyme produced 380 ppm peracetic acid within 1 minute and increased over 30 minutes to 2280 ppm. The *in situ* enzymatic production of PAA in the presence of polyvinyl alcohol produced 456 ppm within 1 minute and increased to 1976 ppm over 30 minutes. Table 1 shows the concentrations of PAA produced from *in situ* enzymatic production of PAA in the presence of a film-former.

**TABLE 1**

| Enzymatic production of PAA in the presence of polyvinyl alcohol | | | |
|---|---|---|---|
| | 1 min (ppm) | 5 min (ppm) | 30 min (ppm) |
| Control (no enzyme) | 114 | 114 | 114 |
| Control (with enzyme) | 380 | 1178 | 2280 |
| Elvanol® 51-04 | 456 | 798 | 1976 |

### EXAMPLE 5

### IN SITU GENERATION OF PERACETIC ACID IN A FILM-FORMING COMPOSITION WITH AN ENZYME HAVING PERHYDROLASE ACTIVITY

This example illustrates the *in situ* generation of peracetic acid with an enzyme having perhydrolase activity in the presence of a film-forming composition with additional performance-enhancing additives. The reaction was performed using the perhydrolase enzyme from *T*. *neapolitana.* Into a 75 mL glass bottle equipped with a magnetic stir bar, was placed 12.3 mL of 0.2 M sodium bicarbonate buffer (pH 7.2; 44 mM), 36.2 mL of deionized water, 41.7 mg of Turpinal® SL (Thermphos, Vlissingen, the Netherlands), 0.95 mL of triacetin (89 mM), 6.0 g of Elvanol® 51-04 (20 wt% aqueous solution, DuPont, Wilmington, DE, USA), 0.5 g of PEG-300 (Dow, Midland, MI, USA) and 0.25 g Silwet® L-77 (Setre Chemical Company, Memphis, TN, USA) and stirred at room temperature until the sample was homogeneous. At this time, 0.51 mL of 30% hydrogen peroxide (88 mM) was added, followed immediately by 125 µL of 20 mg/mL of *T. neapolitana* perhydrolase enzyme (based on wt % protein in enzyme powder) in 50 mM sodium bicarbonate buffer (pH 7.2). The hydrogen peroxide and peracetic acid concentrations were monitored over time using the cerium sulfate/sodium thiosulfate titration test (Greenspan, F. P.; MacKeller, D. G. Anal. Chem., 20: 1061-1063, 1948). Control reactions were performed with (12.3 mL 0.2 M sodium bicarbonate buffer, pH 7.2, 50 mM; 0.51 mL 30% hydrogen peroxide, 100 mM; 36.2 mL DI (deionized water) water; 41.7 mg Turpinal® SL; 0.95 mL triacetin, 0.1 M; 125 µL of 20 mg/mL of *T. neapolitana* perhydrolase enzyme (based on wt % protein in enzyme powder) in 50 mM sodium bicarbonate buffer, pH 7.2) and without added enzyme (identical to above control conditions without enzyme). Over a 30 minute time period, the control reaction performed with triacetin and hydrogen peroxide with no added enzyme produced 114 ppm peracetic acid from the chemical reaction of triacetin and hydrogen peroxide. The control reaction performed with triacetin and hydrogen peroxide with added enzyme produced 380 ppm peracetic acid within 1 minute and increased over 30 minutes to 2280 ppm. The *in situ* enzymatic production of PAA in a film-forming composition produced 342 ppm within 1 minute and increased to 1900 ppm over 30 minutes. Table 2 shows the concentrations of PAA produced from *in situ* enzymatic production of PAA in the presence of a film-forming composition with additional performance-enhancing additives.

**TABLE 2**

| Enzymatic production of PAA in a film-forming composition | | | |
|---|---|---|---|
| | 1 min (ppm) | 5 min (ppm) | 30 min (ppm) |
| Control (no enzyme) | 114 | 114 | 114 |
| Control (with enzyme) | 380 | 1178 | 2280 |
| Elvanol® 51-04/PEG-300/Silwet® L-77 | 342 | 1102 | 1900 |

### EXAMPLE 6

### IN SITU ENZYMATIC GENERATION OF PERACETIC ACID IN A FILM-FORMING COMPOSITION CONTAINING A SECOND BIOCIDE AND A RHEOLOGY MODIFIER

This example illustrates the *in situ* generation of peracetic acid with an enzyme having perhydrolase activity in the presence of a film-forming composition that contains a rheology modifier and a second biocide. Into a 100 mL glass bottle equipped with a stir bar, was placed 25 g of a 20 wt% aqueous solution of Elvanol® 51-04, 14 g of deionized water, 0.8 g of Rheovis® FRC, 0.5 g of PEG 300 and 0.15 g BTC® 885 and stirred at room temperature until the sample was homogeneous. At this time, 1.09 g of triacetin (100 mM) and 0.67 g of 30 wt% hydrogen peroxide (100 mM) were added and stirred to mix. The pH was adjusted to above 7.0 with addition of 8 g of 0.2 M sodium bicarbonate buffer (32 mM). Following the pH adjustment, 125 µL of *T. neapolitana* perhydrolase enzyme (20 mg/mL solution (based on wt % protein in enzyme powder) in 50 mM sodium bicarbonate buffer) was added and the timer started. A control reaction was performed identical to the above conditions, except without enzyme. The peracetic acid concentrations were monitored over time at 5 minutes, 30 minutes and 1 hour using the cerium sulfate/sodium thiosulfate titration test. Over a 1 hour time period, the control reaction with no added enzyme produced 487 ppm peracetic acid from the chemical reaction of triacetin and hydrogen peroxide. The *in situ* enzymatic production of PAA in a film-forming composition produced 2112 ppm within 5 minutes and increased to 2593 ppm over 1 hour. Table 3 shows the concentrations of enzymatically generated PAA in a film-forming composition.

**TABLE 3**

| PAA produced enzymatically in a film-forming composition containing a second biocide and a rheology modifier | | | |
|---|---|---|---|
| | 5 min (ppm) | 30 min (ppm) | 60 min (ppm) |
| No enzyme | 206 | 258 | 487 |
| *T. neapolitana* | 2112 | 2446 | 2593 |

### EXAMPLE 7

### SHEAR-THINNING INDEX OF A REMOVABLE ANTIMICROBIAL COATING COMPOSITION

The "pseudoplastic index" or "shear thinning index" (STI) provides an indication as to resistance of the composition to sagging and dripping. A common measurement determines the viscosity at two different shear rates. The value recorded at the lower shear rate is divided by the value at the higher shear rate obtain the STI. Generally, the higher the STI, the higher the resistance to sagging and dripping the coating material will have. The STI values as used in herein were determined by measuring the viscosities 30 minutes after addition of the enzyme. The viscosities were measured at room temperature using a Brookfield Digital Viscometer Model DV-II (Brookfield Engineering Laboratories, Middleboro, MA, USA) with RV Series spindle #6. The shear-thinning index (STI) was calculated by dividing the viscosity measured at 1 rpm by the viscosity measured at 10 rpm. The STI for the inventive coating composition A (see Table 4 for composition) is given in Table 5. At 1 rpm the viscosity of film-forming composition A was 12.8 Pa·s and decreased to 4.86 Pa·s at 10 rpm. The shear-thinning index for this coating composition is 2.74.

**TABLE 4**

| Coating composition A for enzymatic peracid production | |
|---|---|
| Ingredient | wt% |
| Elvanol® 51-04 (20 wt% solution) | 50 |
| DI water | 26.94 |
| Sodium bicarbonate (0.2 M) | 16 |
| Tricetin | 2.18 |
| Rheovis® FRC | 2.2 |
| Hydrogen peroxide (30 wt%) | 1.13 |
| PEG-300 | 1.0 |
| BTC® 885 | 0.3 |
| *T. neapolitana* | 0.25 |
| (20 mg/mL in 50 mM NaHCO₃) | |

**TABLE 5**

| Viscosities and shear-thinning index | | | | |
|---|---|---|---|---|
| Coating composition | Temperature (°C) | Viscosity at 1 rpm (Pa·s) | Viscosity at 10 rpm (Pa·s) | STI |
| A *(T. neapoliana)* | 25 | 12.8 | 4.68 | 2.74 |

### EXAMPLE 8

### CLONING AND EXPRESSION OF ACETYL XYLAN ESTERASE FROM THERMOTOGA MARITIMA

A gene encoding acetyl xylan esterase from *T. maritima* MSB8 as reported in GENBANK® (accession # NP_227893.1, SEQ ID NO: 8) was synthesized (DNA 2.0, Menlo Park, CA, USA). The gene was subsequently amplified by PCR (0.5 min at 94 °C, 0.5 min at 55 °C, 1 min at 70 °C, 30 cycles) using primers identified as SEQ ID NO: 22 and SEQ ID NO: 23. The resulting nucleic acid product (SEQ ID NO: 24) was cut with restriction enzymes *Pst*I and *Xba*I and subcloned between the *Pst*I and *Xba*I sites in pUC19 to generate the plasmid identified as pSW207. The codon optimized version of *T. maritima* MSB8 acetyl xylan esterase for expression in *E*. *coli* was synthesized and subsequently amplified by PCR (0.5 min at 94 °C, 0.5 min at 55 °C, 1 min at 70 °C, 30 cycles) using primers identified as SEQ ID NOs:25 and 26. The resulting nucleic acid product (SEQ ID NO: 27) was cut with restriction enzymes EcoRI and *Pst*I and subcloned between the *EcoR*I and *Pst*I sites in pTrc99A (GENBANK® Accession no. M22744) to generate the plasmid identified as pSW228. The plasmids pSW207 and pSW228 were used to transform *E. coli* KLP18 (U.S. Patent Application Pub. 2008/0176299 herein incorporated by reference) to generate the strain identified as KLP18/pSW207 and KLP18/pSW228, respectively. KLP18/pSW207 and KLP18/pSW228 were grown in the LB medium at 37 °C with shaking up to OD₆₀₀ₙₘ 0.4-0.5, at which time IPTG was added to a final concentration of 1 mM, and incubation continued for 2-3 h. Cells were harvested by centrifugation and SDS-PAGE was performed to confirm expression of the perhydrolase at 20-40% of total soluble protein.

### EXAMPLE 9

### CONSTRUCTION OF THERMOTOGA MARITIMA ACETYL XYLAN ESTERASE MUTATION AT RESIDUE C277

The C277 (Cys277) position of *T. maritima* acetyl xylan esterase was changed to Ser using an oligonucleotide primer pair (SEQ ID NOs: 28 and 29) that was designed based on the codon optimized sequence of *T. maritima* acetyl xylan esterase (SEQ ID NO: 30) in the plasmid pSW228. The mutation was made using Quikchange (Stratagene, Cedar Creek, TX, USA) according to the manufacturer's instructions. Amplified plasmids were treated with 1 U of DpnI at 37 °C for 1 hour. Treated plasmids were used to transform chemically competent *E*. *coli* XL1-Blue (Stratagene). Transformants were streaked on LB agar plates supplemented with 0.1 mg ampicillin/mL and grown overnight at 37 °C. Up to five individual colonies were picked and the plasmid DNA sequenced to confirm the expected mutation.

### EXAMPLE 10

### EXPRESSION OF THERMOTOGA MARITIMA ACETYL XYLAN ESTERASE C277S MUTANT IN E. COLI KLP18

Plasmids with the confirmed acetyl xylan esterase C277S mutation were used to transform *E*. *coli* KLP18 (Example 1) to generate the strain identified as KLP18/pSW228/C277S. Transformants were gown in the LB medium at 37 °C with shaking up to OD₆₀₀ₙₘ 0.4-0.5, at which time IPTG was added to a final concentration of 1 mM, and incubation continued for 2-3 h. Cells were harvested by centrifugation and SDS-PAGE was performed to confirm expression of the acetyl xylan esterase C277S mutant at 20-40% of total soluble protein.

### EXAMPLE 11

### PREPARATION OF SPRAY-DRIED T. MARITIMA ACETYL XYLAN ESTERASE WILD TYPE AND ITS MUTANT C277S

Fermentation protocol used to grow *E*. *coli* KLP18/pSW228 (to produce *T. maritima* acetyl xylan esterase) or *E. coli* KLP18/pSW228/C277S (to produce *T. maritima* acetyl xylan esterase C277S mutant) was similar to that described in Example 2, except that it was performed at a 10-L scale. Cell pastes were harvested by centrifugation at 5,000 x *g* for 15 minutes, and stored frozen at -80 °C for later use.

A sample of the cell paste was resuspended (200 g/L) in 50 mM phosphate buffer pH 7.0 supplemented with 1.0 mM Dithiothreitol (DTT). Resuspended cells were passed through a French Pressure Cell twice to ensure >95% cell lysis. Lysed cells were centrifuged for 30 minutes at 12,000 xg, and the supernatant was heated at 75°C for 20 minutes, followed by quenching in an ice bath for 2 minutes. Precipitated protein was removed by centrifugation for 10 minutes at 11,000 x *g*. SDS-PAGE indicated that the CE-7 enzyme comprised approximately 85-90% of the total protein in the preparation.

A procedure similar to that described in Example 3 was repeated using either cell paste prepared as described in above. The resulting *T. maritima* perhydrolase spray-dried enzyme powder was: 95.5 % dry weight, 27.8 wt% protein, 86.1 pNPA U/mg protein. The resulting *T. maritima* C277S perhydrolase spray-dried enzyme powder was: 95.5 % dry weight, 25.5 wt% protein, 129.5 pNPA U/mg protein.

### EXAMPLE 12

### lN SITU ENZYMATIC GENERATION OF PERACETIC ACID IN A FILM-FORMING COMPOSITION USING MULTIPLE PERHYDROLASES

In this Example the *in situ* enzymatic generation of PAA in a film-forming composition using different suitable perhydrolases to generate PAA and the ability to use a second biocidal active and a rheology modifier in the formulation was demonstrated.

Similar coating compositions as described in Example 6 were made using the same process as described above but using the ingredients and compositions in wt% as listed in Table 6. Coating composition B generates PAA in a film-forming composition using TAED and sodium percarbonate without enzyme and is used for comparison only. Coating composition C lacks enzyme and serves as a control to the enzyme-containing compositions D, E and F. The initial pH of each enzymatic reaction mixture was about 7.0. The initial pH of the TAED reaction mixture was about 8.0.

Determination of the concentration of PAA in the reaction mixtures were performed according to the method described by Karst et al. (Anal. Chem., 69: 3623-3627,1997). At the desired time points (1 min, 5 min, etc.), an aliquot of the reaction mixture was removed (approx. 25 - 50 mg) and diluted by weight with 5 mM phosphoric acid to terminate the reaction. A typical dilution was 1:35 for samples with PAA concentration greater than 2000 ppm, 1:25 for samples with PAA concentration around 1000 ppm, and 1:10 for samples with PAA concentration below 200 ppm. Approximately 200 µL (or more to completely fill the filter cup) of the diluted aliquot was transferred to a non-sterile 10,000 NMWL (nominal molecular weight limit) filter unit (Nanosep 10K Omega, PALL Life Sciences, East Hills, NY, USA) and filtered by centrifugation at 12,000 rpm for 7 minutes. An aliquot (100 µL) of the resulting filtrate was transferred to a 1.8-mL screw cap HPLC vial (1.8 mL ROBO Autosampler Vial, VWR International, West Chester, PA, USA) containing 300 µL of deionized water, then 100 µL of 20 mM MTS (methyl-p-tolyl-sulfide) in acetonitrile was added, the vials capped, and the contents briefly mixed prior to a 10 minute incubation at about 25 °C in the absence of light. To each vial was then added 400 µL of acetonitrile and 100 µL of a solution of triphenylphosphene (120 mM) in acetonitrile, the vial recapped, and the resulting solution mixed and incubated at about 25 °C for 30 minutes in the absence of light. To each vial was then added 100 µL of 2.5 mM N,N-diethyl-m-toluamide (DEET; HPLC external standard) and the resulting solution analyzed by HPLC as described above.

The PAA concentrations over time are given in Table 7. The maximum observed concentrations of PAA for all enzyme-containing as well as the TAED-containing compositions was observed between 0.5 and 2 hours after initiating the respective chemical reaction. After the maximum concentrations have been reached, the concentration decreases with a time profile that can be mathematically approximated by an exponential decay function of the form c(t) = k·exp [-t/(In(2)·τ)], wherein c(t) describes the concentration of PAA as a function of the reaction time t. The parameters k and τ are constants that may differ for each composition with τ indicating the approximate half-time, i.e., the time at which the PAA concentration has decayed to half its maximum value. The approximate half times were calculated by fitting the PAA concentration data over time for times between 2 and 120 hours to the above exponential decay function using the method of least squares. The half times τ are also given in Table 7. As can be seen, the half times for the enzymatically generated PAA are similar and between about 27 and 29 hours. In contrast, the half time for the non-enzymatically generated PAA of Coating Composition B is more than 7 hours shorter. Hence, the enzymatically generated compositions provide for slower reduction of the PAA concentrations in the liquid coating composition over time. This can be beneficial in situations where a specific minimum PAA concentration is desired to be present over a longer period of time.

**TABLE 6**

| Coating compositions B to F | | | | | |
|---|---|---|---|---|---|
| Ingredient | Concentration (wt%) | | | | |
| | Coating composition B (control, TAED) | Coating composition C (control, no enzyme) | Coating composition D | Coating composition E | Coating composition F |
| Elvanol® 51-04 | 10 | 10 | 10 | 10 | 10 |
| Rheovis® FRC | 1.6 | 1.6 | 1.6 | 1.6 | 1.6 |
| PEG-300 | 1.0 | 1.0 | 1.0 | 1.0 | 1.0 |
| BTC® 885 | 0.3 | 0.3 | 0.3 | 0.3 | 0.3 |
| Triacetin | - | 2.18 | 2.18 | 2.18 | 2.18 |
| Hydrogen peroxide (30 wt%) | - | 1.13 | 1.13 | 1.13 | 1.13 |
| Sodium bicarbonate (0.2M) | - | 16 | 16 | 16 | 16 |
| *T. neapolitana* wild type perhydrolase (20 mg/mL) | - | - | 0.25 | - | - |
| *T. maritima* wild type perhydrolase (20 mg/mL) | - | - | - | 0.25 | - |
| *T. maritima* C277S mutant perhydrolase (20 mg/mL) | - | - | - | - | 0.05 |
| TAED B 675 | 0.69 | - | - | - | - |
| Provox® C (88 wt% sodium percarbonate) | 0.72 | - | - | - | - |
| DI water | remainder | remainder | remainder | remainder | remainder |

**TABLE 7**

| PAA produced enzymatically in a film-forming composition using multiple perhydrolases | | | | | | | | | | |
|---|---|---|---|---|---|---|---|---|---|---|
| Coating composition | 1 min (ppm) | 5 min (ppm) | 0.5 h (ppm) | 1 h (ppm) | 2h (ppm) | 6h (ppm) | 24 h (ppm) | 48 h (ppm) | 120 h (ppm) | Half-time τ (hours) |
| B (TAED) | 2512 | 2864 | 2912 | 2829 | 2242 | 1568 | 723 | 263 | 34 | 20.1 |
| C (no enzyme) | 245 | 247 | 289 | 310 | 404 | 540 | 546 | 333 | 92 | n.d. |
| D | 564 | 1149 | 2151 | 2192 | 1855 | 1825 | 1533 | 656 | 115 | 28.4 |
| E | 954 | 1878 | 2530 | 2653 | 2513 | 2327 | 1531 | 787 | 126 | 27.1 |
| F | 387 | 1401 | 2399 | 2484 | 2402 | 1914 | 1206 | 593 | 111 | 27.1 |

| | | | | | | | | | | |
|---|---|---|---|---|---|---|---|---|---|---|
| n.d. indicates "not determined" | | | | | | | | | | |

### EXAMPLE 13

### THE EFFICACY OF THE FILM-FORMING ANTIMICROBIAL COMPOSITION G ON STAPHYLOCOCCUS AUREUS

The antimicrobial properties of the film-forming composition G (Table 8) were tested using the NFC test method and *Staphylococcus aureus* (ATCC 6538) as the test microorganism. The composition of G in wt% is given in Table 8. Combined reagents after mixing were 100 wt%. A volume of 0.05 mL of the coating composition was applied evenly per coupon. With a five minute exposure, a 5.6 log reduction in colony-forming units (CFU) or greater was observed, which is equivalent to a reduction of the CFU number by at least 99.999%.

**TABLE 8**

| Antimicrobial NFC test on composition G with *S*. *aureus* | |
|---|---|
| Ingredient (wt%) | G |
| Elvanol® 51-04 (20 wt% solution) | 50 |
| DI water | 29.87 |
| Sodium bicarbonate (0.2 M) | 16 |
| Tricetin | 0.78 |
| Rheovis® FRC | 1.6 |
| Hydrogen peroxide (30 wt%) | 0.4 |
| PEG-300 | 1.0 |
| BTC® 885 | 0.3 |
| *T. maritima C277S* mutant | 0.05 |
| Perhydrolase (20 mg/mL in 50 mM NaHCO₃) | |
| Contact time (min) | 5 |
| Log₁₀ CFU reduction | 5.6 |

### EXAMPLE 14

### THE EFFICACY OF THE FILM-FORMING ANTIMICROBIAL COMPOSITION H ON STAPHYLOCOCCUS AUREUS

The antimicrobial properties of the film-forming composition H (Table 9) were tested using the RSS test method and *Staphylococcus aureus* (ATCC 6538) as the test microorganism. The composition of H in wt% is given in Table 9. All combined reagents equaled 100 wt%. A volume of 0.05 mL of the coating composition was applied evenly per coupon. As shown in Table 9, a 3.1 log reduction in CFU was achieved for composition H, which is equivalent to a reduction of the CFU number by at least 99.9%.

**TABLE 9**

| Antimicrobial RSS test on composition H with *S*. *aureus* | |
|---|---|
| Ingredient (wt%) | H |
| Elvanol® 51-04 (20 wt% solution) | 50 |
| DI water | 26.4 |
| Sodium bicarbonate (0.2 M) | 16 |
| Tricetin | 2.18 |
| Rheovis® FRC | 2.2 |
| Hydrogen peroxide (30 wt%) | 1.13 |
| PEG-300 | 1.0 |
| BTC® 885 | 0.3 |
| Glucopon® 215 UP | 0.5 |
| PC 5450 NF | 0.2 |
| Biosoft® N25-7 | 0.01 |
| *T. maritima* C277S mutant | 0.05 |
| Perhydrolase (20 mg/mL in 50 mM NaHCO₃) | |
| Contact time (min) | 5 |
| Log₁₀ CFU reduction | 3.1 |

### EXAMPLE 15

### COMPARISON BETWEEN COMMERCIALLY AVAILABLE PAA SOLUTION AND IN SITU-GENERATED PAA IN A FILM

This example compares the PAA concentration in a film over time between a commercially available equilibrium PAA solution doped into a film-forming composition Pack A and *in situ* generated PAA in a film-forming composition.

The film-forming compositions of H and #141 B in wt% are given in Table 10. Mixing all components results in 100 wt% of the antimicrobial coating composition. For composition #141 B, commercially available PAA (90 µL of a 32 wt% solution) was added to Pack A in lieu of *in situ* generation.

**TABLE 10**

| Film-forming compositions H and #141B | | |
|---|---|---|
| Ingredient (wt%) | H | #141B |
| Elvanol® 51-04 (20 wt% solution) | 50 | 10 |
| DI water | 26.4 | 84.4 |
| Sodium bicarbonate (0.2 M) | 16 | |
| Tricetin | 2.18 | |
| Rheovis® FRC | 2.2 | 2.2 |
| Hydrogen peroxide (30 wt%) | 1.13 | |
| PEG-300 | 1.0 | 1.0 |
| BTC® 885 | 0.3 | 0.3 |
| Glucopon® 215 UP | 0.5 | 0.5 |
| PC 5450 NF | 0.2 | 0.2 |
| Biosoft® N25-7 | 0.01 | 0.01 |
| *T. maritima C277S* mutant | 0.05 | |
| perhydrolase (20 mg/mL in 50 mM NaHCO₃) | | |
| PAA (32 wt%) | | 90 µL |

After mixing all components of the compositions together, films were applied to plastic panels using an 8-path wet film applicator (5 mil film depth, model No. 15, Paul N. Gardner Co. Inc., Pompano Beach, FL, USA). The PAA concentrations in the films were monitored at regular intervals as the film dried. In a typical experiment, the films were tacky to the touch within 30 minutes and completely dry by one hour. Determination of the concentration of PAA in the reaction mixtures were performed according to the method described by Karst *et al.* (Anal. Chem., 69: 3623-3627,1997). See details in example 12.

The PAA concentration in coating composition H was observed to increase from 1925 ppm PAA at 1 minute to a maximum of 2599 ppm PAA at 10 minutes, until dropping to 732 ppm PAA at one hour, while the PAA concentration in composition #141B steadily dropped over one hour from a maximum of 2849 ppm PAA at one minute to 22 ppm PAA at one hour. This example highlights the benefit of the inventive film-forming compositions where PAA is generated in situ rather than previously prepared.

**TABLE 11**

| PAA concentration over time in films of coating compositions H and #141B | | |
|---|---|---|
| Time | H | #141B |
| | ppm | ppm |
| 1 min | 1925 | 2849 |
| 5 min | 2383 | 2656 |
| 10 min | 2599 | 1741 |
| 20 min | 2190 | 534 |
| 30 min | 2001 | 138 |
| 1 h | 732 | 22 |

### EXAMPLE 16

### APPLICATION AND REMOVAL OF COATING COMPOSITION H

Coating composition H (Table 9) was applied to an aluminum panel using an 8-path wet film applicator (5 mil film depth, model No. 15, Paul N. Gardner Co. Inc., Pompano Beach, FL, USA). The resulting coating after drying had an excellent appearance characterized by the absence of coating defects such as sags, foam or bubbles, craters or uncovered areas. The dry film was easily removed by rinsing the panel with 30-32 °C tap water. There was no visible residue left behind on the aluminum panel, demonstrating the good removal characteristics of the film.

## Claims

1. A method of providing control of microorganisms at a locus comprising the steps:
a) forming a composition by combining components comprising:
i) a film-forming water soluble or water-dispersible agent wherein the agent is polyvinyl alcohol, polyvinyl alcohol copolymer or polyvinyl pyrrolidone;
ii) an inert solvent;
iii) a carboxylic acid ester substrate;
iv) a peroxygen source;
v) an enzyme catalyst having perhydrolase activity thereby forming at least one peroxyacid to provide at least a first antimicrobial agent; and
vi) a rheology modifier;
b) applying said composition to said locus; and
c) allowing said composition to dry thereby forming a coating on said locus; wherein said components further comprise a second antimicrobial agent, wherein said second antimicrobial agent comprises a quaternary ammonium compound.

2. The method of Claim 1 wherein said enzyme catalyst is selected from carbohydrate esterases of CE-7 family.

3. The method of Claim 1 , wherein said peroxyacid is produced at a concentration of at least 20 ppm within about 5 minutes to about 2 hours of combining said components.

4. A method of providing control of microorganisms at a locus comprising the steps:
(a) providing a first premixed component comprising an inert solvent and a film-forming agent wherein the agent is polyvinyl alcohol, polyvinyl alcohol copolymer or polyvinyl pyrrolidone;
(b) providing a second premixed component comprising an enzyme catalyst having perhydrolase activity, a carboxylic acid ester substrate and a peroxygen source;
(c) mixing said first premixed component and said second premixed component to obtain a liquid coating composition comprising a first antimicrobial agent comprising a peroxyacid;
(d) applying said coating composition to the locus; and
(e) allowing said coating composition to dry thereby forming a coating on said locus; wherein at least one premixed component further comprises a second antimicrobial agent wherein said second antimicrobial agent comprises a quaternary ammonium compound.

5. The method of Claim 4, wherein said first and second premixed component are provided in a multi-compartment system; wherein the first and second premixed components remain separated before step (c).

6. The method of Claim 4, wherein one premixed component is in liquid form and one premixed component is in solid form.

7. The method of Claim 4 wherein said enzyme catalyst comprises carbohydrate esterases of CE-7 family.

8. An antimicrobial composition comprising components which comprise:
a) a film-forming water soluble or water-dispersible agent wherein the agent is polyvinyl alcohol, polyvinyl alcohol copolymer or polyvinyl pyrrolidone;
b) an inert solvent;
c) a carboxylic acid ester substrate;
d) a peroxygen source;
e) an enzyme catalyst having perhydrolase activity; and
f) a rheology modifier;
wherein, upon combining said components, a peroxyacid is formed;
wherein said components further comprises a component that is an antimicrobial agent comprising a quaternary ammonium compound

9. The composition of Claim 8 wherein at least one or more said components are separately packaged from the other components in a multi-compartment system prior to their combination to form said peroxyacid.

10. The composition of Claim 8, wherein said peroxyacid is produced at a concentration of at least 20 ppm within about 5 minutes to about 2 hours of combining said components.

11. An article comprising a coating on at least one surface thereof with an antimicrobial composition, wherein the antimicrobial composition comprises:
a) a film-forming water soluble or water-dispersible agent wherein the agent is polyvinyl alcohol, polyvinyl alcohol copolymer or polyvinyl pyrrolidone;
b) an inert solvent;
c) a carboxylic acid ester substrate;
d) a peroxygen source;
e) an enzyme catalyst having perhydrolase activity;
f) a rheology modifier; and
g) a quaternary ammonium compound
wherein, upon combining components of (a) through (eg), a peroxyacid is formed.

12. The article of Claim I wherein the article is equipment used in the food or beverage industry.

## Patentansprüche

1. Verfahren zum Bereitstellen der Bekämpfung von Mikroorganismen an einem Ort, wobei das Verfahren die folgenden Schritte aufweist:
a) Bilden einer Zusammensetzung durch Vereinigen von Komponenten, die umfassen:
i) ein filmbildendes wasserlösliches oder wasserdispergierbares Agens, wobei das Agens Polyvinylalkohol, Polyvinylalkohol-Copolymer oder Polyvinylpyrrolidon ist;
ii) ein inertes Lösungsmittel;
iii) ein Carbonsäureester-Substrat;
iv) eine Peroxidquelle;
v) einen Enzymkatalysator mit Perhydrolase-Aktivität, wodurch mindestens eine Peroxysäure gebildet wird, um mindestens einen ersten antimikrobiellen Wirkstoff bereitzustellen; und
vi) einen Viskositätsveränderer;
b) Aufbringen der Zusammensetzung auf den Ort; und
c) Trocknen lassen der Zusammensetzung, um dadurch eine Beschichtung auf dem Ort zu bilden;
wobei die Komponenten ferner einen zweiten antimikrobiellen Wirkstoff aufweisen, wobei der zweite antimikrobielle Wirkstoff eine quartäre Ammoniumverbindung aufweist.

2. Verfahren nach Anspruch 1, wobei der Enzymkatalysator unter Kohlenhydrat-Esterasen der CE-7-Familie ausgewählt ist.

3. Verfahren nach Anspruch 1, wobei die Peroxysäure innerhalb von etwa 5 Minuten bis etwa 2 Stunden nach Vereinigung der Komponenten in einer Konzentration von mindestens 0,002 % (20 ppm) produziert wird.

4. Verfahren zum Bereitstellen der Bekämpfung von Mikroorganismen an einem Ort, wobei das Verfahren die folgenden Schritte aufweist:
(a) Bereitstellen einer ersten vorgemischten Komponente, die ein inertes Lösungsmittel und ein filmbildendes Agens aufweist, wobei das Agens Polyvinylalkohol, Polyvinylalkohol-Copolymer oder Polyvinylpyrrolidon ist;
(b) Bereitstellen einer zweiten vorgemischten Komponente, die einen Enzymkatalysator mit Perhydrolase-Aktivität, ein Carbonsäureester-Substrat und eine Peroxidquelle aufweist;
(c) Vermischen der ersten vorgemischte Komponente und der zweiten vorgemischten Komponente, um eine flüssige Beschichtungszusammensetzung zu erhalten, die einen ersten antimikrobiellen Wirkstoff aufweist, der eine Peroxysäure umfasst;
(d) Aufbringen der Beschichtungszusammensetzung auf den Ort; und
(e) Trocknen lassen der Zusammensetzung, um dadurch eine Beschichtung auf dem Ort zu bilden;
wobei mindestens eine vorgemischte Komponente ferner einen zweiten antimikrobiellen Wirkstoff aufweist, wobei der zweite antimikrobielle Wirkstoff eine quartäre Ammoniumverbindung aufweist.

5. Verfahren nach Anspruch 4, wobei die erste und die zweite vorgemischte Komponente in einem Mehrfächersystem bereitgestellt werden, wobei die ersten und zweiten vorgemischten Komponenten vor Schritt (c) getrennt bleiben.

6. Verfahren nach Anspruch 4, wobei eine vorgemischte Komponente in flüssiger Form vorliegt und eine vorgemischte Komponente in fester Form vorliegt.

7. Verfahren nach Anspruch 4, wobei der Enzymkatalysator Kohlenhydrat-Esterasen der CE-7 Familie aufweist.

8. Antimikrobielle Zusammensetzung, die Komponenten aufweist, die umfassen:
a) ein filmbildendes wasserlösliches oder wasserdispergierbares Agens, wobei das Agens Polyvinylalkohol, Polyvinylalkohol-Copolymer oder Polyvinylpyrrolidon ist;
b) ein inertes Lösungsmittel;
c) ein Carbonsäureester-Substrat;
d) eine Peroxidquelle;
e) einen Enzymkatalysator mit Perhydrolase-Aktivität; und
f) einen Viskositätsveränderer;
wobei nach Vereinigung der Komponenten eine Peroxysäure gebildet wird;
wobei die Komponenten ferner eine Komponente aufweisen, die ein antimikrobieller Wirkstoff ist, der eine quartäre Ammoniumverbindung aufweist.

9. Zusammensetzung nach Anspruch 8, wobei mindestens eine oder mehrere der Komponenten vor ihrer Vereinigung zur Bildung der Peroxysäure in einem Mehrfächersystem getrennt von den anderen Komponenten verpackt sind.

10. Zusammensetzung nach Anspruch 8, wobei die Peroxysäure innerhalb von etwa 5 Minuten bis etwa 2 Stunden nach Vereinigung der Komponenten in einer Konzentration von mindestens 0,002 % (20 ppm) produziert wird.

11. Gegenstand, der auf mindestens einer seiner Oberflächen eine Beschichtung mit einer antimikrobiellen Zusammensetzung aufweist, wobei die antimikrobielle Zusammensetzung aufweist:
a) ein filmbildendes wasserlösliches oder wasserdispergierbares Agens, wobei das Agens Polyvinylalkohol, Polyvinylalkohol-Copolymer oder Polyvinylpyrrolidon ist;
b) ein inertes Lösungsmittel;
c) ein Carbonsäureester-Substrat;
d) eine Peroxidquelle;
e) einen Enzymkatalysator mit Perhydrolase-Aktivität;
f) einen Viskositätsveränderer; und
g) eine quartäre Ammoniumverbindung;
wobei nach Vereinigung der Komponenten (a) bis (g) eine Peroxysäure gebildet wird.

12. Gegenstand nach Anspruch 11, wobei der Gegenstand eine in der Nahrungsmittel- oder Getränkeindustrie verwendete Ausrüstung ist.

## Revendications

1. Procédé de fourniture du contrôle des micro-organismes au niveau d'un lieu comprenant les étapes:
a) de formation d'une composition par combinaison de composants comprenant:
i) un agent soluble dans l'eau ou dispersible dans l'eau formant film dans lequel l'agent est le poly(alcool de vinyle), le copolymère de poly(alcool de vinyle) ou la polyvinyl pyrrolidone;
ii) un solvant inerte;
iii) un substrat ester d'acide carboxylique;
iv) une source de peroxygène;
v) un catalyseur de type enzyme ayant une activité perhydrolase formant de là au moins un preroxyacide pour fournir au moins un premier agent antimicrobien; et
vi) un agent de modification de la rhéologie;
b) d'application de ladite composition audit lieu; et
c) laisser ladite composition sécher formant de là un revêtement sur ledit lieu;
dans lequel lesdits composants comprennent en outre un second agent antimicrobien, dans lequel ledit second agent antimicrobien comprend un composé d'ammonium quaternaire.

2. Procédé selon la revendication 1, dans lequel ledit catalyseur de type enzyme est sélectionné parmi les carbohydrate estérases de la famille CE-7.

3. Procédé selon la revendication 1, dans lequel ledit peroxyacide est produit à une concentration d'au moins 20 ppm durant environ 5 minutes jusqu'à environ 2 heures après la combinaison desdits composants.

4. Procédé de fourniture du contrôle des micro-organismes au niveau d'un lieu comprenant les étapes:
(a) de fourniture d'un premier composant prémélangé comprenant un solvant inerte et un agent formant film dans lequel l'agent est le poly(alcool de vinyle), le copolymère de poly(alcool de vinyle) ou la polyvinyl pyrrolidone;
(b) de fourniture d'un second composant prémélangé comprenant un catalyseur de type enzyme ayant une activité perhydrolase, un substrat ester d'acide carboxylique et une source de peroxygène;
(c) de mélange dudit premier composant prémélangé et dudit second composant prémélangé pour obtenir une composition de revêtement liquide comprenant un premier agent antimicrobien comprenant un peroxyacide;
(d) d'application de ladite composition de revêtement au lieu; et
(e) laisser ladite composition de revêtement sécher formant de là un revêtement sur ledit lieu;
dans lequel au moins un composant prémélangé comprend en outre un second agent antimicrobien dans lequel ledit second agent antimicrobien comprend un composé d'ammonium quaternaire.

5. Procédé selon la revendication 4, dans lequel lesdits premier et second composants prémélangés sont proposés dans un système à compartiments multiples; dans lequel le premier et le second composants prémélangés restent séparés avant l'étape (c).

6. Procédé selon la revendication 4, dans lequel un composant prémélangé se présente sous une forme liquide et un composant prémélangé se présente sous une forme solide.

7. Procédé selon la revendication 4, dans lequel ledit catalyseur de type enzyme comprend des carbohydrate estérases de la famille CE-7.

8. Composition antimicrobienne comprenant des composants qui comprennent:
a) un agent soluble dans l'eau ou dispersible dans l'eau formant film dans lequel l'agent est le poly(alcool de vinyle), le copolymère de poly(alcool de vinyle) ou la polyvinyl pyrrolidone;
b) un solvant inerte;
c) un substrat ester d'acide carboxylique;
d) une source de peroxygène;
e) un catalyseur de type enzyme ayant une activité perhydrolase; et
f) un agent de modification de la rhéologie;
dans lequel, à l'issue de la combinaison desdits composants, un peroxyacide est formé;
dans laquelle lesdits composants comprennent en outre un composant qui est un agent antimicrobien comprenant un composé d'ammonium quaternaire.

9. Composition selon la revendication 8, dans laquelle au moins un ou plusieurs desdits composants est(sont) conditionné(s) séparément vis-à-vis des autres composants dans un système à compartiments multiples avant leur combinaison pour former ledit peroxyacide.

10. Composition selon la revendication 8, dans laquelle ledit peroxyacide est produit à une concentration d'au moins 20 ppm dans les environ 5 minutes à environ 2 heures après la combinaison desdits composants.

11. Article comprenant un revêtement sur au moins une surface de celui-ci avec une composition antimicrobienne, dans lequel la composition antimicrobienne comprend:
a) un agent soluble dans l'eau ou dispersible dans l'eau formant film dans lequel l'agent est le poly(alcool de vinyle), le copolymère de poly(alcool de vinyle) ou la polyvinyl pyrrolidone;
b) un solvant inerte;
c) un substrat ester d'acide carboxylique;
d) une source de peroxygène;
e) un catalyseur de type enzyme ayant une activité perhydrolase;
f) un agent de modification de la rhéologie; et
g) un composé d'ammonium quaternaire
dans lequel, à l'issue de la combinaison des composants de (a) jusqu'à (g), un peroxyacide est formé.

12. Article selon la revendication 11, dans lequel l'article est un équipement utilisé dans l'industrie alimentaire ou des boissons.


### SEQUENCE LISTING

<110> DuPont company stauffer, christine
<120> enzymatic in-situ preparation of peracid-based removable antimicrobial coating
<130> CL4706
<140> CL4706
   <141> 2009-06-29
<160> 30
<170> PatentIn version 3.5
<210> 1
   <211> 318
   <212> PRT
   <213> Bacillus subtilis 31954
<400> 1
   Met Gln Leu Phe Asp Leu Pro Leu Asp Gln Leu Gln Thr Tyr Lys Pro
   1               5                   10                  15
   Glu Lys Thr Ala Pro Lys Asp Phe Ser Glu Phe Trp Lys Leu Ser Leu
               20                  25                  30
   Glu Glu Leu Ala Lys Val Gln Ala Glu Pro Asp Leu Gln Pro Val Asp
           35                  40                  45
   Tyr Pro Ala Asp Gly Val Lys Val Tyr Arg Leu Thr Tyr Lys Ser Phe
       50                  55                  60
   Gly Asn Ala Arg Ile Thr Gly Trp Tyr Ala Val Pro Asp Lys Gln Gly
   65                  70                  75                  80
   Pro His Pro Ala Ile Val Lys Tyr His Gly Tyr Asn Ala Ser Tyr Asp
                   85                  90                  95
   Gly Glu Ile His Glu Met Val Asn Trp Ala Leu His Gly Tyr Ala Ala
               100                 105                 110
   Phe Gly Met Leu Val Arg Gly Gln Gln Ser Ser Glu Asp Thr Ser Ile
           115                 120                 125
   Ser Leu His Gly His Ala Leu Gly Trp Met Thr Lys Gly Ile Leu Asp
       130                 135                 140
   Lys Asp Thr Tyr Tyr Tyr Arg Gly Val Tyr Leu Asp Ala Val Arg Ala
   145                 150                 155                 160
   Leu Glu Val Ile Ser Ser Phe Asp Glu Val Asp Glu Thr Arg Ile Gly
   165 170 175
   Val Thr Gly Gly Ser Gln Gly Gly Gly Leu Thr Ile Ala Ala Ala Ala
   180 185 190
   Leu Ser Asp Ile Pro Lys Ala Ala Val Ala Asp Tyr Pro Tyr Leu Ser
   195 200 205
   Asn Phe Glu Arg Ala Ile Asp Val Ala Leu Glu Gln Pro Tyr Leu Glu
   210 215 220
   Ile Asn Ser Phe Phe Arg Arg Asn Gly Ser Pro Glu Thr Glu Val Gln
   225 230 235 240
   Ala Met Lys Thr Leu Ser Tyr Phe Asp Ile Met Asn Leu Ala Asp Arg
   245 250 255
   Val Lys Val Pro Val Leu Met Ser Ile Gly Leu Ile Asp Lys Val Thr
   260 265 270
   Pro Pro Ser Thr Val Phe Ala Ala Tyr Asn His Leu Glu Thr Glu Lys
   275 280 285
   Glu Leu Lys Val Tyr Arg Tyr Phe Gly His Glu Tyr Ile Pro Ala Phe
   290 295 300
   Gln Thr Glu Lys Leu Ala Phe Phe Lys Gln His Leu Lys Gly
   305 310 315
<210> 2
   <211> 318
   <212> PRT
   <213> Bacillus subtilis subsp. subtilis strain 168
<400> 2
   Met Gln Leu Phe Asp Leu Pro Leu Asp Gln Leu Gln Thr Tyr Lys Pro
   1 5 10 15
   Glu Lys Thr Ala Pro Lys Asp Phe Ser Glu Phe Trp Lys Leu Ser Leu
   20 25 30
   Glu Glu Leu Ala Lys Val Gln Ala Glu Pro Asp Leu Gln Pro Val Asp
   35 40 45
   Tyr Pro Ala Asp Gly Val Lys Val Tyr Arg Leu Thr Tyr Lys Ser Phe
   50 55 60
   Gly Asn Ala Arg Ile Thr Gly Trp Tyr Ala Val Pro Asp Lys Glu Gly
   65 70 75 80
   Pro His Pro Ala Ile Val Lys Tyr His Gly Tyr Asn Ala Ser Tyr Asp
   85 90 95
   Gly Glu Ile His Glu Met Val Asn Trp Ala Leu His Gly Tyr Ala Thr
   100 105 110
   Phe Gly Met Leu Val Arg Gly Gln Gln Ser Ser Glu Asp Thr Ser Ile
   115 120 125
   Ser Pro His Gly His Ala Leu Gly Trp Met Thr Lys Gly Ile Leu Asp
   130 135 140
   Lys Asp Thr Tyr Tyr Tyr Arg Gly Val Tyr Leu Asp Ala Val Arg Ala
   145 150 155 160
   Leu Glu Val Ile Ser Ser Phe Asp Glu Val Asp Glu Thr Arg Ile Gly
   165 170 175
   Val Thr Gly Gly Ser Gln Gly Gly Gly Leu Thr Ile Ala Ala Ala Ala
   180 185 190
   Leu Ser Asp Ile Pro Lys Ala Ala Val Ala Asp Tyr Pro Tyr Leu Ser
   195 200 205
   Asn Phe Glu Arg Ala Ile Asp Val Ala Leu Glu Gln Pro Tyr Leu Glu
   210 215 220
   Ile Asn Ser Phe Phe Arg Arg Asn Gly Ser Pro Glu Thr Glu Val Gln
   225 230 235 240
   Ala Met Lys Thr Leu Ser Tyr Phe Asp Ile Met Asn Leu Ala Asp Arg
   245 250 255
   Val Lys Val Pro Val Leu Met Ser Ile Gly Leu Ile Asp Lys Val Thr
   260 265 270
   Pro Pro Ser Thr Val Phe Ala Ala Tyr Asn His Leu Glu Thr Lys Lys
   275 280 285
   Glu Leu Lys Val Tyr Arg Tyr Phe Gly His Glu Tyr Ile Pro Ala Phe
   290 295 300
   Gln Thr Glu Lys Leu Ala Phe Phe Lys Gln His Leu Lys Gly
   305 310 315
<210> 3
   <211> 318
   <212> PRT
   <213> Bacillus subtilis ATCC 6633
<400> 3
   Met Gln Leu Phe Asp Leu Pro Leu Asp Gln Leu Gln Thr Tyr Lys Pro
   1               5                   10                  15
   Glu Lys Thr Thr Pro Asn Asp Phe Ser Glu Phe Trp Lys Ser Ser Leu
               20                  25                  30
   Asp Glu Leu Ala Lys Val Lys Ala Ala Pro Asp Leu Gln Leu Val Asp
           35                  40                  45
   Tyr Pro Ala Asp Gly Val Lys Val Tyr Arg Leu Thr Tyr Lys Ser Phe
       50                  55                  60
   Gly Asn Ala Arg Ile Thr Gly Trp Tyr Ala Val Pro Asp Lys Glu Gly
   65                  70                  75                  80
   Pro His Pro Ala Ile Val Lys Tyr His Gly Tyr Asn Ala Ser Tyr Asp
                   85                  90                  95
   Gly Glu Ile His Glu Met Val Asn Trp Ala Leu His Gly Tyr Ala Ala
               100                 105                 110
   Phe Gly Met Leu Val Arg Gly Gln Gln Ser Ser Glu Asp Thr Ser Ile
           115                 120                 125
   Ser Pro His Gly His Ala Leu Gly Trp Met Thr Lys Gly Ile Leu Asp
       130                 135                 140
   Lys Asp Thr Tyr Tyr Tyr Arg Gly Val Tyr Leu Asp Ala Val Arg Ala
   145                 150                 155                 160
   Leu Glu Val Ile Ser Ser Phe Asp Glu Val Asp Glu Thr Arg Ile Gly
                   165                 170                 175
   Val Thr Gly Gly Ser Gln Gly Gly Gly Leu Thr Ile Ala Ala Ala Ala
               180                 185                 190
   Leu Ser Asp Ile Pro Lys Ala Ala Val Ala Asp Tyr Pro Tyr Leu Ser
           195                 200                 205
   Asn Phe Glu Arg Ala Ile Asp Val Ala Leu Glu Gln Pro Tyr Leu Glu
       210                 215                 220
   Ile Asn Ser Phe Phe Arg Arg Asn Gly Ser Pro Glu Thr Glu Glu Lys
   225                 230                 235                 240
   Ala Met Lys Thr Leu Ser Tyr Phe Asp Ile Met Asn Leu Ala Asp Arg
                   245                 250                 255
   Val Lys Val Pro Val Leu Met Ser Ile Gly Leu Ile Asp Lys Val Thr
   260 265 270
   Pro Pro Ser Thr Val Phe Ala Ala Tyr Asn His Leu Glu Thr Glu Lys
   275 280 285
   Glu Leu Lys Val Tyr Arg Tyr Phe Gly His Glu Tyr Ile Pro Ala Phe
   290 295 300
   Gln Thr Glu Lys Leu Ala Phe Phe Lys Gln His Leu Lys Gly
   305 310 315
<210> 4
   <211> 318
   <212> PRT
   <213> Bacillus licheniformis ATCC 14580
<400> 4
   Met Gln Gln Pro Tyr Asp Met Pro Leu Glu Gln Leu Tyr Gln Tyr Lys
   1 5 10 15
   Pro Glu Arg Thr Ala Pro Ala Asp Phe Lys Glu Phe Trp Lys Gly Ser
   20 25 30
   Leu Glu Glu Leu Ala Asn Glu Lys Ala Gly Pro Gln Leu Glu Pro His
   35 40 45
   Glu Tyr Pro Ala Asp Gly Val Lys Val Tyr Trp Leu Thr Tyr Arg Ser
   50 55 60
   Ile Gly Gly Ala Arg Ile Lys Gly Trp Tyr Ala Val Pro Asp Arg Gln
   65 70 75 80
   Gly Pro His Pro Ala Ile Val Lys Tyr His Gly Tyr Asn Ala Ser Tyr
   85 90 95
   Asp Gly Asp Ile His Asp Ile Val Asn Trp Ala Leu His Gly Tyr Ala
   100 105 110
   Ala Phe Gly Met Leu Val Arg Gly Gln Asn Ser Ser Glu Asp Thr Glu
   115 120 125
   Ile Ser His His Gly His Val Pro Gly Trp Met Thr Lys Gly Ile Leu
   130 135 140
   Asp Pro Lys Thr Tyr Tyr Tyr Arg Gly Val Tyr Leu Asp Ala Val Arg
   145 150 155 160
   Ala Val Glu Val Val Ser Gly Phe Ala Glu Val Asp Glu Lys Arg Ile
   165 170 175
   Gly Val Ile Gly Ala Ser Gln Gly Gly Gly Leu Ala Val Ala Val Ser
   180 185 190
   Ala Leu Ser Asp Ile Pro Lys Ala Ala Val Ser Glu Tyr Pro Tyr Leu
   195 200 205
   Ser Asn Phe Gln Arg Ala Ile Asp Thr Ala Ile Asp Gln Pro Tyr Leu
   210 215 220
   Glu Ile Asn Ser Phe Phe Arg Arg Asn Thr Ser Pro Asp Ile Glu Gln
   225 230 235 240
   Ala Ala Met His Thr Leu Ser Tyr Phe Asp Val Met Asn Leu Ala Gln
   245 250 255
   Leu Val Lys Ala Thr Val Leu Met Ser Ile Gly Leu Val Asp Thr Ile
   260 265 270
   Thr Pro Pro Ser Thr Val Phe Ala Ala Tyr Asn His Leu Glu Thr Asp
   275 280 285
   Lys Glu Ile Lys Val Tyr Arg Tyr Phe Gly His Glu Tyr Ile Pro Pro
   290 295 300
   Phe Gln Thr Glu Lys Leu Ala Phe Leu Arg Lys His Leu Lys
   305 310 315
<210> 5
   <211> 320
   <212> PRT
   <213> Bacillus pumilis
<400> 5
   Met Gln Leu Phe Asp Leu Ser Leu Glu Glu Leu Lys Lys Tyr Lys Pro
   1 5 10 15
   Lys Lys Thr Ala Arg Pro Asp Phe Ser Asp Phe Trp Lys Lys Ser Leu
   20 25 30
   Glu Glu Leu Arg Gln Val Glu Ala Glu Pro Thr Leu Glu Ser Tyr Asp
   35 40 45
   Tyr Pro Val Lys Gly Val Lys Val Tyr Arg Leu Thr Tyr Gln Ser Phe
   50 55 60
   Gly His Ser Lys Ile Glu Gly Phe Tyr Ala Val Pro Asp Gln Thr Gly
   65 70 75 80
   Pro His Pro Ala Leu Val Arg Phe His Gly Tyr Asn Ala Ser Tyr Asp
   85 90 95
   Gly Gly Ile His Asp Ile Val Asn Trp Ala Leu His Gly Tyr Ala Thr
   100 105 110
   Phe Gly Met Leu Val Arg Gly Gln Gly Gly Ser Glu Asp Thr Ser Val
   115 120 125
   Thr Pro Gly Gly His Ala Leu Gly Trp Met Thr Lys Gly Ile Leu Ser
   130 135 140
   Lys Asp Thr Tyr Tyr Tyr Arg Gly Val Tyr Leu Asp Ala Val Arg Ala
   145 150 155 160
   Leu Glu Val Ile Gln Ser Phe Pro Glu Val Asp Glu His Arg Ile Gly
   165 170 175
   Val Ile Gly Gly Ser Gln Gly Gly Ala Leu Ala Ile Ala Ala Ala Ala
   180 185 190
   Leu Ser Asp Ile Pro Lys Val Val Val Ala Asp Tyr Pro Tyr Leu Ser
   195 200 205
   Asn Phe Glu Arg Ala Val Asp Val Ala Leu Glu Gln Pro Tyr Leu Glu
   210 215 220
   Ile Asn Ser Tyr Phe Arg Arg Asn Ser Asp Pro Lys Val Glu Glu Lys
   225 230 235 240
   Ala Phe Glu Thr Leu Ser Tyr Phe Asp Leu Ile Asn Leu Ala Gly Trp
   245 250 255
   Val Lys Gln Pro Thr Leu Met Ala Ile Gly Leu Ile Asp Lys Ile Thr
   260 265 270
   Pro Pro Ser Thr Val Phe Ala Ala Tyr Asn His Leu Glu Thr Asp Lys
   275 280 285
   Asp Leu Lys Val Tyr Arg Tyr Phe Gly His Glu Phe Ile Pro Ala Phe
   290 295 300
   Gln Thr Glu Lys Leu Ser Phe Leu Gln Lys His Leu Leu Leu Ser Thr
   305 310 315 320
<210> 6
   <211> 320
   <212> PRT
   <213> Clostridium thermocellum ATCC 27405
<400> 6
   Met Ala Gln Leu Tyr Asp Met Pro Leu Glu Glu Leu Lys Lys Tyr Lys
   1               5                   10                  15
   Pro Ala Leu Thr Lys Gln Lys Asp Phe Asp Glu Phe Trp Glu Lys Ser
               20                  25                  30
   Leu Lys Glu Leu Ala Glu Ile Pro Leu Lys Tyr Gln Leu Ile Pro Tyr
           35                  40                  45
   Asp Phe Pro Ala Arg Arg Val Lys Val Phe Arg Val Glu Tyr Leu Gly
       50                  55                  60
   Phe Lys Gly Ala Asn Ile Glu Gly Trp Leu Ala Val Pro Glu Gly Glu
   65                  70                  75                  80
   Gly Leu Tyr Pro Gly Leu Val Gln Phe His Gly Tyr Asn Trp Ala Met
                   85                  90                  95
   Asp Gly Cys Val Pro Asp Val Val Asn Trp Ala Leu Asn Gly Tyr Ala
               100                 105                 110
   Ala Phe Leu Met Leu Val Arg Gly Gln Gln Gly Arg Ser Val Asp Asn
           115                 120                 125
   Ile Val Pro Gly Ser Gly His Ala Leu Gly Trp Met Ser Lys Gly Ile
       130                 135                 140
   Leu Ser Pro Glu Glu Tyr Tyr Tyr Arg Gly Val Tyr Met Asp Ala Val
   145                 150                 155                 160
   Arg Ala Val Glu Ile Leu Ala Ser Leu Pro Cys Val Asp Glu Ser Arg
                   165                 170                 175
   Ile Gly Val Thr Gly Gly Ser Gln Gly Gly Gly Leu Ala Leu Ala Val
               180                 185                 190
   Ala Ala Leu Ser Gly Ile Pro Lys Val Ala Ala Val His Tyr Pro Phe
           195                 200                 205
   Leu Ala His Phe Glu Arg Ala Ile Asp Val Ala Pro Asp Gly Pro Tyr
       210                 215                 220
   Leu Glu Ile Asn Glu Tyr Leu Arg Arg Asn Ser Gly Glu Glu Ile Glu
   225                 230                 235                 240
   Arg Gln Val Lys Lys Thr Leu Ser Tyr Phe Asp Ile Met Asn Leu Ala
   245 250 255
   Pro Arg Ile Lys Cys Arg Thr Trp Ile Cys Thr Gly Leu Val Asp Glu
   260 265 270
   Ile Thr Pro Pro Ser Thr Val Phe Ala Val Tyr Asn His Leu Lys Cys
   275 280 285
   Pro Lys Glu Ile Ser Val Phe Arg Tyr Phe Gly His Glu His Met Pro
   290 295 300
   Gly Ser Val Glu Ile Lys Leu Arg Ile Leu Met Asp Glu Leu Asn Pro
   305 310 315 320
<210> 7
   <211> 325
   <212> PRT
   <213> Thermotoga neapolitana
<400> 7
   Met Ala Phe Phe Asp Met Pro Leu Glu Glu Leu Lys Lys Tyr Arg Pro
   1 5 10 15
   Glu Arg Tyr Glu Glu Lys Asp Phe Asp Glu Phe Trp Arg Glu Thr Leu
   20 25 30
   Lys Glu Ser Glu Gly Phe Pro Leu Asp Pro Val Phe Glu Lys Val Asp
   35 40 45
   Phe His Leu Lys Thr Val Glu Thr Tyr Asp Val Thr Phe Ser Gly Tyr
   50 55 60
   Arg Gly Gln Arg Ile Lys Gly Trp Leu Leu Val Pro Lys Leu Ala Glu
   65 70 75 80
   Glu Lys Leu Pro Cys Val Val Gln Tyr Ile Gly Tyr Asn Gly Gly Arg
   85 90 95
   Gly Phe Pro His Asp Trp Leu Phe Trp Pro Ser Met Gly Tyr Ile Cys
   100 105 110
   Phe Val Met Asp Thr Arg Gly Gln Gly Ser Gly Trp Met Lys Gly Asp
   115 120 125
   Thr Pro Asp Tyr Pro Glu Gly Pro Val Asp Pro Gln Tyr Pro Gly Phe
   130 135 140
   Met Thr Arg Gly Ile Leu Asp Pro Gly Thr Tyr Tyr Tyr Arg Arg Val
   145 150 155 160
   Phe Val Asp Ala Val Arg Ala Val Glu Ala Ala Ile Ser Phe Pro Arg
   165 170 175
   Val Asp Ser Arg Lys Val Val Val Ala Gly Gly Ser Gln Gly Gly Gly
   180 185 190
   Ile Ala Leu Ala Val Ser Ala Leu Ser Asn Arg Val Lys Ala Leu Leu
   195 200 205
   Cys Asp Val Pro Phe Leu Cys His Phe Arg Arg Ala Val Gln Leu Val
   210 215 220
   Asp Thr His Pro Tyr Val Glu Ile Thr Asn Phe Leu Lys Thr His Arg
   225 230 235 240
   Asp Lys Glu Glu Ile Val Phe Arg Thr Leu Ser Tyr Phe Asp Gly Val
   245 250 255
   Asn Phe Ala Ala Arg Ala Lys Val Pro Ala Leu Phe Ser Val Gly Leu
   260 265 270
   Met Asp Thr Ile Cys Pro Pro Ser Thr Val Phe Ala Ala Tyr Asn His
   275 280 285
   Tyr Ala Gly Pro Lys Glu Ile Arg Ile Tyr Pro Tyr Asn Asn His Glu
   290 295 300
   Gly Gly Gly Ser Phe Gln Ala Ile Glu Gln Val Lys Phe Leu Lys Arg
   305 310 315 320
   Leu Phe Glu Glu Gly
   325
<210> 8
   <211> 325
   <212> PRT
   <213> Thermotoga maritima MSB8
<400> 8
   Met Ala Phe Phe Asp Leu Pro Leu Glu Glu Leu Lys Lys Tyr Arg Pro
   1 5 10 15
   Glu Arg Tyr Glu Glu Lys Asp Phe Asp Glu Phe Trp Glu Glu Thr Leu
   20 25 30
   Ala Glu Ser Glu Lys Phe Pro Leu Asp Pro Val Phe Glu Arg Met Glu
   35 40 45
   Ser His Leu Lys Thr Val Glu Ala Tyr Asp Val Thr Phe Ser Gly Tyr
   50 55 60
   Arg Gly Gln Arg Ile Lys Gly Trp Leu Leu Val Pro Lys Leu Glu Glu
   65 70 75 80
   Glu Lys Leu Pro Cys Val Val Gln Tyr Ile Gly Tyr Asn Gly Gly Arg
   85 90 95
   Gly Phe Pro His Asp Trp Leu Phe Trp Pro Ser Met Gly Tyr Ile Cys
   100 105 110
   Phe Val Met Asp Thr Arg Gly Gln Gly Ser Gly Trp Leu Lys Gly Asp
   115 120 125
   Thr Pro Asp Tyr Pro Glu Gly Pro Val Asp Pro Gln Tyr Pro Gly Phe
   130 135 140
   Met Thr Arg Gly Ile Leu Asp Pro Arg Thr Tyr Tyr Tyr Arg Arg Val
   145 150 155 160
   Phe Thr Asp Ala Val Arg Ala Val Glu Ala Ala Ala Ser Phe Pro Gln
   165 170 175
   Val Asp Gln Glu Arg Ile Val Ile Ala Gly Gly Ser Gln Gly Gly Gly
   180 185 190
   Ile Ala Leu Ala Val Ser Ala Leu Ser Lys Lys Ala Lys Ala Leu Leu
   195 200 205
   Cys Asp Val Pro Phe Leu Cys His Phe Arg Arg Ala Val Gln Leu Val
   210 215 220
   Asp Thr His Pro Tyr Ala Glu Ile Thr Asn Phe Leu Lys Thr His Arg
   225 230 235 240
   Asp Lys Glu Glu Ile Val Phe Arg Thr Leu Ser Tyr Phe Asp Gly Val
   245 250 255
   Asn Phe Ala Ala Arg Ala Lys Ile Pro Ala Leu Phe Ser Val Gly Leu
   260 265 270
   Met Asp Asn Ile Cys Pro Pro Ser Thr Val Phe Ala Ala Tyr Asn Tyr
   275 280 285
   Tyr Ala Gly Pro Lys Glu Ile Arg Ile Tyr Pro Tyr Asn Asn His Glu
   290 295 300
   Gly Gly Gly Ser Phe Gln Ala Val Glu Gln Val Lys Phe Leu Lys Lys
   305 310 315 320
   Leu Phe Glu Lys Gly
   325
<210> 9
   <211> 320
   <212> PRT
   <213> Thermoanaerobacterium sp.
<210> 10
   <211> 340
   <212> PRT
   <213> Bacillus sp. NRRL B-14911
<400> 10
   Met Arg Thr Val Pro Ala Pro Val Phe Leu Glu Arg Ser Gly Glu Met
   1 5 10 15
   Asn Leu Phe Asp Met Pro Leu Glu Glu Leu Gln His Tyr Lys Pro Ala
   20 25 30
   Gln Thr Arg Gln Asp Asp Phe Glu Ser Phe Trp Lys Lys Arg Ile Glu
   35 40 45
   Glu Asn Ser Gln Tyr Pro Leu Asn Ile Glu Val Met Glu Arg Val Tyr
   50 55 60
   Pro Val Pro Gly Val Arg Val Tyr Asp Ile Tyr Phe Asp Gly Phe Arg
   65 70 75 80
   Asn Ser Arg Ile His Gly Val Tyr Val Thr Pro Glu Thr Pro Gly Ala
   85 90 95
   Asp Thr Pro Ala Ala Val Ile Phe His Gly Tyr Asn Trp Asn Thr Leu
   100 105 110
   Gln Pro His Tyr Ser Phe Lys His Val Ile Gln Gly Ile Pro Val Leu
   115 120 125
   Met Val Glu Val Arg Gly Gln Asn Leu Leu Ser Pro Asp Arg Asn His
   130 135 140
   Tyr Gly Asn Gly Gly Pro Gly Gly Trp Met Thr Leu Gly Val Met Asp
   145 150 155 160
   Pro Asp Gln Tyr Tyr Tyr Ser Leu Val Tyr Met Asp Cys Phe Arg Ser
   165 170 175
   Ile Asp Ala Val Arg Glu Leu Ser Arg Lys Arg Ser Val Phe Val Glu
   180 185 190
   Gly Gly Ser Gln Gly Gly Ala Leu Ala Ile Ala Ala Ala Ala Leu Gln
   195 200 205
   Asp Asp Ile Leu Leu Ala Leu Ala Asp Ile Pro Phe Leu Thr His Phe
   210 215 220
   Lys Arg Ser Val Glu Leu Ser Ser Asp Gly Pro Tyr Gln Glu Ile Ser
   225 230 235 240
   His Tyr Phe Lys Val His Asp Pro Leu His Gln Thr Glu Glu Gln Val
   245 250 255
   Tyr Gln Thr Leu Ser Tyr Val Asp Cys Met Asn Met Ala Ser Met Val
   260 265 270
   Glu Cys Pro Val Leu Leu Ser Ala Gly Leu Glu Asp Ile Val Cys Pro
   275 280 285
   Pro Ser Ser Ala Phe Ala Leu Phe Asn His Leu Gly Gly Pro Lys Glu
   290 295 300
   Ile Arg Ala Tyr Pro Glu Tyr Ala His Glu Val Pro Ala Val His Glu
   305 310 315 320
   Glu Glu Lys Leu Lys Phe Ile Ser Ser Arg Leu Lys Asn Arg Glu Lys
   325 330 335
   Arg Cys Arg Pro
   340
<210> 11
   <211> 319
   <212> PRT
   <213> Bacillus halodurans C-125
<400> 11
   Met Pro Leu Ile Asp Met Pro Leu Thr Glu Leu Lys Glu Tyr Met Gly
   1 5 10 15
   Arg Asn Pro Lys Pro Asp Asp Phe Thr Glu Tyr Trp Asp Arg Ala Leu
   20 25 30
   Gln Glu Met Arg Lys Val Asn Pro Asn Val Glu Leu Ile Pro Ser Asp
   35 40 45
   Phe Gln Thr Thr Tyr Ala Glu Cys Phe His Leu Tyr Phe Thr Gly Val
   50 55 60
   Arg Gly Ala Arg Ile His Ala Lys Tyr Val Arg Pro Arg His Thr Ser
   65 70 75 80
   Gly Thr His Pro Ala Val Ile His Phe His Gly Tyr Thr Met Asn Ala
   85 90 95
   Gly Glu Trp Thr Gly Leu Leu His Tyr Ala Ala Leu Gly Tyr Ser Val
   100 105 110
   Leu Ala Met Asp Val Arg Gly Gln Gly Gly Leu Ser Glu Asp Thr Gly
   115 120 125
   Gly Val Lys Gly Asn Thr His Ser Gly His Ile Ile Arg Gly Leu Asp
   130 135 140
   Asp Asn Ala Asp Gln Leu Leu Phe Arg His Val Phe Leu Asp Thr Ala
   145 150 155 160
   Gln Leu Ala Asn Ile Val Met Asn Leu Pro Glu Val Asp Glu Glu Arg
   165 170 175
   Val Ala Val Thr Gly Trp Ser Gln Gly Gly Ala Leu Ala Ile Ala Cys
   180 185 190
   Ala Ala Leu Glu Pro Lys Ile Lys Lys Val Ala Pro Val Tyr Pro Phe
   195 200 205
   Leu Ser Asp Tyr Gln Arg Val Trp Glu Met Asp Leu Ala Glu Lys Ala
   210 215 220
   Tyr Asp Glu Leu Gln Thr Tyr Phe Arg Arg Phe Asp Pro Gln His Arg
   225 230 235 240
   Arg Glu Ala Glu Ile Phe Thr Lys Leu Gly Tyr Ile Asp Ile Gln His
   245 250 255
   Leu Ala Pro Leu Val Lys Gly Glu Val Leu Leu Ala Val Gly Leu Met
   260 265 270
   Asp Thr Val Cys Pro Pro Ser Thr Gln Phe Ala Met Tyr Asn Lys Leu
   275 280 285
   Thr Thr Thr Lys Ser Ile Glu Leu Tyr Pro Asp Phe Ala His Glu Asp
   290 295 300
   Leu Pro Gly His Arg Asp Arg Ile Phe Gln Phe Leu Ser Asp Leu
   305 310 315
<210> 12
   <211> 317
   <212> PRT
   <213> Bacillus clausii KSM-K16
<400> 12
   Met Pro Leu Val Asp Met Pro Leu Arg Glu Leu Leu Ala Tyr Glu Gly
   1 5 10 15
   Ile Asn Pro Lys Pro Ala Asp Phe Asp Gln Tyr Trp Asn Arg Ala Lys
   20 25 30
   Thr Glu Ile Glu Ala Ile Asp Pro Glu Val Thr Leu Val Glu Ser Ser
   35 40 45
   Phe Gln Cys Ser Phe Ala Asn Cys Tyr His Phe Tyr Tyr Arg Ser Ala
   50 55 60
   Gly Asn Ala Lys Ile His Ala Lys Tyr Val Gln Pro Lys Ala Gly Glu
   65 70 75 80
   Lys Thr Pro Ala Val Phe Met Phe His Gly Tyr Gly Gly Arg Ser Ala
   85 90 95
   Glu Trp Ser Ser Leu Leu Asn Tyr Val Ala Ala Gly Phe Ser Val Phe
   100 105 110
   Tyr Met Asp Val Arg Gly Gln Gly Gly Thr Ser Glu Asp Pro Gly Gly
   115 120 125
   Val Arg Gly Asn Thr Tyr Arg Gly His Ile Ile Arg Gly Leu Asp Ala
   130 135 140
   Gly Pro Asp Ala Leu Phe Tyr Arg Ser Val Phe Leu Asp Thr Val Gln
   145 150 155 160
   Leu Val Arg Ala Ala Lys Thr Leu Pro His Ile Asp Lys Thr Arg Leu
   165 170 175
   Met Ala Thr Gly Trp Ser Gln Gly Gly Ala Leu Thr Leu Ala Cys Ala
   180 185 190
   Ala Leu Val Pro Glu Ile Lys Arg Leu Ala Pro Val Tyr Pro Phe Leu
   195 200 205
   Ser Asp Tyr Lys Arg Val Trp Gln Met Asp Leu Ala Val Arg Ser Tyr
   210 215 220
   Lys Glu Leu Ala Asp Tyr Phe Arg Ser Tyr Asp Pro Gln His Lys Arg
   225 230 235 240
   His Gly Glu Ile Phe Glu Arg Leu Gly Tyr Ile Asp Val Gln His Leu
   245 250 255
   Ala Asp Arg Ile Gln Gly Asp Val Leu Met Gly Val Gly Leu Met Asp
   260 265 270
   Thr Glu Cys Pro Pro Ser Thr Gln Phe Ala Ala Tyr Asn Lys Ile Lys
   275 280 285
   Ala Lys Lys Ser Tyr Glu Leu Tyr Pro Asp Phe Gly His Glu His Leu
   290 295 300
   Pro Gly Met Asn Asp His Ile Phe Arg Phe Phe Thr Ser
   305 310 315
<210> 13
   <211> 320
   <212> PRT
   <213> Thermoanaerobacterium saccharolyticum
<400> 13
   Met Gly Leu Phe Asp Met Pro Leu Gln Lys Leu Arg Glu Tyr Thr Gly
   1 5 10 15
   Thr Asn Pro Cys Pro Glu Asp Phe Asp Glu Tyr Trp Asp Arg Ala Leu
   20 25 30
   Asp Glu Met Arg Ser Val Asp Pro Lys Ile Lys Met Lys Lys Ser Ser
   35 40 45
   Phe Gln Val Pro Phe Ala Glu Cys Tyr Asp Leu Tyr Phe Thr Gly Val
   50 55 60
   Arg Gly Ala Arg Ile His Ala Lys Tyr Ile Arg Pro Lys Thr Glu Gly
   65 70 75 80
   Lys His Pro Ala Leu Ile Arg Phe His Gly Tyr Ser Ser Asn Ser Gly
   85 90 95
   Asp Trp Asn Asp Lys Leu Asn Tyr Val Ala Ala Gly Phe Thr Val Val
   100 105 110
   Ala Met Asp Ala Arg Gly Gln Gly Gly Gln Ser Gln Asp Val Gly Gly
   115 120 125
   Val Asn Gly Asn Thr Leu Asn Gly His Ile Ile Arg Gly Leu Asp Asp
   130 135 140
   Asp Ala Asp Asn Met Leu Phe Arg His Ile Phe Leu Asp Thr Ala Gln
   145 150 155 160
   Leu Ala Gly Ile Val Met Asn Met Pro Glu Ile Asp Glu Asp Arg Val
   165 170 175
   Ala Val Met Gly Pro Ser Gln Gly Gly Gly Leu Ser Leu Ala Cys Ala
   180 185 190
   Ala Leu Glu Pro Lys Ile Arg Lys Val Val Ser Glu Tyr Pro Phe Leu
   195 200 205
   Ser Asp Tyr Lys Arg Val Trp Asp Leu Asp Leu Ala Lys Asn Ala Tyr
   210 215 220
   Gln Glu Ile Thr Asp Tyr Phe Arg Leu Phe Asp Pro Arg His Glu Arg
   225 230 235 240
   Glu Asn Glu Val Phe Thr Lys Leu Gly Tyr Ile Asp Val Lys Asn Leu
   245 250 255
   Ala Lys Arg Ile Lys Gly Asp Val Leu Met Cys Val Gly Leu Met Asp
   260 265 270
   Gln Val Cys Pro Pro Ser Thr Val Phe Ala Ala Tyr Asn Asn Ile Gln
   275 280 285
   Ser Lys Lys Asp Ile Lys Val Tyr Pro Asp Tyr Gly His Glu Pro Met
   290 295 300
   Arg Gly Phe Gly Asp Leu Ala Met Gln Phe Met Leu Glu Leu Tyr Ser
   305 310 315 320
<210> 14
   <211> 326
   <212> PRT
   <213> Thermotoga lettingae
<400> 14
   Met Val Tyr Phe Asp Met Pro Leu Glu Asp Leu Arg Lys Tyr Leu Pro
   1               5                   10                  15
   Gln Arg Tyr Glu Glu Lys Asp Phe Asp Asp Phe Trp Lys Gln Thr Ile
               20                  25                  30
   His Glu Thr Arg Gly Tyr Phe Gln Glu Pro Ile Leu Lys Lys Val Asp
           35                  40                  45
   Phe Tyr Leu Gln Asn Val Glu Thr Phe Asp Val Thr Phe Ser Gly Tyr
       50                  55                  60
   Arg Gly Gln Lys Ile Lys Gly Trp Leu Ile Leu Pro Lys Phe Arg Asn
   65                  70                  75                  80
   Gly Lys Leu Pro Cys Val Val Glu Phe Val Gly Tyr Gly Gly Gly Arg
                   85                  90                  95
   Gly Phe Pro Tyr Asp Trp Leu Leu Trp Ser Ala Ala Gly Tyr Ala His
               100                 105                 110
   Phe Ile Met Asp Thr Arg Gly Gln Gly Ser Asn Trp Met Lys Gly Asp
           115                 120                 125
   Thr Pro Asp Tyr Glu Asp Asn Pro Ser Asp Pro Gln Tyr Pro Gly Phe
       130                 135                 140
   Leu Thr Lys Gly Val Leu Asn Pro Glu Thr Tyr Tyr Tyr Arg Arg Val
   145                 150                 155                 160
   Phe Met Asp Ala Phe Met Ala Val Glu Thr Ile Ser Gln Leu Glu Gln
                   165                 170                 175
   Ile Asp Ser Gln Thr Ile Ile Leu Ser Gly Ala Ser Gln Gly Gly Gly
               180                 185                 190
   Ile Ala Leu Ala Val Ser Ala Leu Ser Ser Lys Val Met Ala Leu Leu
           195                 200                 205
   Cys Asp Val Pro Phe Leu Cys His Tyr Lys Arg Ala Val Gln Ile Thr
       210                 215                 220
   Asp Ser Met Pro Tyr Ala Glu Ile Thr Arg Tyr Cys Lys Thr His Ile
   225                 230                 235                 240
   Asp Lys Ile Gln Thr Val Phe Arg Thr Leu Ser Tyr Phe Asp Gly Val
                   245                 250                 255
   Asn Phe Ala Ala Arg Ala Lys Cys Pro Ala Leu Phe Ser Val Gly Leu
   260 265 270
   Met Asp Asp Ile Cys Pro Pro Ser Thr Val Phe Ala Ala Tyr Asn Tyr
   275 280 285
   Tyr Ala Gly Glu Lys Asp Ile Arg Ile Tyr Pro Tyr Asn Asn His Glu
   290 295 300
   Gly Gly Gly Ser Phe His Thr Leu Glu Lys Leu Lys Phe Val Lys Lys
   305 310 315 320
   Thr Ile Ser Met Arg Glu
   325
<210> 15
   <211> 325
   <212> PRT
   <213> Thermotoga petrophilia
<400> 15
   Met Ala Phe Phe Asp Leu Pro Leu Glu Glu Leu Lys Lys Tyr Arg Pro
   1 5 10 15
   Glu Arg Tyr Glu Glu Lys Asp Phe Asp Glu Phe Trp Glu Gly Thr Leu
   20 25 30
   Ala Glu Asn Glu Lys Phe Pro Leu Asp Pro Val Phe Glu Arg Met Glu
   35 40 45
   Ser His Leu Lys Thr Val Glu Ala Tyr Asp Val Thr Phe Ser Gly Tyr
   50 55 60
   Met Gly Gln Arg Ile Lys Gly Trp Leu Leu Val Pro Lys Leu Glu Glu
   65 70 75 80
   Glu Lys Leu Pro Cys Val Val Gln Tyr Ile Gly Tyr Asn Gly Gly Arg
   85 90 95
   Gly Phe Pro His Asp Trp Leu Phe Trp Pro Ser Met Gly Tyr Ile Cys
   100 105 110
   Phe Val Met Asp Thr Arg Gly Gln Gly Ser Gly Trp Met Lys Gly Asp
   115 120 125
   Thr Pro Asp Tyr Pro Glu Asp Pro Val Asp Pro Gln Tyr Pro Gly Phe
   130 135 140
   Met Thr Arg Gly Ile Leu Asp Pro Arg Thr Tyr Tyr Tyr Arg Arg Val
   145 150 155 160
   Phe Thr Asp Ala Val Arg Ala Val Glu Ala Ala Ala Ser Phe Pro Arg
   165 170 175
   Val Asp His Glu Arg Ile Val Ile Ala Gly Gly Ser Gln Gly Gly Gly
   180 185 190
   Ile Ala Leu Ala Val Ser Ala Leu Ser Lys Lys Ala Lys Ala Leu Leu
   195 200 205
   Cys Asp Val Pro Phe Leu Cys His Phe Arg Arg Ala Val Gln Leu Val
   210 215 220
   Asp Thr His Pro Tyr Ala Glu Ile Thr Asn Phe Leu Lys Thr His Arg
   225 230 235 240
   Asp Lys Glu Glu Ile Val Phe Arg Thr Leu Ser Tyr Phe Asp Gly Val
   245 250 255
   Asn Phe Ala Val Arg Ala Lys Ile Pro Ala Leu Phe Ser Val Gly Leu
   260 265 270
   Met Asp Asn Ile Cys Pro Pro Ser Thr Val Phe Ala Ala Tyr Asn His
   275 280 285
   Tyr Ala Gly Pro Lys Glu Ile Arg Ile Tyr Pro Tyr Asn Asn His Glu
   290 295 300
   Gly Gly Gly Ser Phe Gln Ala Ile Glu Gln Val Lys Phe Leu Lys Arg
   305 310 315 320
   Leu Phe Glu Lys Gly
   325
<210> 16
   <211> 325
   <212> PRT
   <213> Thermotoga sp. RQ2
<400> 16
   Met Ala Phe Phe Asp Leu Pro Leu Glu Glu Leu Lys Lys Tyr Arg Pro
   1 5 10 15
   Glu Arg Tyr Glu Glu Lys Asp Phe Asp Glu Phe Trp Lys Glu Thr Leu
   20 25 30
   Ala Glu Ser Glu Lys Phe Pro Leu Asp Pro Val Phe Glu Arg Met Glu
   35 40 45
   Ser His Leu Lys Thr Val Glu Val Tyr Asp Val Thr Phe Ser Gly Tyr
       50                  55                  60
   Arg Gly Gln Arg Ile Lys Gly Trp Leu Leu Val Pro Lys Leu Glu Glu
   65                  70                  75                  80
   Glu Lys Leu Pro Cys Val Val Gln Tyr Ile Gly Tyr Asn Gly Gly Arg
                   85                  90                  95
   Gly Phe Pro His Asp Trp Leu Phe Trp Pro Ser Met Gly Tyr Ile Cys
               100                 105                 110
   Phe Val Met Asp Thr Arg Gly Gln Gly Ser Gly Trp Leu Lys Gly Asp
           115                 120                 125
   Thr Pro Asp Tyr Pro Glu Asp Pro Val Asp Pro Gln Tyr Pro Gly Phe
       130                 135                 140
   Met Thr Arg Gly Ile Leu Asp Pro Arg Thr Tyr Tyr Tyr Arg Arg Val
   145                 150                 155                 160
   Phe Thr Asp Ala Val Arg Ala Val Glu Ala Ala Ala Ser Phe Pro Arg
                   165                 170                 175
   Val Asp His Glu Arg Ile Val Ile Ala Gly Gly Ser Gln Gly Gly Gly
               180                 185                 190
   Ile Ala Leu Ala Val Ser Ala Leu Ser Lys Lys Ala Lys Ala Leu Leu
           195                 200                 205
   Cys Asp Val Pro Phe Leu Cys His Phe Arg Arg Ala Val Gln Leu Val
       210                 215                 220
   Asp Thr His Pro Tyr Ala Glu Ile Thr Asn Phe Leu Lys Thr His Arg
   225                 230                 235                 240
   Asp Lys Glu Glu Ile Val Phe Arg Thr Leu Ser Tyr Phe Asp Gly Val
                   245                 250                 255
   Asn Phe Ala Val Arg Ala Lys Ile Pro Ala Leu Phe Ser Val Gly Leu
               260                 265                 270
   Met Asp Asn Ile Cys Pro Pro Ser Thr Val Phe Ala Ala Tyr Asn His
           275                 280                 285
   Tyr Ala Gly Pro Lys Glu Ile Arg Ile Tyr Pro Tyr Asn Asn His Glu
       290                 295                 300
   Gly Gly Gly Ser Phe Gln Ala Ile Glu Gln Val Lys Phe Leu Lys Arg
   305 310 315 320
   Leu Phe Glu Lys Gly
   325
<210> 17
   <211> 329
   <212> PRT
   <213> Thermotoga sp. RQ2
<400> 17
   Met Ala Leu Phe Asp Met Pro Leu Glu Lys Leu Arg Ser Tyr Leu Pro
   1 5 10 15
   Asp Arg Tyr Glu Glu Glu Asp Phe Asp Leu Phe Trp Lys Glu Thr Leu
   20 25 30
   Glu Glu Ser Arg Lys Phe Pro Leu Asp Pro Ile Phe Glu Arg Val Asp
   35 40 45
   Tyr Leu Leu Glu Asn Val Glu Val Tyr Asp Val Thr Phe Ser Gly Tyr
   50 55 60
   Arg Gly Gln Arg Ile Lys Ala Trp Leu Ile Leu Pro Val Val Lys Lys
   65 70 75 80
   Glu Glu Arg Leu Pro Cys Ile Val Glu Phe Ile Gly Tyr Arg Gly Gly
   85 90 95
   Arg Gly Phe Pro Phe Asp Trp Leu Phe Trp Ser Ser Ala Gly Tyr Ala
   100 105 110
   His Phe Val Met Asp Thr Arg Gly Gln Gly Thr Ser Arg Val Lys Gly
   115 120 125
   Asp Thr Pro Asp Tyr Cys Asp Glu Pro Ile Asn Pro Gln Phe Pro Gly
   130 135 140
   Phe Met Thr Arg Gly Ile Leu Asp Pro Arg Thr Tyr Tyr Tyr Arg Arg
   145 150 155 160
   Val Phe Thr Asp Ala Val Arg Ala Val Glu Thr Ala Ser Ser Phe Pro
   165 170 175
   Gly Ile Asp Pro Glu Arg Ile Ala Val Val Gly Thr Ser Gln Gly Gly
   180 185 190
   Gly Ile Ala Leu Ala Val Ala Ala Leu Ser Glu Ile Pro Lys Ala Leu
   195 200 205
   Val Ser Asn Val Pro Phe Leu Cys His Phe Arg Arg Ala Val Gln Ile
   210 215 220
   Thr Asp Asn Ala Pro Tyr Ser Glu Ile Val Asn Tyr Leu Lys Val His
   225 230 235 240
   Arg Asp Lys Glu Glu Ile Val Phe Arg Thr Leu Ser Tyr Phe Asp Gly
   245 250 255
   Val Asn Phe Ala Ala Arg Ala Lys Ile Pro Ala Leu Phe Ser Val Ala
   260 265 270
   Leu Met Asp Lys Thr Cys Pro Pro Ser Thr Val Phe Ala Ala Tyr Asn
   275 280 285
   His Tyr Ala Gly Pro Lys Glu Ile Lys Val Tyr Pro Phe Asn Glu His
   290 295 300
   Glu Gly Gly Glu Ser Phe Gln Arg Met Glu Glu Leu Arg Phe Met Lys
   305 310 315 320
   Arg Ile Leu Lys Gly Glu Phe Lys Ala
   325
<210> 18
   <211> 27
   <212> DNA
   <213> artificial sequence
<220>
   <223> primer f27405
<400> 18
   atggcacaat tatatgatat gcctttg 27
<210> 19
   <211> 28
   <212> DNA
   <213> artificial sequence
<220>
   <223> primer r27405
<400> 19
   ttacggattc agctcatcca taagtatc 28
<210> 20
   <211> 978
   <212> DNA
   <213> artificial sequence
<220>
   <223> plasmid pSW196 plus SEQ ID 18 and 19
<400> 20
   atggctttct ttgacatgcc gctggaagaa ctgaaaaagt accgtccgga acgttacgag      60
   gaaaaagact ttgacgaatt ttggcgcgaa accctgaaag aatccgaggg tttcccactg     120
   gacccggtat ttgaaaaagt tgacttccac ctgaagaccg tcgaaactta cgacgtcacc     180
   ttcagcggtt atcgtggcca gcgtatcaaa ggttggctgc tggtaccgaa actggcggaa     240
   gagaaactgc cgtgtgttgt tcagtacatt ggttacaacg gtggccgtgg tttcccgcac     300
   gactggctgt tctggccgtc tatgggttac atctgcttcg ttatggacac ccgtggtcag     360
   ggtagcggtt ggatgaaggg tgatactccg gactacccgg aaggtccggt ggacccgcag     420
   tacccgggct tcatgacgcg cggcatcctg gatcctggca cctattacta ccgtcgtgtg     480
   tttgtcgatg ccgtgcgcgc cgttgaagcc gctatcagct tcccacgcgt cgattctcgt     540
   aaagtggtag ttgctggtgg ctctcaaggt ggcggcattg cactggcagt ttccgcgctg     600
   tccaaccgtg ttaaagccct gctgtgcgat gttccgttcc tgtgccactt ccgtcgtgcg     660
   gtacagctgg tggacaccca cccgtacgta gaaattacga acttcctgaa aacccatcgt     720
   gataaagaag agatcgtatt ccgtaccctg tcttactttg atggcgttaa ttttgcggct     780
   cgtgcaaaag taccggcgct gttcagcgta ggtctgatgg acactatttg tccgccgtct     840
   accgtattcg cagcctacaa ccactacgct ggtccgaaag aaatccgcat ctacccgtac     900
   aacaaccacg aaggtggtgg ttctttccag gcaatcgaac aggttaaatt cctgaaacgc     960
   ctgttcgaag aaggctaa                                                   978
<210> 21
   <211> 182
   <212> PRT
   <213> Bacillus subtilis ATCC 31954
<400> 21
   Arg Gly Gln Gln Ser Ser Glu Asp Thr Ser Ile Ser Leu His Gly His
   1               5                   10                  15
   Ala Leu Gly Trp Met Thr Lys Gly Ile Leu Asp Lys Asp Thr Tyr Tyr
               20                  25                  30
   Tyr Arg Gly Val Tyr Leu Asp Ala Val Arg Ala Leu Glu Val Ile Ser
           35                  40                  45
   Ser Phe Asp Glu Val Asp Glu Thr Arg Ile Gly Val Thr Gly Gly Ser
       50                  55                  60
   Gln Gly Gly Gly Leu Thr Ile Ala Ala Ala Ala Leu Ser Asp Ile Pro
   65                  70                  75                  80
   Lys Ala Ala Val Ala Asp Tyr Pro Tyr Leu Ser Asn Phe Glu Arg Ala
                   85                  90                  95
   Ile Asp Val Ala Leu Glu Gln Pro Tyr Leu Glu Ile Asn Ser Phe Phe
   100 105 110
   Arg Arg Asn Gly Ser Pro Glu Thr Glu Val Gln Ala Met Lys Thr Leu
   115 120 125
   Ser Tyr Phe Asp Ile Met Asn Leu Ala Asp Arg Val Lys Val Pro Val
   130 135 140
   Leu Met Ser Ile Gly Leu Ile Asp Lys Val Thr Pro Pro Ser Thr Val
   145 150 155 160
   Phe Ala Ala Tyr Asn His Leu Glu Thr Glu Lys Glu Leu Lys Val Tyr
   165 170 175
   Arg Tyr Phe Gly His Glu
   180
<210> 22
   <211> 50
   <212> DNA
   <213> artificial sequence
<220>
   <223> primer
<400> 22
   taactgcagt aaggaggaat aggacatggc cttcttcgat ttacccactc 50
<210> 23
   <211> 38
   <212> DNA
   <213> artificial sequence
<220>
   <223> primer
<400> 23
   tgatctagat tagcctttct caaatagttt tttcaaga 38
<210> 24
   <211> 1012
   <212> DNA
   <213> Thermotoga maritima
<400> 24
   taactgcagt aaggaggaat aggacatggc cttcttcgat ttaccactcg aagaactgaa 60
   gaaatatcgt ccagagcggt acgaagagaa agacttcgat gagttctggg aagagacact 120
   cgcagagagc gaaaagttcc ccttagaccc cgtcttcgag aggatggagt ctcacctcaa 180
   aacagtcgaa gcgtacgatg tcaccttctc cggatacagg ggacagagga tcaaagggtg 240
   gctccttgtt ccaaaactgg aagaagaaaa acttccctgc gttgtgcagt acataggata 300
   caacggtgga agaggattcc ctcacgactg gctgttctgg ccttctatgg gttacatatg      360
   tttcgtcatg gatactcgag gtcagggaag cggctggctg aaaggagaca caccggatta      420
   ccctgagggt cccgttgacc ctcagtatcc aggattcatg acaagaggaa tactggatcc      480
   cagaacttac tactacagac gagtcttcac ggacgctgtc agagccgttg aagctgctgc      540
   ttcttttcct caggtagatc aagaaagaat cgtgatagct ggaggcagtc agggtggcgg      600
   aatagccctt gcggtgagcg ctctctcaaa gaaagcaaag gctcttctgt gcgatgtgcc      660
   gtttctgtgt cacttcagaa gagcagtaca gcttgtggat acgcatccat acgcggagat      720
   cacgaacttt ctaaagaccc acagagacaa ggaagaaatc gtgttcagga ctctttccta      780
   tttcgatgga gtgaacttcg cagccagagc gaagatccct gcgctgtttt ctgtgggtct      840
   catggacaac atttgtcctc cttcaacggt tttcgctgcc tacaattact acgctggacc      900
   gaaggaaatc agaatctatc cgtacaacaa ccacgaggga ggaggctctt tccaagcggt      960
   tgaacaggtg aaattcttga aaaaactatt tgagaaaggc taatctagat ca             1012
<210> 25
   <211> 47
   <212> DNA
   <213> Artificial Sequence
<220>
   <223> Primer
<400> 25
   taagaattct aaggaatagg acatggcgtt tcttcgacct gcctctg 47
<210> 26
   <211> 36
   <212> DNA
   <213> Artificial Sequence
<220>
   <223> Primer
<400> 26
   aaactgcagt tagcccttct caaacagttt cttcag 36
<210> 27
   <211> 978
   <212> DNA
   <213> Thermotoga maritima
<400> 27
   atggccttct tcgatttacc actcgaagaa ctgaagaaat atcgtccaga gcggtacgaa       60
   gagaaagact tcgatgagtt ctgggaagag acactcgcag agagcgaaaa gttcccctta      120
   gaccccgtct tcgagaggat ggagtctcac ctcaaaacag tcgaagcgta cgatgtcacc      180
   ttctccggat acaggggaca gaggatcaaa gggtggctcc ttgttccaaa actggaagaa      240
   gaaaaacttc cctgcgttgt gcagtacata ggatacaacg gtggaagagg attccctcac      300
   gactggctgt tctggccttc tatgggttac atatgtttcg tcatggatac tcgaggtcag 360
   ggaagcggct ggctgaaagg agacacaccg gattaccctg agggtcccgt tgaccctcag 420
   tatccaggat tcatgacaag aggaatactg gatcccagaa cttactacta cagacgagtc 480
   ttcacggacg ctgtcagagc cgttgaagct gctgcttctt ttcctcaggt agatcaagaa 540
   agaatcgtga tagctggagg cagtcagggt ggcggaatag cccttgcggt gagcgctctc 600
   tcaaagaaag caaaggctct tctgtgcgat gtgccgtttc tgtgtcactt cagaagagca 660
   gtacagcttg tggatacgca tccatacgcg gagatcacga actttctaaa gacccacaga 720
   gacaaggaag aaatcgtgtt caggactctt tcctatttcg atggagtgaa cttcgcagcc 780
   agagcgaaga tccctgcgct gttttctgtg ggtctcatgg acaacatttg tcctccttca 840
   acggttttcg ctgcctacaa ttactacgct ggaccgaagg aaatcagaat ctatccgtac 900
   aacaaccacg agggaggagg ctctttccaa gcggttgaac aggtgaaatt cttgaaaaaa 960
   ctatttgaga aaggctaa 978
<210> 28
   <211> 23
   <212> DNA
   <213> Artificial Sequence
<220>
   <223> Primer
<400> 28
   ggacaacatc tcacctcctt cta 23
<210> 29
   <211> 23
   <212> DNA
   <213> Artificial Sequence
<220>
   <223> Primer
<400> 29
   tagaaggagg tgagatgttg tcc 23
<210> 30
   <211> 975
   <212> DNA
   <213> Artificial Sequence
<220>
   <223> Codon optimized sequence
<400> 30
   atggcgttct tcgacctgcc tctggaagaa ctgaagaaat accgtccaga gcgttacgaa 60
   gagaaggact tcgacgagtt ctgggaggaa actctggcgg agagcgaaaa gtttccgctg 120
   gacccagtgt tcgagcgtat ggaatctcac ctgaaaaccg tggaggcata tgacgttact 180
   ttttctggtt accgtggcca gcgtatcaaa ggctggctgc tggttccgaa actggaggaa    240
   gaaaaactgc cgtgcgtagt tcagtacatc ggttacaacg gtggccgtgg ctttccgcac    300
   gattggctgt tctggccgtc tatgggctac atttgcttcg tcatggatac tcgtggtcag    360
   ggttccggct ggctgaaagg cgatactccg gattatccgg agggcccggt agacccgcag    420
   taccctggct tcatgacgcg tggtattctg gatccgcgta cctattacta tcgccgcgtt    480
   tttaccgatg cagttcgtgc cgtagaggcc gcggcttctt tccctcaggt tgaccaggag    540
   cgtattgtta tcgctggtgg ctcccagggt ggcggcatcg ccctggcggt atctgcgctg    600
   agcaagaaag ctaaggcact gctgtgtgac gtcccgttcc tgtgtcactt ccgtcgcgct    660
   gttcagctgg tagataccca tccgtacgcg gagattacta acttcctgaa aactcaccgc    720
   gacaaagaag aaatcgtttt ccgcaccctg tcctatttcg acggcgttaa cttcgcggct    780
   cgtgcaaaaa ttccggcact gttctctgtt ggtctgatgg acaacatctg ccctccttct    840
   accgttttcg cggcatataa ctattatgcg ggtccgaaag aaatccgtat ctatccgtac    900
   aacaaccacg aaggcggtgg tagctttcag gctgttgaac aagtgaaatt cctgaagaaa    960
   ctgtttgaga agggc                                                      975